# EUROPEAN PATENT APPLICATION

(11) **EP 3 904 348 A1**
(43) Date of publication of application: **03.11.2021**
(21) Application number: 19902618.8
(22) Date of filing: 19.12.2019
(51) Int. Cl.: C07D 403/04, A61K 31/4375, A61K 31/4412, A61P 35/00

(54) **AMINOPYRIDINE COMPOUND, PREPARATION METHOD THEREFOR AND USE THEREOF**

(30) Priority: 28.12.2018 CN 201811616758; 24.04.2019 CN 201910332212
(71) Applicant: Sichuan Kelun-Biotech Biopharmaceutical Co., Ltd., Chengdu, Sichuan 611138 (CN)
(72) Inventor: LIU, Jinming, Chengdu, Sichuan 611138 (CN); HE, Ting, Chengdu, Sichuan 611138 (CN); CAI, Jiaqiang, Chengdu, Sichuan 611138 (CN); DONG, Zhenwen, Chengdu, Sichuan 611138 (CN); TIAN, Qiang, Chengdu, Sichuan 611138 (CN); SONG, Hongmei, Chengdu, Sichuan 611138 (CN); XUE, Tongtong, Chengdu, Sichuan 611138 (CN); WANG, Lichun, Chengdu, Sichuan 611138 (CN); WANG, Jingyi, Chengdu, Sichuan 611138 (CN)
(74) Representative: Turner, Craig Robert
(86) International application number: PCT/CN2019/126444
(87) International publication number: WO 2020/135195

(57) **Abstract**

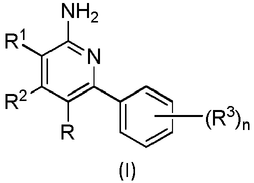

The present invention discloses an aminopyridine compounds, a preparation method and use thereof, particularly the aminopyridine compounds of formula (I), pharmaceutical compositions containing the same, the method for preparing the same and the use thereof in the prophylaxis or treatment of an adenosine A2a receptor related disease.

## Description

### FIELD OF THE INVENTION

The present invention relates to aminopyridine compounds as adenosine receptor antagonists, methods and intermediates for preparing the same, pharmaceutical compositions containing the same and the therapeutic use thereof.

### BACKGROUND OF THE INVENTION

Adenosine is a signaling molecule that inhibits inflammation and immune response in body. Extracellular adenosines have two main sources, i.e., transportation of intracellular adenosines and hydrolysis of extracellular adenine ribosides. Adenosine receptors are a type of G protein-coupled receptors (GPCR). This family of receptors mainly include four types, A1, A2a, A2b and A3 receptors. The A2a and A2b receptors are coupled to a Gs protein that activates adenylate cyclases to stimulate the production of an intracellular signaling molecule of cyclic adenosine monophosphate (cAMP).

Adenosine A2a receptors are expressed on the surfaces of some cells in the immune system, such as T cells, NK cells, macrophages and dendritic cells. Adenosines produced by tumors may interact with adenosine A2a receptors on the surfaces of tumor tissue infiltrative immune cells, which results in the increased amount of cAMP in the immune cells, thereby inhibiting the ability of the immune cells to attack tumors, causing immune tolerance in body, and further allowing tumor cells to escape from the immune surveillance of the body mainly in two manners: (1) blocking the activation and functioning of immune cells that can kill tumor cells; and (2) increasing the quantity of regulatory T cells (T-regs) that can inhibit the response of immune cells to tumor cells. By these mechanisms, tumor cells escape from the surveillance and attack of the immune system and improve their survival rate. In A2a receptor gene knockout mouse, CD8+T cells can exhibit enhanced anti-tumor immune effect and significantly inhibit tumor proliferation. Melanoma or lymphoma cells are easier to grow when transplanted into a wild-type mouse than adenosine A2a receptor gene knockout mouse, and adenosine A2a receptor gene knockout mouse have better response to tumor vaccines.

Adenosine A2a receptors are expressed at a high level on immune cells. Activation of adenosine A2a receptors may promote the development of immune tolerance in body, promote the formation of "immune escape" or "immune suppression" of tumor cells, and create favorable conditions for the development and progress of tumors. Adenosine A2a receptor antagonists directly target the adenosine A2a receptors on the surfaces of immune cells, inhibit the activation of these receptors, thereby inhibit the generation of cAMP in the immune cells, and eliminate the inhibition of T cell immune function that is mediated by adenosine A2a receptor activation, thereby achieving therapeutic effect on tumors. Therefore, adenosine A2a receptor inhibitors are promising therapeutic drugs for tumors in the pharmaceutical industry.

CPI-444 from Corvus Company is a compound that has an antagonistic effect on adenosine A2a receptors, with the indication of tumor. CPI-444 was previously used for the treatment of central nervous system diseases in clinical trials. WO2001062233A2 discloses aminopyridine or aminopyrimidine compounds which have antagonistic effects on adenosine A2a receptors and may serve as therapeutic agents for Parkinson's disease or Alzheimer's disease. WO2003035639A1 discloses an aminopyrimidine compound which has an antagonistic effect on adenosine A2a receptors and may serve as a therapeutic agent for Parkinson's disease or depression. WO200357689A1 discloses an aminopyrimidine compound which has an antagonistic effect on adenosine A2a receptors and may serve as a therapeutic agent for diseases such as depression, dementia, Parkinson's disease, anxiety and pain. WO2005079800A1 discloses an aminopyrimidine compound which has an antagonistic effect on adenosine A2a receptors and may serve as a therapeutic agent for diseases such as Parkinson's disease or Huntington's disease. WO2011095625A1 discloses an aminotriazine compound which has an antagonistic effect on adenosine A2a receptors and may serve as a therapeutic agent for dyskinesia, stroke, or Parkinson's disease. WO2018130184A1 discloses an aminotriazine compound which has an antagonistic effect on adenosine A2a receptors and may serve as a therapeutic agent for cancer. Adenosine is an endogenous regulator of a variety of physiological functions, for example, exhibits a sedative effect in the central nervous system. In WO2001062233A2, WO2002014282A1, WO2003035639A1, WO200357689A1, WO2005079800A1 and WO2011095625A1 all focusing on the treatment of central nervous system diseases, the compounds involved are designed to pass through the blood brain barrier and interact with adenosine A2a receptors in the central nervous system. However, the concentration of adenosines in tumor tissues is higher than that in the brain, and a larger amount of compounds are needed to eliminate immunosuppression, in order to obtain the therapeutic effect on tumors. Therefore, this may cause toxic side effects to the central nervous system.

WO2002014282A1 discloses a 2-aminopyridine compound represented by general formula (A), 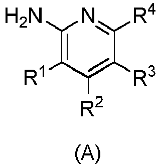 which has an antagonistic effect on adenosine A2a receptors and may serve as a therapeutic agent for Parkinson's disease and inflammatory bowel diseases. This patent application discloses a class of compounds with an amide group. However, this patent application does not disclose data for the activity of such compounds on adenosine A1 and A2a receptors and data for the distribution in brain tissues of the compounds.

Adenosine A2a receptor antagonists are promising drugs in the pharmaceutical industry. In order to achieve better therapeutic effects on tumors and better meet market needs, there is an urgent need to develop an adenosine A2a receptor antagonist for tumor therapy, especially with low toxic side effects to the central nervous system.

### SUMMARY OF THE INVENTION

The present invention provides an aminopyridine compound, which has a good antagonistic effect on adenosine A2a receptors but a weak antagonistic effect on adenosine A1 receptors, thus having good antitumor activity. The compound of the present invention also has a variety of excellent properties, such as good physical and chemical properties (e.g., solubility, physical and/or chemical stability), good pharmacokinetic properties (e.g., excellent drug exposure and excellent oral absorption), good safety (lower toxicity and/or fewer side effects, broader treatment window). In particular, some compounds of the present invention have peripheral selectivity and cannot pass through the blood brain barrier into the brain, and therefore have low or no toxic side effects to the central nervous system when exerting anti-peripheral tumor effects.

In some aspects, the present invention provides a compound or a pharmaceutically acceptable salt, stereoisomer, tautomer, polymorph, solvate, *N*-oxide, isotopically labeled compound, metabolite or prodrug thereof, wherein the compound has a structure of formula (I): 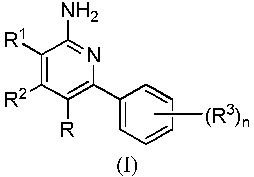 wherein:
R is 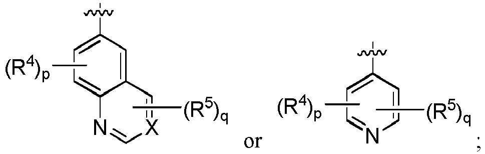
R¹ is selected from the group consisting of hydrogen, halogen, cyano, C₁₋₆ alkyl, C₁₋₆ haloalkyl, C₃₋₆ cycloalkyl, carboxyl, C₁₋₆ alkyl-OC(O)-, R^{a}R^{b}N-C(O)-, 5- to 6-membered heterocyclyl and 5- to 6-membered heteroaryl, wherein the heterocyclyl and the heteroaryl groups are optionally substituted with substituents independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
R² is selected from the group consisting of hydrogen, halogen, cyano, C₁₋₆ alkyl, C₁₋₆ haloalkyl, carboxyl and R^{a}R^{b}N-C(O)-;
R³ is selected from the group consisting of hydrogen, halogen, cyano, C₁₋₆ alkyl, C₁₋₆ haloalkyl and C₁₋₆ alkoxyl;
R⁴ is selected from the group consisting of hydrogen, halogen, C₁₋₆ alkyl, and C₁₋₆ haloalkyl;
R⁵ is selected from the group consisting of hydrogen, halogen, C₁₋₆ alkyl, and C₁₋₆ haloalkyl;
R^{a} and R^{b} are each independently selected from the group consisting of hydrogen, C₁₋₆ alkyl, C₃₋₆ cycloalkyl, hydroxy-C₁₋₆ alkyl-, C₁₋₆ alkoxy-C₁₋₆ alkyl- and 5- to 6-membered heterocyclyl-C₁₋₆ alkyl-;
n is selected from 0, 1 or 2;
p is independently selected from 0, 1 or 2; and
q is independently selected from 0, 1 or 2;
provided that:
when R is 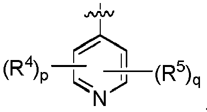 , p+q≥2 and at least one of (R⁴)ₚ and (R⁵)_{q} is halogen.

In another aspect, the present invention provides a pharmaceutical composition, comprising a prophylactically or therapeutically effective amount of the compound of the present invention or a pharmaceutically acceptable salt, stereoisomer, tautomer, polymorph, solvate, N-oxide, isotopically labeled compound, metabolite or prodrug thereof, and one or more pharmaceutically acceptable carriers.

In another aspect, the present invention provides a method for preparing a pharmaceutical composition, comprising combining the compound of the present invention or a pharmaceutically acceptable salt, stereoisomer, tautomer, polymorph, solvate, N-oxide, isotopically labeled compound, metabolite or prodrug thereof, with one or more pharmaceutically acceptable carriers.

In another aspect, the present invention provides use of the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention in the preparation of a medicament for the prophylaxis or treatment of an adenosine A2a receptor related disease.

In another aspect, the present invention provides the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention for use in inhibiting the activity of an adenosine A2a receptor.

In another aspect, the present invention provides the compound of the present invention or a pharmaceutically acceptable salt, stereoisomer, tautomer, polymorph, solvate, N-oxide, isotopically labeled compound, metabolite or prodrug thereof or the pharmaceutical composition of the present invention for use in the prophylaxis or treatment of an adenosine A2a receptor related disease.

In another aspect, the present invention provides a method for the prophylaxis or treatment of an adenosine A2a receptor related disease, comprising administering to a subject in need thereof an effective amount of the compound of the present invention or a pharmaceutically acceptable salt, stereoisomer, tautomer, polymorph, solvate, N-oxide, isotopically labeled compound, metabolite or prodrug thereof or the pharmaceutical composition of the present invention.

In another aspect, the present invention provides a method for preparing the compound of the present invention.

### Definitions

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

As used herein, the terms "contain", "include", "comprise", "have", or "relate to", as well as other variations used herein are inclusive or open-ended, and do not exclude other unlisted elements or method steps, even though such unlisted elements or method steps are not necessarily present (that is, these terms also include the terms "essentially consist of ..." and "consist of ...").

As used herein, the term "alkyl" means a linear or branched saturated aliphatic hydrocarbyl group. In some embodiments, the term "C₁₋₁₂ alkyl" refers to a linear or branched alkyl having 1-12 carbon atoms. For example, as used herein, the term "C₁₋₆ alkyl" refers to a linear or branched alkyl having 1-6 carbon atoms (such as methyl, ethyl, n-propyl, isopropyl, n-butyl, isobutyl, sec-butyl, *tert-butyl,* n-pentyl, or n-hexyl), which is optionally substituted with one or more (e.g., 1 to 3) suitable substituents such as halogen (in which case the group may be referred to as "haloalkyl", such as CF₃, C₂F₅, CHF₂, CH₂F, CH₂CF₃, CH₂Cl or -CH₂CH₂CF₃, *etc.*)*.*

As used herein, the term "cycloalkyl" refers to a saturated or partially unsaturated nonaromatic monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system, such as bicyclo[1.1.1]pentyl, bicyclo[2.2.1]heptyl, bicyclo[3.2.1]octyl or bicyclo[5.2.0]nonyl, or decahydronaphthalene *etc*.), which is optionally substituted with one or more (e.g., 1 to 3) suitable substituents. The cycloalkyl has 3 to 15 carbon atoms, such as 3 to 6 carbon atoms. For example, the term "C₃₋₆ cycloalkyl" refers to a saturated or partially unsaturated nonaromatic monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the term "alkoxy" means an alkyl (as defined above) in which an oxygen atom is inserted at any reasonable position, such as C₁₋₈ alkoxy, C₁₋₆ alkoxy, C₁₋₄ alkoxy, or C₁₋₃ alkoxy. Representative examples of C₁₋₆ alkoxy include, but are not limited to, methoxy, ethoxy, propoxy, isopropoxy, n-propoxy, isopropoxy, n-butoxy, isobutoxy, tert-butoxy, pentyloxy, hexyloxy, -CH₂-OCH₃. The alkoxy may be optionally substituted with one or more (such as 1 to 3) substituents which may be the same or different.

As used herein, the term "halo" or "halogen" is defined to include fluorine, chlorine, bromine or iodine.

As used herein, the term "haloalkyl" refers to an alkyl substituted with one or more (e.g., 1 to 3) halogen atoms which may be the same or different. The terms "C₁₋₈ haloalkyl", "C₁₋₆ haloalkyl" and "C₁₋₃ haloalkyl" refer to haloalkyl groups having 1 to 8 carbon atoms, 1to 6 carbon atoms and 1 to 3 carbon atoms, respectively, such as -CF₃, -C₂F₅, -CHF₂, -CH₂F, -CH₂CF₃, -CH₂Cl or -CH₂CH₂CF₃.

As used herein, the term "heterocyclyl" refers to a saturated or partially unsaturated monocyclic or polycyclic group, which has in the ring, for example, 2, 3, 4, 5, 6, 7, 8, or 9 carbon atoms and one or more (for example, 1, 2, 3, or 4) hetero atoms selected from nitrogen, oxygen or S(O)ₘ (wherein m is an integer of 0 to 2). Examples include, but are not limited to oxiranyl, aziridinyl, azetidinyl, oxetanyl, tetrahydrofuranyl, pyrrolidinyl, pyrrolidonyl, imidazolidinyl, pyrazolidinyl, tetrahydropyranyl, piperidinyl, morpholinyl, dithianyl, thiomorpholinyl, piperazinyl, trithianyl. For example, it can be a 5- to 6-membered heterocyclyl.

As used herein, the terms "heteroaryl" and "heteroaromatic ring" refer to a monocyclic, bicyclic or tricyclic aromatic ring system containing at least one hetero atom selected from N, O and S, which, for example, has 5, 6, 8, 9, 10, 11, 12, 13 or 14 ring atoms, particularly 1, 2, 3, 4, 5, 6, 9 or 10 carbon atoms. Moreover, in each case, it can be benzo-fused. For example, heteroaryl or heteroaromatic ring may be selected from the group consisting of thienyl (ring), furyl (ring), pyrrolyl (ring), oxazolyl (ring), thiazolyl (ring), imidazolyl (ring), pyrazolyl (ring), isoxazolyl (ring), isothiazolyl (ring), oxadiazolyl (ring), triazolyl (ring), thiadiazolyl (ring), and benzo derivatives thereof; or pyridyl (ring), pyridazinyl (ring), pyrimidinyl (ring), pyrazinyl (ring), triazinyl (ring), and benzo derivatives thereof.

The term "carboxy" refers to the group -COOH.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted", the substituent may be either (1) not substituted, or (2) substituted. If a carbon of a substituent is described as being optionally substituted with one or more of a list of substituents, one or more of the hydrogens on the carbon (to the extent there are any) may optionally be separately and/or together replaced with an independently selected substituent. If a nitrogen of a substituent is described as being optionally substituted with one or more from a list of substituents, one or more of the hydrogens on the nitrogen (to the extent there are any) may each optionally be replaced with an independently selected substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5, 6, 7, 8, 9 or 10) where appropriate.

As used herein, unless specified, the point of attachment of a substituent can be at any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as ²H, ³H, deuterium D, tritium T; carbon, such as ¹¹C, ¹³C, and ¹⁴C; chlorine, such as ³⁷Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line (-), a solid wedge ( 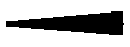 ), or a dotted wedge ( 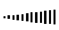 ). The use of a solid line to depict bonds to asymmetric carbon atoms is meant to indicate that all possible stereoisomers (e.g., specific enantiomers, racemic mixtures, *etc.*) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free from, or where appropriate, in a form of a pharmaceutically acceptable derivative. In the present invention, the pharmaceutically acceptable derivative includes, but is not limited to a pharmaceutically acceptable salt, solvate, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. A suitable base addition salt is formed from a base which forms a pharmaceutically acceptable salt.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-chloroperbenzoic acid (MCPBA), hydrogen peroxide, alkyl hydroperoxides such as *tert*-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems", Vol. 14, ACS Symposium Series (T. Higuchi and V. Stella), and "Bioreversible Carriers in Drug Design", Pergamon Press, 1987 (Edited by E. B. Roche, American Pharmaceutical Association). The prodrug in accordance with the invention can, for example, be produced by replacing appropriate functionalities present in the compound of the present invention with certain moieties known to those skilled in the art as "pro-moieties" (as described, for example, in "Design of Prodrugs" by H. Bundgaard (Elsevier, 1985)).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in Protective Groups in Organic Chemistry, ed. J.F.W. McOmie, Plenum Press, 1973, and T.W. Greene & P.G.M. Wuts, Protective Groups in Organic Synthesis, John Wiley & Sons, 1991, which are incorporated herein by reference. The protecting groups may be removed at a convenient subsequent stage using methods known from the art.

The term "about" refers to a range within ±10%, preferably within ±5%, and more preferably within ±2% of the specified value.

### Compound

In a first aspect, the present invention provides a compound or a pharmaceutically acceptable salt, stereoisomer, tautomer, polymorph, solvate, N-oxide, isotopically labeled compound, metabolite or prodrug thereof, wherein the compound has a structure of formula (I): 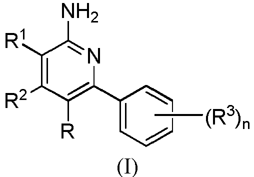 wherein:
R is 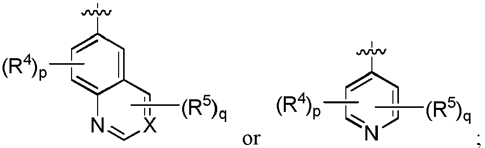
X is N or CH;
R¹ is selected from the group consisting of hydrogen, halogen, cyano, C₁₋₆ alkyl, C₁₋₆ haloalkyl, C₃₋₆ cycloalkyl, carboxyl, C₁₋₆ alkyl-OC(O)-, R^{a}R^{b}N-C(O)-, 5- to 6-membered heterocyclyl and 5- to 6-membered heteroaryl, wherein the heterocyclyl and the heteroaryl groups are optionally substituted with substituents independently selected from the groups consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
R² is selected from the group consisting of hydrogen, halogen, cyano, C₁₋₆ alkyl, C₁₋₆ haloalkyl, carboxyl and R^{a}R^{b}N-C(O)-;
R³ is selected from the group consisting of hydrogen, halogen, cyano, C₁₋₆ alkyl, C₁₋₆ haloalkyl and C₁₋₆ alkoxyl;
R⁴ is selected from the group consisting of hydrogen, halogen, C₁₋₆ alkyl, and C₁₋₆ haloalkyl;
R⁵ is selected from the group consisting of hydrogen, halogen, C₁₋₆ alkyl, and C₁₋₆ haloalkyl;
R^{a} and R^{b} are each independently selected from the group consisting of hydrogen, C₁₋₆ alkyl, C₃₋₆ cycloalkyl, hydroxy-C₁₋₆ alkyl-, C₁₋₆ alkoxy-C₁₋₆ alkyl- and 5- to 6-membered heterocyclyl-C₁₋₆ alkyl-;
n is selected from 0, 1 or 2;
p is independently selected from 0, 1 or 2; and
q is independently selected from 0, 1 or 2;
provided that:
when R is 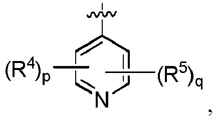 p+q≥2 and at least one of (R⁴)ₚ and (R⁵)_{q} is halogen.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R^{a} and R^{b} are each independently selected from the group consisting of hydrogen, C₁₋₃ alkyl, C₃₋₆ cycloalkyl, hydroxy-C₁₋₃ alkyl-, C₁₋₃ alkoxy-C₁₋₃ alkyl- and 5- to 6-membered heterocyclyl-C₁₋₃ alkyl-;
preferably, R^{a} and R^{b} are each independently selected from the group consisting of hydrogen and C₁₋₃ alkyl;
preferably, R^{a} and R^{b} are each independently selected from the group consisting of hydrogen and methyl;
preferably, R^{a} is hydrogen, and R^{b} is hydrogen or methyl.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, C₁₋₃ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, carboxy, C₁₋₃ alkyl-OC(O)- and R^{a}R^{b}N-C(O);
preferably, R¹ is selected from the group consisting of hydrogen, halogen, cyano, carboxy, CH₃-OC(O)-, NH₂-C(O)- and NH(CH₃)-C(O)-;
preferably, R¹ is selected from the group consisting of hydrogen, fluorine, chlorine, bromine, iodine, cyano, carboxyl, CH₃-OC(O)-, NH₂-C(O)- and CH₃-NH-C(O)-;
preferably, R¹ is selected from the group consisting of hydrogen, bromine, cyano, carboxyl, CH₃-OC(O)-, NH₂-C(O)- and NH(CH₃)-C(O)-.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R¹ is selected from 5- to 6-membered heterocyclyl and 5- to 6-membered heteroaryl, wherein the 5- to 6-membered heterocyclyl and the 5- to 6-membered heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of C₁₋₃ alkyl, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of methyl, ethyl, n-propyl, isopropyl, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of methyl, ethyl, n-propyl, isopropyl, hydroxymethyl, hydroxyethyl, OH-(CH₂)₃-, OH-CH(CH₃)-CH₂-, OH-C(CH₃)₂-CH₂-, CH₃O-CH₂-, CH₃O-CH₂CH₂-, CH₃O-(CH₂)₃-, CH₃CH₂O-CH₂-, CH₃CH₂O-CH₂CH₂-, CH₃CH₂O-(CH₂)₃-, CH₃CH₂CH₂O-(CH₂)₃-, NH₂-CH₂-, NH₂-CH₂CH₂-, NH(CH₃)-CH₂-, NH(CH₃)-CH₂CH₂-, N(CH₃)₂-CH₂-, N(CH₃)₂-CH₂CH₂-, NH₂-C(O)-CH₂-, NH₂-C(O)-CH₂CH₂-, NH(CH₃)-C(O)-CH₂-, NH(CH₃)-C(O)-CH₂CH₂-, N(CH₃)₂-C(O)-CH₂-, N(CH₃)₂-C(O)-CH₂CH₂-, NH₂-C(O)-, NH(CH₃)-C(O)-, N(CH₃)₂-C(O)-, CH₃O-CH₂O-CH₂-, CH₃O-CH₂O-CH₂CH₂-, CH₃O-CH₂O-(CH₂)₃-, CH₃O-CH₂CH₂O-CH₂-, CH₃O-CH₂CH₂O-CH₂CH₂-, CH₃O-CH₂CH₂O-(CH₂)₃-, CH₃CH₂O-CH₂CH₂O-CH₂- and CH₃CH₂O-CH₂CH₂O-CH₂-CH₂-;
preferably, the 5- to 6-membered heterocyclyl and the 5- to 6-membered heteroaryl are optionally substituted with a substituent independently selected from the group consisting of methyl, ethyl, hydroxymethyl, hydroxyethyl, OH-CH(CH₃)-CH₂-, OH-C(CH₃)₂-CH₂-, CH₃O-CH₂-, CH₃O-CH₂CH₂-, CH₃CH₂O-CH₂-, CH₃CH₂O-CH₂CH₂-, NH₂-CH₂-, NH₂-CH₂CH₂-, NH(CH₃)-CH₂-, NH(CH₃)-CH₂CH₂-, NH₂-C(O)-CH₂-, NH₂-C(O)-CH₂CH₂-, NH(CH₃)-C(O)-CH₂-, NH(CH₃)-C(O)-CH₂CH₂-, NH₂-C(O)-, NH(CH₃)-C(O)-, CH₃O-CH₂O-CH₂-, CH₃O-CH₂O-CH₂CH₂-, CH₃O-CH₂CH₂O-CH₂-, CH₃O-CH₂CH₂O-CH₂CH₂- and CH₃CH₂O-CH₂CH₂O-CH₂-.

In some embodiments, R¹ is a 5- to 6-membered heteroaryl.

In some embodiments, the 5- to 6-membered heteroaryl is selected from the group consisting of pyrrolyl, pyrazolyl, imidazolyl, triazolyl, tetrazolyl, oxazolyl, isoxazolyl, thiazolyl, isothiazolyl, oxadiazolyl, thiadiazolyl, pyridyl, pyridazinyl, pyrimidinyl, pyrazinyl and triazinyl;
preferably, the 5- to 6-membered heteroaryl is selected from the group consisting of pyrazolyl, imidazolyl, triazolyl, tetrazolyl, oxazolyl, isoxazolyl, thiazolyl, isothiazolyl, oxadiazolyl, and thiadiazolyl;
preferably, the 5- to 6-membered heteroaryl is selected from the group consisting of pyrazolyl, imidazolyl, triazolyl, tetrazolyl, oxadiazolyl and thiadiazolyl;
preferably, the 5- to 6-membered heteroaryl is selected from the group consisting of pyrazolyl, triazolyl, tetrazolyl, oxadiazolyl, and thiadiazolyl;
preferably, the 5- to 6-membered heteroaryl is selected from the group consisting of pyrazolyl, tetrazolyl, and oxadiazolyl;
preferably, the 5- to 6-membered heteroaryl is selected from

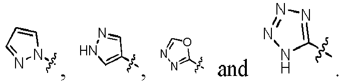

In some embodiments, R¹ is selected from

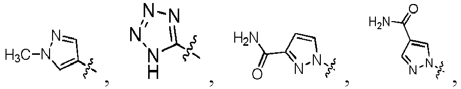

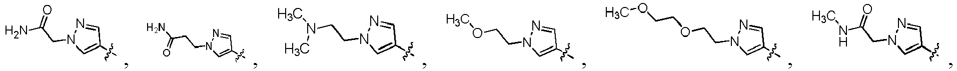

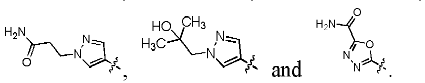

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R² is selected from the group consisting of hydrogen, halogen, cyano, C₁₋₃ alkyl, C₁₋₃ haloalkyl, carboxyl and R^{a}R^{b}N-C(O)-;
preferably, R² is selected from the group consisting of hydrogen, fluorine, chlorine, bromine, iodine, cyano, methyl, ethyl, n-propyl, isopropyl, carboxyl and NH₂-C(O)-;
preferably, R² is selected from the group consisting of hydrogen, fluorine, chlorine, bromine and cyano;
preferably, R² is selected from the group consisting of hydrogen, chlorine and cyano.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R³ is selected from the group consisting of hydrogen, halogen, cyano, C₁₋₃ alkyl, C₁₋₃ haloalkyl and C₁₋₃ alkoxyl;
preferably, R³ is selected from the group consisting of fluorine, chlorine, bromine and iodine;
preferably, R³ is fluorine.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R⁴ is selected from the group consisting of hydrogen, halogen, C₁₋₃ alkyl, and C₁₋₃ haloalkyl;
preferably, R⁴ is selected from the group consisting of hydrogen, fluorine, chlorine, bromine, iodine, C₁₋₃ alkyl, and C₁₋₃ haloalkyl;
preferably, R⁴ is selected from the group consisting of hydrogen, fluorine, chlorine, bromine, iodine, methyl, ethyl, n-propyl, and isopropyl;
preferably, R⁴ is hydrogen or methyl.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
R⁵ is selected from the group consisting of hydrogen, halogen, C₁₋₃ alkyl, and C₁₋₃ haloalkyl;
preferably, R⁵ is selected from the group consisting of hydrogen, fluorine, chlorine, bromine, iodine, C₁₋₃ alkyl, and C₁₋₃ haloalkyl;
preferably, R⁵ is selected from the group consisting of hydrogen, fluorine, chlorine, bromine, iodine, methyl, ethyl, n-propyl, and isopropyl;
preferably, R⁵ is chlorine or methyl.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
q is 1 or 2.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
q is 1.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
p is 1 or 2.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
p is 0 or 1.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein:
n is 1 or 2.

In some embodiments, the present invention provides the compound as described above, or a pharmaceutically acceptable salt, stereoisomer, tautomer, polymorph, solvate, N-oxide, isotopically labeled compound, metabolite or prodrug thereof, wherein: R is

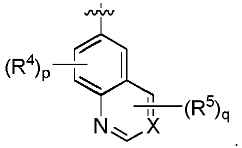

In other words, the compound of formula (I) has the structure of formula (Ia): 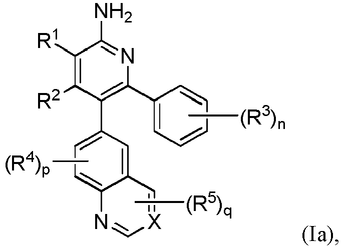 wherein R¹, R², R³, R⁴, R⁵, X, n, p and q are as defined above.

In some embodiments of the compound of formula (Ia), R¹ is selected from the group consisting of a 5- to 6-membered heterocyclyl and a 5- to 6-membered heteroaryl, wherein the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-; and wherein R^{a} and R^{b} are each independently selected from the group consisting of hydrogen and C₁₋₃ alkyl.

In some other embodiments of the compound of formula (Ia), R¹ is selected the group consisting of a 5- to 6-membered heterocyclyl and a 5- to 6-membered heteroaryl, wherein the heterocyclyl and the heteroaryl are optionally substituted with a substituent independently selected from the group consisting of C₁₋₆ alkyl, C₁₋₆ haloalkyl, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-; and wherein R^{a} and R^{b} are each independently selected from the group consisting of hydrogen, methyl, ethyl and propyl.

In some preferred embodiments, R¹ is selected from

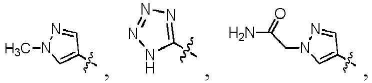

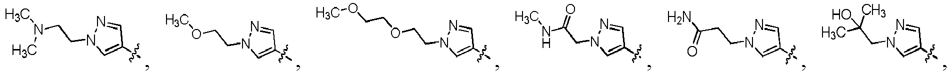

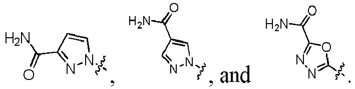

In some embodiments of the compound of formula (Ia), X is N. In yet other embodiments, X is CH.

In some embodiments of the compound of formula (Ia), R² is selected from the group consisting of hydrogen, halogen and cyano, preferably selected from the group consisting of hydrogen, chlorine and cyano; and R³ is selected from halogen, preferably fluorine.

In some embodiments of the compound of formula (Ia), R⁴ is hydrogen; and R⁵ is selected from C₁₋₃ alkyl, preferably methyl.

In some embodiments, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, where the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
X is selected from N and CH;
R² is selected from the group consisting of hydrogen, halogen, cyano, C₁₋₃ alkyl, C₁₋₃ haloalkyl, carboxyl and R^{a}R^{b}N-C(O)-;
R³ is selected from the group consisting of hydrogen, halogen, cyano, C₁₋₃ alkyl, C₁₋₃ haloalkyl and C₁₋₃ alkoxyl;
R⁴ is selected from the group consisting of hydrogen, halogen, C₁₋₃ alkyl, and C₁₋₃ haloalkyl;
R⁵ is selected from the group consisting of hydrogen, halogen, C₁₋₃ alkyl and C₁₋₃ haloalkyl; preferably, R⁵ is selected from the group consisting of hydrogen, fluorine, chlorine, bromine, iodine, C₁₋₃ alkyl and C₁₋₃ haloalkyl; preferably, R⁵ is selected from the group consisting of hydrogen, fluorine, chlorine, bromine, iodine, methyl, ethyl, propyl and isopropyl;
R^{a} and R^{b} are each independently selected from the group consisting of hydrogen and C₁₋₃ alkyl;
q is 1;
p is 1;
n is 1 or 2.

In some other embodiments, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₄ alkyl-, C₁₋₃ alkoxy-C₁₋₃ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
preferably, R¹ is selected from the group consisting of hydrogen, halogen, cyano, carboxy, C₁₋₃ alkyl-OC(O)-, R^{a}R^{b}N-C(O)- and 5- to 6-membered heteroaryl, wherein the heteroaryl is optionally substituted with a substituent independently selected from the group consisting of C₁₋₃ alkyl, hydroxy-C₁₋₄ alkyl-, C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, R¹ is selected from the group consisting of hydrogen, halogen, cyano, carboxy, CH₃O-C(O)-, NH₂-C(O)-, NH(CH₃)-C(O)- and 5- to 6-membered heteroaryl, wherein the heteroaryl is optionally substituted with a substituent independently selected from the group consisting of C₁₋₃ alkyl, hydroxy-C₁₋₄ alkyl-, C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, R¹ is selected from the group consisting of hydrogen, bromine, CH₃O-C(O)-, cyano, carboxyl, NH₂-C(O)-, NH(CH₃)-C(O)-, 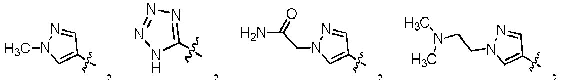 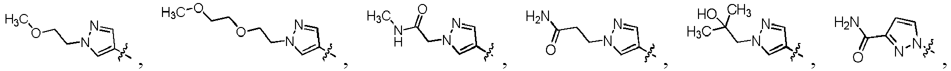 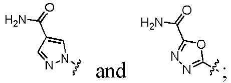
X is selected from N and CH;
R² is selected from the group consisting of hydrogen, chlorine and cyano;
R³ is fluorine;
R⁴ is hydrogen;
R⁵ is methyl;
R^{a} and R^{b} are each independently selected from the group consisting of hydrogen, methyl, ethyl and propyl;
q is 1;
p is 1;
n is 1 or 2.

In a preferred embodiment, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, R^{a}R^{b}N-C(O)- and 5- to 6-membered heteroaryl, wherein the heteroaryl is optionally substituted with a substituent independently selected from the group consisting of hydroxy-C₁₋₄ alkyl- and R^{a}R^{b}N-C(O)-C₁₋₃ alkyl-;
preferably, R¹ is selected from the group consisting of hydrogen, NH₂-C(O)-, 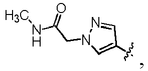 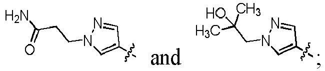
X is selected from N and CH;
R² is selected from the group consisting of hydrogen, chlorine and cyano;
R³ is fluorine;
R⁴ is hydrogen;
R⁵ is methyl;
R^{a} and R^{b} are each independently selected from the group consisting of hydrogen, methyl, ethyl and propyl;
q is 1;
p is 1;
n is 1 or 2.

In some embodiments, the present invention provides the compound of formula (I) as described above, or a pharmaceutically acceptable salt, stereoisomer, tautomer, polymorph, solvate, N-oxide, isotopically labeled compound, metabolite or prodrug thereof, wherein:
R is 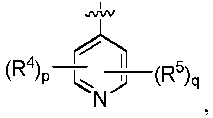
preferably, R is 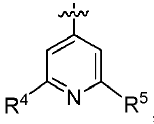 , and wherein at least one of R⁴ and R⁵ is halogen.

In other words, the compound of formula (I) has the structure of formula (Ib): 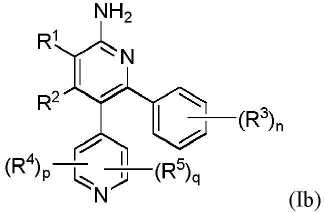 where R¹, R², R³, R⁴, R⁵, n, p and q are as defined above;
preferably the structure of formula (Ic): 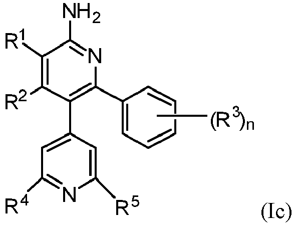 where R¹, R², R³, R⁴, R⁵, and n are as defined above.

In some embodiments of the compound of formula (Ib) or (Ic), R¹ is preferably selected from pyrazolyl and oxadiazolyl optionally substituted with a substituent independently selected from the group consisting of hydroxy-C₁₋₄ alkyl- (for example, hydroxy-C₁₋₃ alkyl-), R^{a}R^{b}N-C(O)-C₁₋₃ alkyl- and R^{a}R^{b}N-C(O)-;
More preferably, R¹ is selected from pyrazolyl and oxadiazolyl optionally substituted with a substituent independently selected from the group consisting of hydroxymethyl, hydroxyethyl, OH-(CH₂)₃-, OH-CH(CH₃)-CH₂-, OH-C(CH₃)₂-CH₂-, NH₂-C(O)-CH₂-, NH₂-C(O)-CH₂CH₂-, NH(CH₃)-C(O)-CH₂-, NH(CH₃)-C(O)-CH₂CH₂-, N(CH₃)₂-C(O)-CH₂-, N(CH₃)₂-C(O)-CH₂CH₂-, NH₂-C(O)-, NH(CH₃)-C(O)- and N(CH₃)₂-C(O)-;
more preferably, R¹ is selected from 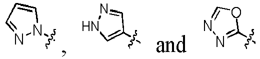 optionally substituted with a substituent independently selected from the group consisting of hydroxymethyl, hydroxyethyl, OH-CH(CH₃)-CH₂-, OH-C(CH₃)₂-CH₂-, NH₂-C(O)-CH₂-, NH₂-C(O)-CH₂CH₂-, NH(CH₃)-C(O)-CH₂-, NH(CH₃)-C(O)-CH₂CH₂-, NH₂-C(O)- and NH(CH₃)-C(O)-;
more preferably, R¹ is selected from 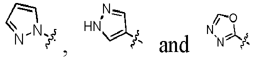 optionally substituted with a substituent independently selected from the group consisting of OH-C(CH₃)₂-CH₂-, NH₂-C(O)-CH₂-, NH₂-C(O)-CH₂CH₂- and NH₂-C(O)-;
more preferably, R¹ is selected from

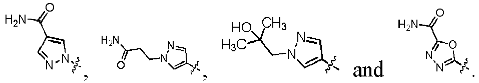

In some embodiments of the compound of formula (Ib) or (Ic), R² is hydrogen; and R³ is halogen, preferably fluorine.

In some embodiments of the compound of formula (Ib) or (Ic), R⁴ is selected from C₁₋₃ alkyl, preferably methyl; and R⁵ is selected from halogen, preferably chlorine.

In some embodiments of the compound of formula (Ib) or (Ic), R^{a} and R^{b} are each independently hydrogen.

In some embodiments of the compound of formula (Ib), R¹ is selected from

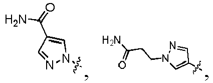

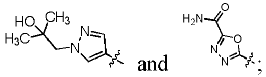

R² is hydrogen; R³ is halogen; R⁴ is selected from C₁₋₃ alkyl; R⁵ is selected from halogen; and n, p and q are each 1.

In some embodiments of the compound of formula (Ic), R¹ is selected from 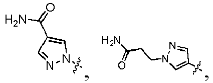 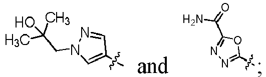 R² is hydrogen; R³ is halogen; R⁴ is selected from C₁₋₃ alkyl; R⁵ is selected from halogen; and n is 1.

In some embodiments of the compound of formula (Ib), R¹ is selected from 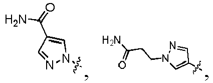 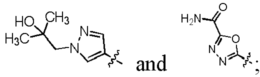 R² is hydrogen; R³ is fluorine; R⁴ is methyl; R⁵ is chlorine; and n, p, and q are each 1.

In some embodiments of the compound of formula (Ic), R¹ is selected from 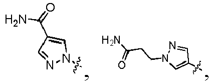 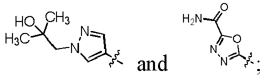 ; R² is hydrogen; R³ is fluorine; R⁴ is methyl; R⁵ is chlorine; and n is 1.

In a second aspect, the present invention provides a compound of formula (Ia) as defined below, or a pharmaceutically acceptable salt, stereoisomer, tautomer, polymorph, solvate, N-oxide, isotopically labeled compound, metabolite or prodrug thereof: 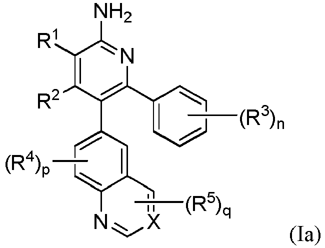 wherein:
X is N or CH;
R¹ is selected from the group consisting of hydrogen, halogen, cyano, C₁₋₆ alkyl, C₁₋₆ haloalkyl, C₃₋₆ cycloalkyl, carboxyl, C₁₋₆ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl- and R^{a}R^{b}N-C(O)-C₁₋₆ alkyl-;
R² is selected from the group consisting of hydrogen, halogen, cyano, C₁₋₆ alkyl, C₁₋₆ haloalkyl, carboxyl and R^{a}R^{b}N-C(O)-;
R³ is selected from the group consisting of hydrogen, halogen, cyano, C₁₋₆ alkyl, C₁₋₆ haloalkyl and C₁₋₆ alkoxyl;
R⁴ is selected from the group consisting of hydrogen, halogen, C₁₋₆ alkyl, and C₁₋₆ haloalkyl;
R⁵ is selected from the group consisting of hydrogen, halogen, C₁₋₆ alkyl, and C₁₋₆ haloalkyl;
R^{a} and R^{b} are each independently selected from the group consisting of hydrogen, C₁₋₆ alkyl, C₃₋₆ cycloalkyl, hydroxy-C₁₋₆ alkyl-, C₁₋₆ alkoxy-C₁₋₆ alkyl- and 5- to 6-membered heterocyclyl-C₁₋₆ alkyl-;
n is selected from 0, 1 or 2;
p is selected from 0, 1 or 2; and
q is selected from 0, 1 or 2.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, carboxy, C₁₋₃ alkyl-OC(O)- and R^{a}R^{b}N-C(O);
preferably, R¹ is selected from the group consisting of hydrogen, halogen, cyano, carboxy, CH₃-OC(O)-, NH₂-C(O)- and NH(CH₃)-C(O)-;
preferably, R¹ is selected from the group consisting of hydrogen, bromine, cyano, carboxyl, CH₃-OC(O)-, NH₂-C(O)- and NH(CH₃)-C(O)-.

In yet other embodiments, R¹ is hydrogen.

In yet other embodiments, R¹ is halogen; preferably, R¹ is fluorine, chlorine, bromine or iodine; preferably, R¹ is bromine.

In yet other embodiments, R¹ is cyano.

In yet other embodiments, R¹ is carboxyl.

In yet other embodiments, R¹ is C₁₋₃ alkyl-OC(O)-; preferably, R¹ is CH₃-OC(O)-.

In yet other embodiments, R¹ is R^{a}R^{b}N-C(O)-; preferably, R¹ is selected from NH₂-C(O)- and NH(CH₃)-C(O)-. In yet other embodiments, R¹ is NH₂-C(O)-.

In yet other embodiments, R¹ is NH(CH₃)-C(O)-.

In yet other embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of 5- to 6-membered heterocyclyl and 5- to 6-membered heteroaryl, wherein the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆alkoxy-C₁₋₆ alkyl-, R^{a}R^{b}N-C₁₋₆ alkyl- and R^{a}R^{b}N-C(O)-C₁₋₆ alkyl-;
preferably, R¹ is selected from the group consisting of 5- to 6-membered heterocyclyl and 5- to 6-membered heteroaryl, wherein the heterocyclyl and the heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₃ alkyl-, C₁₋₃ alkoxy-C₁₋₃ alkyl-, R^{a}R^{b}N-C₁₋₃ alkyl- and R^{a}R^{b}N-C(O)-C₁₋₃ alkyl-;
preferably, R¹ is 5- to 6-membered heteroaryl, wherein the heteroaryl is optionally substituted with a substituent independently selected from the group consisting of C₁₋₃ alkyl;
preferably, R¹ is 5- to 6-membered heteroaryl, wherein the heteroaryl is optionally substituted with a substituent independently selected from the group consisting of methyl, ethyl, n-propyl and isopropyl.

In such embodiments, the 5- to 6-membered heteroaryl is selected from the group consisting of pyrazolyl, triazolyl and tetrazolyl; preferably, the 5- to 6-membered heteroaryl is selected from pyrazolyl and tetrazolyl.

In some preferred embodiments, R¹ is selected from

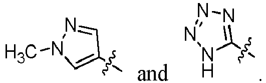

In yet other preferred embodiments, R¹ is

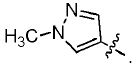

In yet other preferred embodiments, R¹ is

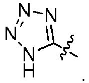

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R² is selected from the group consisting of hydrogen, halogen, cyano, C₁₋₃ alkyl, C₁₋₃ haloalkyl, carboxyl and R^{a}R^{b}N-C(O)-;
preferably, R² is selected from the group consisting of hydrogen, fluorine, chlorine, bromine, iodine, cyano, methyl, ethyl, n-propyl, isopropyl, carboxyl and NH₂-C(O)-;
preferably, R² is hydrogen.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R³ is selected from the group consisting of hydrogen, halogen, cyano, C₁₋₃ alkyl, C₁₋₃ haloalkyl and C₁₋₃ alkoxyl;
preferably, R³ is selected from the group consisting of fluorine, chlorine, bromine and iodine;
preferably, R³ is fluorine.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R⁴ is selected from the group consisting of hydrogen, halogen, C₁₋₃ alkyl, and C₁₋₃ haloalkyl;
preferably, R⁴ is selected from the group consisting of hydrogen, fluorine, chlorine, bromine, iodine, C₁₋₃ alkyl, and C₁₋₃ haloalkyl;
preferably, R⁴ is selected from the group consisting of hydrogen, fluorine, chlorine, bromine, iodine, methyl, ethyl, n-propyl, and isopropyl;
preferably, R⁴ is hydrogen.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R⁵ is selected from the group consisting of hydrogen, halogen, C₁₋₃ alkyl, and C₁₋₃ haloalkyl;
preferably, R⁵ is selected from the group consisting of hydrogen, fluorine, chlorine, bromine, iodine, C₁₋₃ alkyl, and C₁₋₃ haloalkyl;
preferably, R⁵ is selected from the group consisting of hydrogen, fluorine, chlorine, bromine, iodine, methyl, ethyl, n-propyl, and isopropyl;
preferably, R⁵ is methyl.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R⁴ is hydrogen, and R⁵ is methyl.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R^{a} and R^{b} are each independently selected from the group consisting of hydrogen, C₁₋₃ alkyl, C₃₋₆ cycloalkyl, hydroxy C₁₋₃ alkyl-, C₁₋₃ alkoxy-C₁₋₃ alkyl- and 5- to 6-membered heterocyclyl-C₁₋₃ alkyl-;
preferably, R^{a} and R^{b} are each independently selected from hydrogen and C₁₋₃ alkyl;
preferably, R^{a} and R^{b} are each independently selected from hydrogen and methyl;
preferably, R^{a} is hydrogen, and R^{b} is hydrogen or methyl.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
n is 0 or 1; preferably, n is 1.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
p is 0 or 1.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
q is 0 or 1; preferably, q is 1.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
X is N or CH; preferably, X is N.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl- and R^{a}R^{b}N-C(O)-C₁₋₆ alkyl-;
preferably, R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₃ alkyl-, C₁₋₃ alkoxy-C₁₋₃ alkyl-, R^{a}R^{b}N-C₁₋₃ alkyl- and R^{a}R^{b}N-C(O)-C₁₋₃ alkyl-;
preferably, R¹ is selected from the group consisting of hydrogen, halogen, cyano, carboxyl, C₁₋₃ alkyl-OC(O)-, R^{a}R^{b}N-C(O)- and 5- to 6-membered heteroaryl, wherein the heteroaryl is optionally substituted with a substituent independently selected from the group consisting of C₁₋₃ alkyl;
preferably, R¹ is selected from the group consisting of hydrogen, halogen, cyano, carboxyl, CH₃-OC(O)-, NH₂-C(O)-, NH(CH₃)-C(O)- and 5- to 6-membered heteroaryl, wherein the heteroaryl is optionally substituted with a substituent independently selected from the group consisting of C₁₋₃ alkyl;
preferably, R¹ is NH₂-C(O)-; and X is selected from N and CH; preferably, X is N.

In some embodiments according to the second aspect, the present invention provides the compound of formula (Ia) as described above, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl- and R^{a}R^{b}N-C(O)-C₁₋₆ alkyl-;
preferably, R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₃ alkyl-, C₁₋₃ alkoxy-C₁₋₃ alkyl-, R^{a}R^{b}N-C₁₋₃ alkyl- and R^{a}R^{b}N-C(O)-C₁₋₃ alkyl-;
preferably, R¹ is selected from the group consisting of hydrogen, halogen, cyano, carboxyl, C₁₋₃ alkyl-OC(O)-, R^{a}R^{b}N-C(O)- and 5- to 6-membered heteroaryl, wherein the heteroaryl is optionally substituted with a substituent independently selected from the group consisting of C₁₋₃ alkyl;
preferably, R¹ is selected from the group consisting of hydrogen, halogen, cyano, carboxyl, CH₃O-C(O)-, NH₂-C(O)-, NH(CH₃)-C(O)- and 5- to 6-membered heteroaryl, wherein the heteroaryl is optionally substituted with a substituent independently selected from the group consisting of C₁₋₃ alkyl;
preferably, R¹ is NH₂-C(O)-; and X is N;
R⁵ is selected from the group consisting of hydrogen, halogen, C₁₋₃ alkyl and C₁₋₃ haloalkyl; preferably, R⁵ is selected from the group consisting of hydrogen, fluorine, chlorine, bromine, iodine, C₁₋₃ alkyl and C₁₋₃ haloalkyl; preferably, R⁵ is selected from the group consisting of hydrogen, fluorine, chlorine, bromine, iodine, methyl, ethyl, propyl and isopropyl; preferably, R⁵ is methyl; and q is 1.

In a preferred embodiment, R² is hydrogen.

In a preferred embodiment, R³ is selected from the group consisting of fluorine, chlorine, bromine and iodine; preferably, R³ is fluorine; and n is 1.

In a more preferred embodiment, R² is hydrogen; R³ is selected from the group consisting of fluorine, chlorine, bromine and iodine; preferably, R³ is fluorine; and n is 1.

In some embodiments, the compound of the invention as described above is selected from:

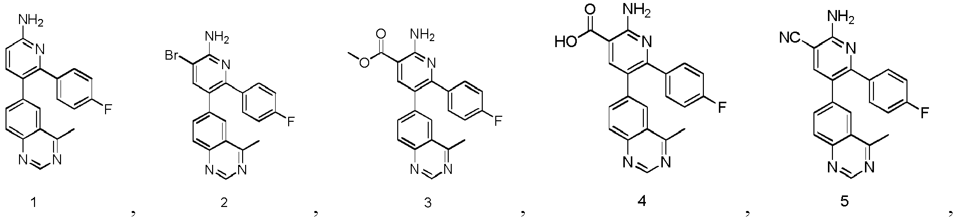

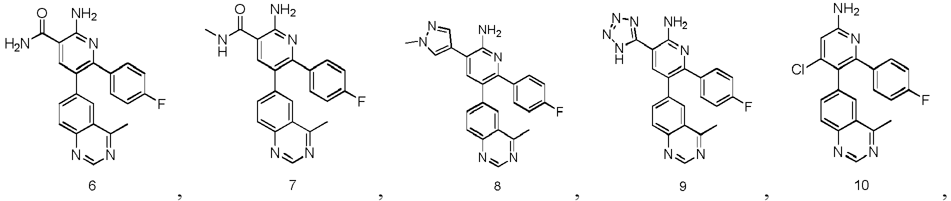

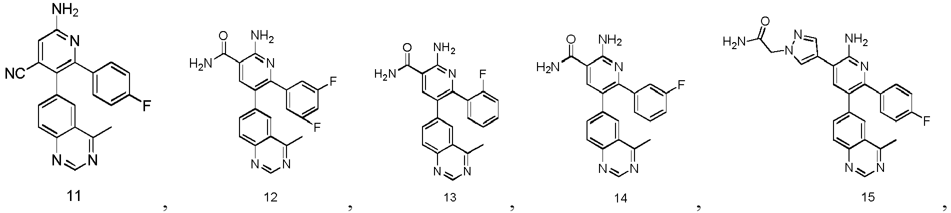

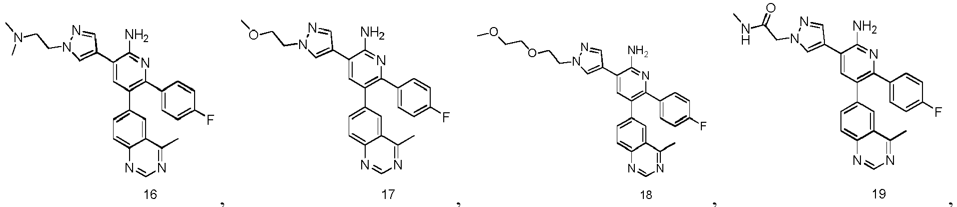

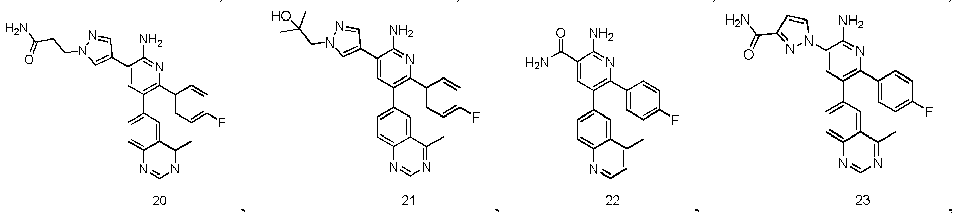

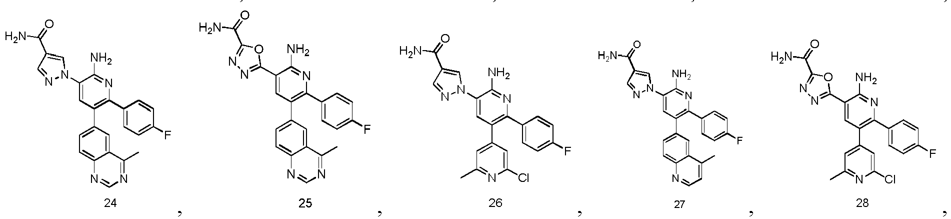

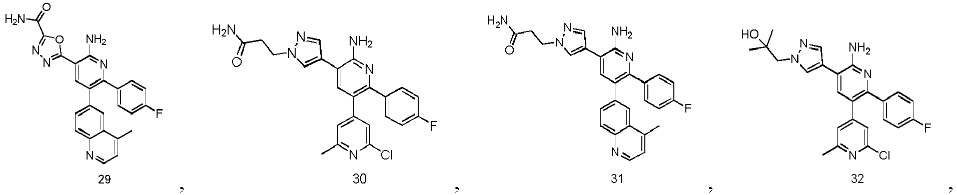

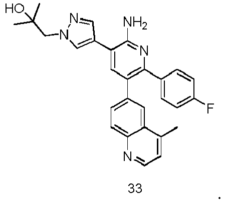

### Preparation Method

In a first aspect, the present invention provides a method for preparing a compound of formula (Ia), wherein R¹ is H, Br, CH₃-O-C(O)-, carboxyl or 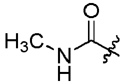 (i.e., a compound of formula (Ia-3), (Ia-4), (Ia-5), (Ia-6) or (Ia-7) in the following Scheme 1): 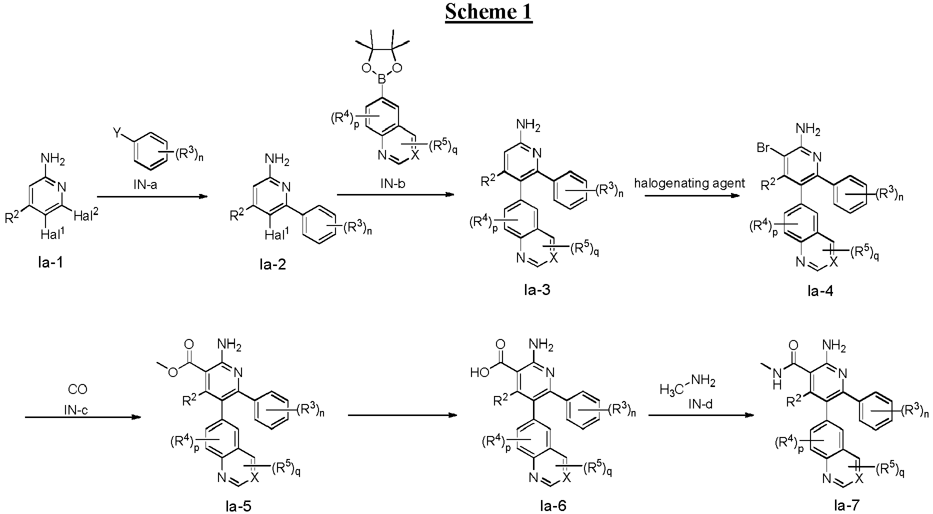 wherein R², R³, R⁴, R⁵, X, p, q, n are as defined above, Hal¹ and Hal² are independently selected from halogen, such as Cl, Br or I; Y is a boronic acid or boronate group, preferably -B(OH)₂ or 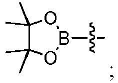 the method comprising the following steps:

### (1) reacting compound Ia-1 with compound IN-a to obtain compound Ia-2;

Compound Ia-2 is obtained via a coupling reaction between Compound Ia-1 and compound IN-a. The coupling reaction is preferably carried out in the presence of a metal catalyst and a base. Preferably, the metal catalyst is a palladium metal catalyst, such as tetra(triphenylphosphine)palladium, [1,1'-bis(diphenylphosphino)ferrocene]palladium dichloride, [1,1'-bis(diphenylphosphino)ferrocene] palladium dichloride dichloromethane complex, bis(triphenylphosphine)palladium dichloride, and palladium acetate, preferably tetra(triphenylphosphine)palladium. The base is an inorganic base, such as potassium carbonate, cesium carbonate, sodium carbonate, sodium bicarbonate, potassium bicarbonate, preferably sodium carbonate. Preferably, the coupling reaction is carried out in a suitable organic solvent, or a mixed solvent of an organic solvent and water, wherein the organic solvent may be selected from the group consisting of 1,4-dioxane, *N*,*N*-dimethylformamide, methanol, ethanol, toluene, and a mixed solvent of the above organic solvent(s) and water, for example, a mixed solvent of toluene, methanol and water. Preferably, the coupling reaction is carried out under a suitable protective atmosphere (for example, a nitrogen atmosphere). Preferably, the coupling reaction is carried out at a suitable temperature which may be 70-100°C, preferably 95°C. Preferably, the coupling reaction is carried out for a suitable period of time which may be 1-24 hours, such as 11 hours.

### (2) reacting compound Ia-2 with compound IN-b to obtain compound Ia-3;

Compound Ia-3 is obtained via a coupling reaction between compound Ia-2 and compound IN-b. The coupling reaction is preferably carried out in the presence of a metal catalyst and a base. Preferably, the metal catalyst is a palladium metal catalyst, such as tetra(triphenylphosphine)palladium, [1,1'-bis(diphenylphosphino)ferrocene]palladium dichloride, [1,1'-bis(diphenylphosphino)ferrocene] palladium dichloride dichloromethane complex, bis(triphenylphosphine)palladium dichloride, and palladium acetate, preferably tetra(triphenylphosphino)palladium. The base is an inorganic base, such as potassium carbonate, cesium carbonate, sodium carbonate, sodium bicarbonate, potassium bicarbonate, preferably potassium carbonate. Preferably, the coupling reaction is carried out in a suitable organic solvent, wherein the organic solvent may be selected from the group consisting of 1,4-dioxane, *N*,*N*-dimethylformamide, methanol, ethanol, toluene, and a mixed solvent of the above organic solvent(s) and water, for example, a mixed solvent of 1,4-dioxane and water. Preferably, the coupling reaction is carried out under a suitable protective atmosphere (for example, a nitrogen atmosphere). Preferably, the coupling reaction is carried out at a suitable temperature which may be 70-100°C, preferably 95°C. Preferably, the coupling reaction is carried out for a suitable period of time which may be 1-24 hours, preferably 12 hours.

### (3) reacting compound Ia-3 with a halogenating agent to obtain compound Ia-4;

Compound Ia-3 is reacted with the halogenating agent in a solvent to obtain compound Ia-4. The halogenating agent used may be a brominating agent such as *N*-bromosuccinimide or bromine, preferably N-bromosuccinimide. The solvent used can merely meet the requirement that it does not inhibit the reaction and can dissolve starting materials to a certain extent, and may be, for example, *N*,*N*-dimethylformamide, *N*-methylpyrrolidone, methanol, ethanol, tetrahydrofuran, 1,4-dioxane and dimethoxyethane, preferably *N*,*N*-dimethylformamide. The reaction temperature is generally -20°C to room temperature, preferably room temperature. The reaction duration is generally 1-6 hours, preferably 2 hours.

### (4) reacting compound Ia-4 with compound IN-c to obtain compound Ia-5;

Compound Ia-5 is obtained from compound Ia-4 via a carbonylation reaction. The carbonylation reaction is preferably carried out in the presence of a metal catalyst and a base. The metal catalyst may be selected from the group consisting of tetra(triphenylphosphine)palladium, [1,1'-bis(diphenylphosphino) ferrocene]palladium dichloride, [1,1'-bis(diphenylphosphino)ferrocene]palladium dichloride dichloromethane complex, bis(triphenylphosphine)palladium dichloride, and palladium acetate, preferably [1,1'-bis(diphenylphosphine)ferrocene]palladium dichloride. The base is selected from triethylamine and diisopropylethylamine, preferably triethylamine. The carbonylation reaction is carried out in a suitable organic solvent, wherein the organic solvent may be selected from the group consisting of 1,4-dioxane, *N*,*N*-dimethylformamide, methanol, toluene, and a mixed solvent of the above organic solvents, for example, a mixed solvent of methanol and *N*,*N*-dimethylformamide. The reaction temperature is generally 80°C to 120°C, preferably 100°C. The reaction duration is generally 12-20 hours, preferably 16 hours.

### (5) hydrolyzing compound Ia-5 to obtain compound Ia-6;

The hydrolysis is carried out preferably in the presence of a base and in a suitable solvent. The base is an inorganic base selected from the group consisting of sodium hydroxide, potassium hydroxide and lithium hydroxide, preferably sodium hydroxide. The solvent is selected from the group consisting of methanol, ethanol, tetrahydrofuran and 1,4-dioxane, preferably methanol. The reaction temperature is generally 25°C to 60°C, preferably 60°C. The reaction duration is generally 4-10 hours, preferably 5 hours.

### (6) reacting compound Ia-6 with compound IN-d to obtain a compound of formula (Ia-7).

The condensation reaction between compound Ia-6 and compound IN-d gives the compound of formula (Ia-7). The condensation reaction is preferably carried out in the presence of a condensing agent. The condensing agent is selected from the group consisting of *N*,*N'-*carbonyldiimidazole, 2-(7-azobenzotriazol-1-yl)-*N,N,N',N'*-tetramethyluronium hexafluorophosphate, benzotriazol-1-yloxy tris(dimethylamino)phosphonium hexafluorophosphate and 1-(3-dimethylaminopropyl)-3-ethyl carbodiimide hydrochloride, preferably *N,N'*-carbonyldiimidazole. The condensation reaction is carried out in a suitable organic solvent, wherein the organic solvent may be selected from the group consisting of tetrahydrofuran, dichloromethane, 1,4-dioxane, and *N,N*-dimethylformamide, preferably tetrahydrofuran. The reaction temperature is preferably room temperature (25-30°C), and the reaction duration is generally 2-6 hours, preferably 3 hours.

In a second aspect, the present invention further provides a method for preparing a compound of formula (Ia), wherein R¹ is cyano or NH₂-C(O)- (i.e., a compound of formula (Ia-8) or (Ia-9) in the following scheme 2): 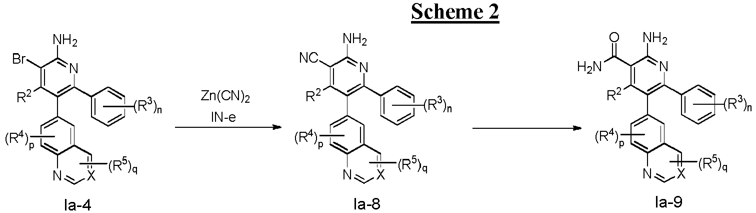 wherein R², R³, R⁴, R⁵, X, p, q, and n are as defined above;
the method comprising the following steps:

### (1) reacting compound Ia-4 with compound IN-e to obtain compound Ia-8;

Compound Ia-8 is obtained via the coupling reaction between compound Ia-4 and compound IN-e. The coupling reaction is preferably carried out in the presence of a metal catalyst. The metal catalyst may be selected from the group consisting of tris(dibenzylideneacetone)dipalladium, tetra(triphenylphosphine) palladium, [1,1'-bis(diphenylphosphino)ferrocene]palladium dichloride, [1,1'-bis(diphenylphosphino) ferrocene]palladium dichloride dichloromethane complex, bis(triphenylphosphine)palladium dichloride, and palladium acetate, preferably tris(dibenzylideneacetone)dipalladium. The coupling reaction is carried out in a suitable organic solvent, wherein the organic solvent may be selected from the group consisting of 1,4-dioxane, *N,N*-dimethylformamide, methanol, ethanol, and toluene, preferably *N,N*-dimethylformamide. Preferably, the coupling reaction is carried out under a suitable protective atmosphere (for example, a nitrogen atmosphere). The reaction temperature is generally 90°C to 140°C, preferably 120°C. Generally, heating is done by microwave. The reaction duration is generally 1-4 hours, preferably 2 hours.

### (2) reacting compound Ia-8 to obtain a compound of formula (Ia-9).

Compound Ia-8 is reacted with a base in a solvent to obtain the compound of formula (Ia-9). The base may be selected from the group consisting of potassium carbonate, sodium carbonate, cesium carbonate, sodium hydroxide and potassium hydroxide, preferably potassium carbonate. The reaction is carried out in a suitable organic solvent, wherein the organic solvent may be selected from the group consisting of toluene, 1,4-dioxane, dimethyl sulfoxide, and water, preferably dimethyl sulfoxide. The reaction temperature is preferably room temperature (25-30°C). The reaction duration is generally 2-10 hours, preferably 2 hours. The reaction may be carried out in the presence of hydrogen peroxide.

In a third aspect, the present invention further provides a method for preparing a compound of formula (Ia-10), as shown in Scheme 3: 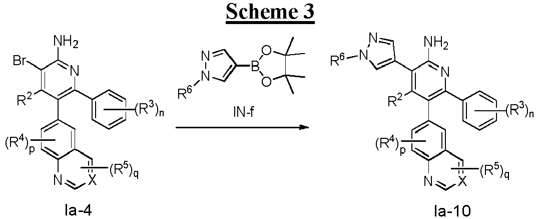 wherein:
R⁶ is selected from the group consisting of H, C₁₋₆ alkyl (such as methyl), C₁₋₆ alkoxy-C₁₋₆ alkyl (e.g., CH₃O-CH₂CH₂-), R^{a}R^{b}N-C₁₋₆ alkyl- (e.g., N(CH₃)₂-CH₂CH₂-), C₁₋₆ alkoxy-C₁₋₆ alkoxy-C₁₋₆ alkyl- (e.g., CH₃O-CH₂CH₂O-CH₂CH₂-) and C₁₋₆ alkoxy-C(O)-C₁₋₆ alkyl- (e.g., CH₃OC(O)-CH₂CH₂- or CH₃CH₂OC(O)-CH₂-); and
R², R³, R⁴, R⁵, R^{a}, R^{b}, X, p, q, and n are as defined above;
the method comprising the step of obtaining the compound of formula (Ia-10) via the coupling reaction between compound Ia-4 and compound IN-f.

The coupling reaction is preferably carried out in the presence of a metal catalyst and a base. Preferably, the metal catalyst is a palladium metal catalyst, such as tetra(triphenylphosphine)palladium, [1,1'-bis(diphenylphosphino)ferrocene]palladium dichloride, [1,1'-bis(diphenylphosphino)ferrocene] palladium dichloride dichloromethane complex, bis(triphenylphosphine)palladium dichloride, and palladium acetate, preferably [1,1'-bis(diphenylphosphino)ferrocene]palladium dichloride. The base is an inorganic base, such as potassium carbonate, cesium carbonate, sodium carbonate, sodium bicarbonate, potassium bicarbonate, preferably cesium carbonate. The coupling reaction is carried out in a suitable organic solvent, wherein the organic solvent may be selected from the group consisting of 1,4-dioxane, *N,N*-dimethylformamide, methanol, ethanol, toluene, and a mixed solvent of the above organic solvent(s) and water, preferably a mixed solvent of 1,4-dioxane and water. Preferably, the coupling reaction is carried out under a suitable protective atmosphere (for example, a nitrogen atmosphere). Preferably, the coupling reaction is carried out at a suitable temperature which may be 70-100°C, preferably 95°C. Preferably, the coupling reaction is carried out for a suitable period of time which may be 1-24 hours, preferably 12 hours.

According to the method shown in Scheme 4, a compound of formula (Ia-11) of the present invention may be obtained by ammonolysis reaction of the compound of formula (Ia-10) (wherein R⁶ is C₁₋₆ alkoxy-C(O)-C₁₋₆ alkyl-, e.g., CH₃OC(O)-CH₂CH₂- or CH₃CH₂OC(O)-CH₂-): 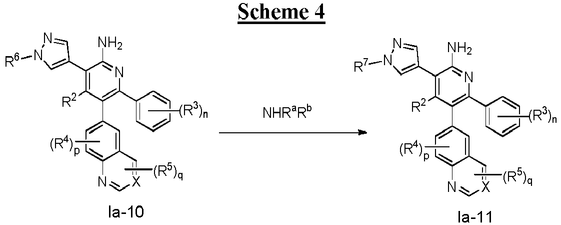 wherein:
R⁷ is R^{a}R^{b}N-C(O)-C₁₋₆ alkyl-, e.g., NH₂-C(O)-CH₂CH₂- or NH(CH₃)-C(O)-CH₂-; and
R², R³, R⁴, R⁵, R^{a}, R^{b}, X, p, q, and n are as defined above.

The ammonolysis reaction may be carried out by reacting the compound of formula (Ia-10) with NHR^{a}R^{b} (e.g., NH₃ or methylamine) in a suitable alcohol (e.g., methanol).

According to the methods described in Schemes 3 and 4, by replacing the compound of (Ia-4) with a compound of formula (Ib-1): 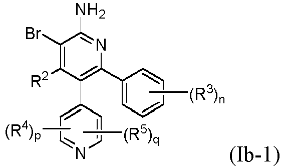 the compound of formula (Ib) of the present invention may be synthesized: 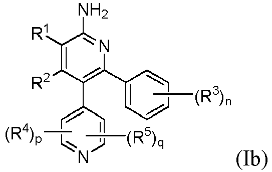 wherein:
R¹ is R^{a}R^{b}N-C(O)-C₁₋₆ alkyl- (such as NH₂-C(O)-CH₂CH₂-); and
R², R³, R⁴, R⁵, R^{a}, R^{b}, p, q, and n are as defined above.

In a fourth aspect, the present invention further provides a method for preparing a compound of (Ia), wherein R¹ is 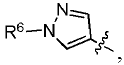 and R⁶ is OH-C(CH₃)₂-CH₂- (i.e., a compound of formula (Ia-13) in the following Scheme 5): 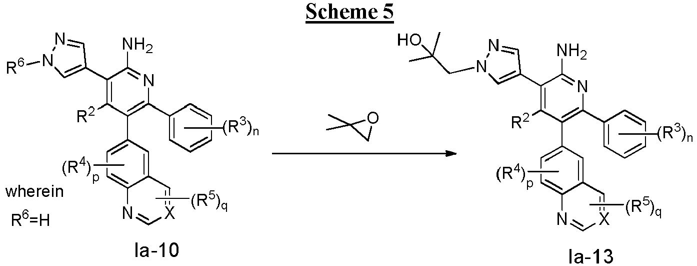 wherein R², R³, R⁴, R⁵, X, p, q, and n are as defined above;
the method comprising the step of producing compound Ia-13 by reacting compound Ia-10 with 2,2-dimethyloxirane in the presence of a base in a suitable organic solvent.

The base may be selected from the group consisting of potassium carbonate, sodium carbonate, cesium carbonate, sodium hydroxide and potassium hydroxide, preferably potassium carbonate. The organic solvent may be selected from polar aprotic solvents, preferably dimethyl sulfoxide. The reaction is carried out at a suitable temperature which may be 70-100°C, preferably 80°C. The reaction is carried out for a suitable period of time, such as 12 hours.

The compound of formula (Ib) of the present invention may be synthesized by replacing the compound of formula (Ia-4) with the compound of formula (Ib-1), according to the methods described in Schemes 3 and 5 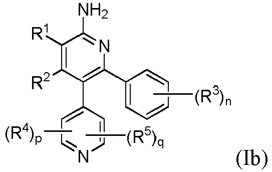 wherein:
R¹ is 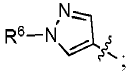
R⁶ is OH-C(CH₃)₂-CH₂-; and
R², R³, R⁴, R⁵, R^{a}, R^{b}, p, q, and n are as defined above.

In a fifth aspect, the present invention further provides a method for preparing a compound of formula (Ia-14): 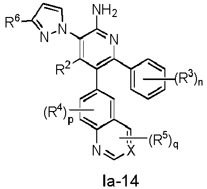 wherein:
R⁶ is R^{a}R^{b}N-C(O)- (e.g., NH₂-C(O)-); and
R², R³, R⁴, R⁵, R^{a}, R^{b}, X, p, q, and n are as defined above;
the method comprising the following steps:
   (1) reacting compound Ia-4 with the compound 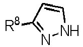 to obtain a compound of formula (Ia-15); 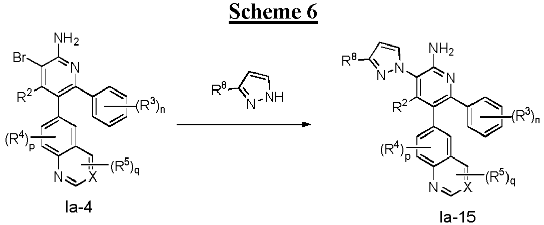 wherein:
      R⁸ is C₁₋₆ alkyl-OC(O)-, preferably C₁₋₃ alkyl-OC(O)-, e.g., EtOC(O)-; and
      R², R³, R⁴, R⁵, X, p, q, and n are as defined above.

The reaction is carried out in the presence of a suitable base. The base may be selected from the group consisting of potassium carbonate, sodium carbonate, cesium carbonate, sodium bicarbonate and potassium bicarbonate, preferably cesium carbonate. The reaction may be carried out in a suitable organic solvent. The organic solvent may be selected from polar aprotic solvents, preferably *N,N-*dimethylformamide. The reaction is carried out under a protective atmosphere (e.g., a nitrogen atmosphere). The reaction is carried out in the presence of a suitable catalyst which is, for example, copper iodide. The reaction is carried out at a suitable temperature which may be 80-120°C, preferably 110°C. Preferably, the coupling reaction is carried out for a suitable period of time, such as 5 hours.

(2) obtaining the compound of formula (Ia-14) by the ammonolysis reaction of the compound of formula (Ia-15).

The ammonolysis reaction may be carried out as described in Scheme 4.

According to the method described above, the compound of formula (Ia-16) may be obtained by replacing the compound 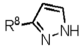 in the step (1) above with a compound 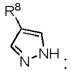 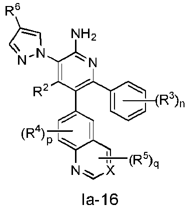 wherein:
R⁶ is R^{a}R^{b}N-C(O)- (e.g., NH₂-C(O)-); and
R², R³, R⁴, R⁵, R^{a}, R^{b}, X, p, q, and n are as defined above.

According to the method described above, the compound of formula (Ib) of the present invention may be synthesized by replacing the compound of formula (Ia-4) with the compound of formula (Ib-1): 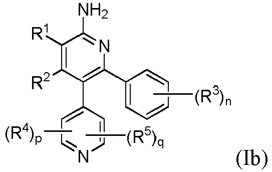 wherein:
R¹ is 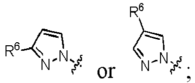
R⁶ is R^{a}R^{b}N-C(O)-, e.g., NH₂-C(O)-; and
R², R³, R⁴, R⁵, R^{a}, R^{b}, p, q, and n are as defined above.

In a sixth aspect, the present invention further provides a method for preparing a compound of formula (Ia-20): 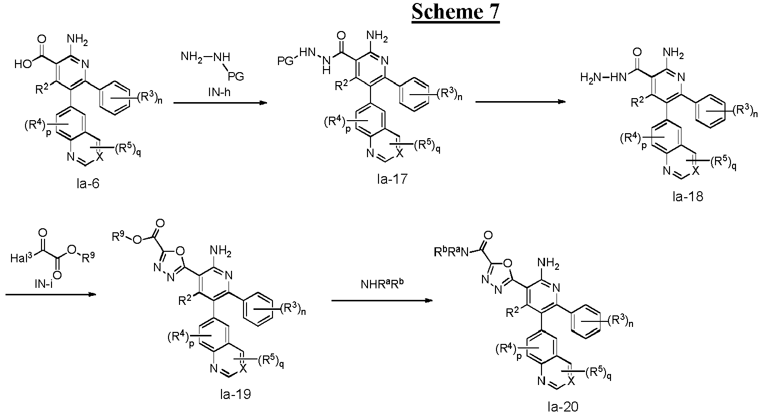 wherein:
R², R³, R⁴, R⁵, R^{a}, R^{b}, X, p, q, and n are as defined above;
PG is an amino protecting group, such as Ts, benzoyl, Cbz, Alloc, methoxycarbonyl, ethoxycarbonyl or tert-butoxycarbonyl (Boc), preferably Boc;
Hal3 is selected from halogen, such as Cl, Br or I, preferably Cl;
R⁹ is C₁₋₆ alkyl, preferably C₁₋₃ alkyl, such as Et;
the method comprising the following steps:

### (1) reacting compound Ia-6 with compound IN-h to obtain compound Ia-17;

The condensation reaction between compound Ia-6 and compound IN-h gives compound Ia-17. The condensation reaction is preferably carried out in the presence of a condensing agent. The condensing agent is selected from the group consisting of *N,N*'-carbonyldiimidazole, 2-(7-azobenzotriazol-1-yl)-*N,N,N',N'-*tetramethyluronium hexafluorophosphate, benzotriazol-1-yloxytris(dimethylamino)phosphonium hexafluorophosphate and 1-(3-dimethylaminopropyl)-3-ethylcarbodiimide hydrochloride, preferably *N,N'-*carbonyldiimidazole. The condensation reaction is carried out in a suitable organic solvent. The organic solvent may be selected from the group consisting of tetrahydrofuran, dichloromethane, 1,4-dioxane, and *N,N-*dimethylfonnamide, preferably *N,N*-dimethylformamide. The reaction temperature is preferably room temperature (25-30°C), and the reaction duration is for example 12 hours.

### (2) obtaining compound Ia-18 through a deprotection reaction of compound Ia-17;

The deprotection reaction may be carried out by reacting compound Ia-17 (such as a solution of compound Ia-17 in an alcohol (such as methanol)) with a suitable protonic acid (such as hydrochloric acid or trifluoroacetic acid) in a suitable organic solvent (such as 1,4-dioxane). The reaction temperature is preferably room temperature (25-30°C), and the reaction duration is for example 16 hours.

### (3) reacting compound Ia-18 with compound IN-i to obtain compound Ia-19;

Compound Ia-18 and compound IN-i are subjected to substitution and dehydration reactions in one-pot to obtain compound Ia-19. The substitution reaction is preferably carried out in the presence of a base selected from the group consisting of triethylamine, potassium carbonate, *N,N-*diisopropylethylamine, preferably *N,N*-diisopropylethylamine. The dehydration reaction is preferably carried out in the presence of a dehydrating agent selected from 4-methylbenzenesulfonyl chloride, trifluoromethanesulfonic anhydride, and preferably 4-methylbenzenesulfonyl chloride. The substitution and dehydration reactions are carried out in a suitable organic solvent. The organic solvent may be selected from 1,4-dioxane and methylene chloride, preferably 1,4-dioxane. The reaction temperature is preferably 0°C to room temperature (25-30°C), and the reaction duration is for example 16 hours.

### (4) obtaining compound Ia-20 through an ammonolysis reaction of compound Ia-19.

The ammonolysis reaction may be carried out as described in Scheme 4.

According to the method as described above, by replacing compound of (Ia-6) with the compound of formula (Ib-2): 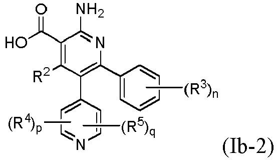 the compound of formula (Ib) of the present invention may be synthesized: 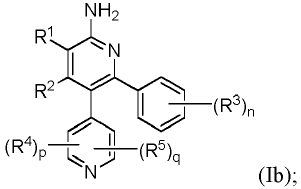 wherein:
R¹ is 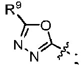
R¹⁰ is R^{a}R^{b}N-C(O)- (such as NH₂-C(O)-); and
R², R³, R⁴, R⁵, R^{a}, R^{b}, p, q, and n are as defined above.

In a seventh aspect, the present invention further provides a method for preparing a compound of formula (Ia), wherein R¹ is 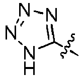 (i.e., a compound of formula Ia-12 in the following Scheme 8): 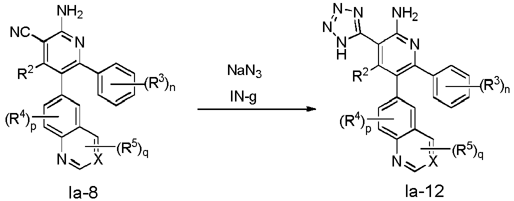 wherein R², R³, R⁴, R⁵, X, p, q, and n are as defined above;
the method comprising the step of carrying out a ring-closure reaction of compound Ia-8 with compound IN-g to obtain the compound of formula (Ia-12).

The ring-closure reaction is carried out in a suitable organic solvent. The organic solvent may be selected from the group consisting of *N,N*-dimethylformamide, *N*-methylpyrrolidone, n-butanol and toluene, preferably *N,N*-dimethylformamide. The reaction temperature is generally 80°C to 120°C, preferably 95°C. The reaction duration is generally 24-48 hours, preferably 48 hours.

### Pharmaceutical Composition

In some embodiments, the present invention provides a pharmaceutical composition or a pharmaceutical preparation, comprising a prophylactically or therapeutically effective amount of the compound of the present invention or a pharmaceutically acceptable salt, stereoisomer, tautomer, polymorph, solvate, N-oxide, isotopically labeled compound, metabolite or prodrug thereof, and one or more pharmaceutically acceptable carriers.

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition or the pharmaceutical preparation of the present invention includes, but is not limited to sterile liquids, such as water and oils, including those of petroleum, animal, vegetable or synthetic origin, such as peanut oil, soybean oil, mineral oil, sesame oil and the like.

The pharmaceutical composition may be in the form of, for example, a solid, semi-solid, liquid, or gaseous formulation.

Water is an exemplary carrier when the pharmaceutical composition is administered intravenously. Physiological saline as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g. Remington's Pharmaceutical Sciences (1990).

The pharmaceutical composition or the pharmaceutical preparation of the present invention can act systemically and/or topically. To this end, it can be administered through a suitable route, such as through injection, (intravenous, intraarterial, subcutaneous, intraperitoneal, intramuscular injection, including dripping), or transdermal administration, or administered orally, buccally, nasally, transmucosally, topically, as an ophthalmic formulation, or via inhalation.

For these routes of administration, the pharmaceutical composition of the present invention can be administered in a suitable dosage form.

The content or amount of the compound of the present invention in the pharmaceutical composition or the pharmaceutical preparation may be about 0.01 mg to about 1000 mg.

In some embodiments, the present invention provides a method for preparing the pharmaceutical composition of the present invention, comprising combining the compound of the present invention or a pharmaceutically acceptable salt, stereoisomer, tautomer, polymorph, solvate, N-oxide, isotopically labeled compound, metabolite or prodrug thereof, with one or more pharmaceutically acceptable carriers.

### Treatment Method and Use

In some embodiments, the present invention provides use of the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention in the preparation of a medicament for the prophylaxis or treatment of an adenosine A2a receptor related disease.

In some embodiments, the present invention provides the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention for inhibiting the activity of an adenosine A2a receptor.

In some embodiments, the present invention provides the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention for use in the prophylaxis or treatment of an adenosine A2a receptor related disease.

In some embodiments, the present invention provides a method for the prophylaxis or treatment of an adenosine A2a receptor related disease, comprising administering to a subject in need thereof an effective amount of the compound of the present invention or a pharmaceutically acceptable salt, stereoisomer, tautomer, polymorph, solvate, N-oxide, isotopically labeled compound, metabolite or prodrug thereof, or the pharmaceutical composition of the present invention.

In some embodiments, the adenosine A2a receptor related disease is a tumor, preferably the disease is cancer.

As used herein, the term "effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g. birds, amphibians, reptiles) and mammals, such as non-human primates, livestock and/or domesticated animals (such as sheep, dog, cat, cow, pig and the like).

### Specific Embodiments of the Invention

The embodiments of the present invention will be described in detail with reference to the examples below. However, those skilled in the art will understand that the following examples are only for the purpose of illustrating the present invention, and should not be construed as limiting the scope of the present invention. If specific conditions are not indicated in the examples, they are carried out under conventional conditions or conditions recommended by the manufacturers. Reagents or instruments used without indicating the manufacturers are all conventional products that are commercially available.

The structures of the compounds were determined by nuclear magnetic resonance (¹H NMR) or mass spectrometry (MS). ¹H NMR assays were conducted on JEOL Eclipse 400 spectrometer, the test solvent was deuterated methanol (CD₃OD), deuterated chloroform (CDCl₃) or hexadeuterated dimethyl sulfoxide (DMSO-*d*₆), and the internal standard was tetramethylsilane (TMS), and the chemical shift (δ) was expressed in parts per million (ppm).

The MS assays were conducted on Agilent (ESI) mass spectrometer (manufacturer: Agilent, model: Agilent 6120B).

Purification was conducted by preparative high-performance liquid chromatography on Agilent 1260, with the column Waters SunFire Prep C18 OBD (19 mm×150 mm×5.0 µm). The preparation of the preparative high-performance liquid chromatography was performed as follows:

### Preparation Method 1:

Chromatographic column temperature: 25°C; flow rate: 20.0 mL/min; detection wavelength: 214 nm; gradient elution: (0 min: 10% (v/v) A, 90% (v/v) B; 16.0 min: 90 % (v/v) A, 10% (v/v) B); mobile phase A: acetonitrile; mobile phase B: 0.05% (v/v) aqueous formic acidsolution.

### Preparation Method 2:

Chromatographic column temperature: 25°C; flow rate: 20.0 mL/min; detection wavelength: 214 nm; gradient elution: (0 min: 10% (v/v) A, 90% (v/v) B; 16.0 min: 90 % (v/v) A, 10% (v/v) B); mobile phase A: acetonitrile; mobile phase B: 0.05% aqueous ammonium bicarbonatesolution.

The silica gel plate used for thin layer chromatography (TLC) was aluminum plate from Merck (20 × 20 cm), and separation and purification by thin layer chromatography was conducted with GF 254 (1 mm thick) produced in Yantai.

The reaction was monitored by thin layer chromatography (TLC) or LC-MS, the developing solvent system included dichloromethane and methanol system, n-hexane and ethyl acetate system, as well as petroleum ether and ethyl acetate system, and was adjusted (by adjusting the volume ratio of the solvents, or by adding triethylamine, *etc*.) according to the polarity of the compound to be separated.

The microwave reaction was conducted by Biotage Initiator + (400 W, RT ∼ 300°C) microwave reactor.

Silica gel (200∼300 mesh) was normally used as a carrier in column chromatography. The eluent system included dichloromethane and methanol system, and petroleum ether and ethyl acetate system, and was adjusted (by adjusting the volume ratio of the solvents, or by adding a small amount of triethylamine, *etc*.) according to the polarity of the compound to be separated.

Unless specified otherwise, the reaction temperature in the following examples was room temperature (20°C-35°C).

The reagents used in the present invention were purchased from companies such as Acros Organics, Aldrich Chemical Company, and Tebo Chemicals.

The abbreviations as used in conventional synthesis methods and the synthesis examples of the compounds and intermediates of the present invention have the meanings shown below.

| Abbreviation | Meaning |
|---|---|
| RT | Room temperature |
| DMF | *N,N*-dimethylformamide |
| NBS | *N*-bromosuccinimide |
| dppf | 1,1'-bis(diphenylphosphino)ferrocene |
| TLC | Thin layer chromatography |
| Pd(dppf)Cl₂ | [1,1'-bis(diphenylphosphino)ferrocene]palladium dichloride |
| min | Minute |

### Preparation of Intermediates

### Intermediate Preparation Example 1: Preparation of 6-amino-2'-chloro-2-(4-fluorophenyl)-6'-methyl-[3,4'-bipyridine]-5-carboxylic acid (In-1)

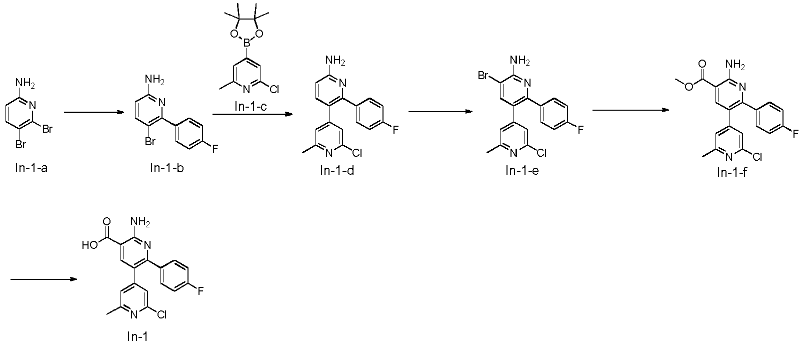

### Step 1: Preparation of 5-bromo-6-(4-fluorophenyl)pyridin-2-amine (In-1-b)

5,6-dibromopyridin-2-amine (**In-1-a**) (2 g, 7.94 mmol), 4-fluorophenylboronic acid (1.11 g, 7.94 mmol) and sodium carbonate (1.68 g, 15.88 mmol) were added to toluene (40 mL), methanol (4 mL) and water (8 mL), purged with nitrogen for three times, added with tetra(triphenylphosphine)palladium (459 mg, 0.4 mmol), and reacted at 95°C for 11 hours. After the reaction was complete, the reaction solution was cooled to room temperature, concentrated, and the residue was purified by silica gel column chromatography (eluent: ethyl acetate/petroleum ether = 1/3 (v/v)) to obtain the title compound of this step (2 g, yield: 94.3%).

MS m/z (ESI): 267.0 [M+H]⁺.

### Step 2: Preparation of 2'-chloro-2-(4-fluorophenyl)-6'-methyl-[3,4'-bipyridine]-6-amine (In-1-d)

5-bromo-6-(4-fluorophenyl)pyridin-2-amine (In-1-b) (1.14 g, 4.27 mmol), 2-chloro-6-methyl-4-(4,4,5 ,5-tetramethyl-1,3,2-dioxaborolan-2-yl)pyridine (In-1-c) (1.4 g, 5.55 mmol) and potassium carbonate (1.18 g, 8.54 mmol) were added to 1,4-dioxane (20 mL) and water (2 mL), purged with nitrogen for three times, added with tetra(triphenylphosphine)palladium (246.9 mg, 0.21 mmol), and reacted at 95°C for 12 hours. After the reaction was complete, the reaction solution was cooled to room temperature, filtered and concentrated, and the residue was purified by silica gel column chromatography (eluent: ethyl acetate/petroleum ether = 3/2 (v/v)) to obtain the title compound of this step (1.1 g, yield: 81.5%).

MS m/z (ESI): 314.1 [M+H]⁺.

### Step 3: Preparation of 5-bromo-2'-chloro-2-(4-fluorophenyl)-6'-methyl-[3,4'-bipyridine]-6-amine (In-1-e)

At 0°C, the solution of 2'-chloro-2-(4-fluorophenyl)-6'-methyl-[3,4'-bipyridine]-6-amine (In-1-c) (2.8 g, 9.08 mmol) in DMF (30 mL) was added with NBS (1.62 g, 9.08 mmol), and reacted at 25°C for 2 hours. The reaction solution was poured into water, stirred for 10 min, and subsequently extracted with ethyl acetate for three times. The organic phases were combined, dried over anhydrous sodium sulfate, and filtered. The filtrate was concentrated, and the residue was purified by silica gel column chromatography (eluent: ethyl acetate/petroleum ether = 1/2 (v/v)) to obtain the title compound of this step (3.0 g, yield: 83.4%).

MS m/z (ESI): 392.0 [M+H]⁺.

### Step 4: Preparation of methyl 6-amino-2'-chloro-2-(4-fluorophenyl)-6'-methyl-[3,4'-bipyridine]-5-carboxylate (In-1-f)

5-bromo-2'-chloro-2-(4-fluorophenyl)-6'-methyl-[3,4'-bipyridine]-6-amine (In-1-e) (1.2 g, 2.93 mmol), Pd(dppf)Cl₂ (119.63 mg, 0.15 mmol), triethylamine (888.45 mg, 8.80 mmol), methanol (3 mL) and DMF (10 mL) were added sequentially to a stainless steel high pressure reactor, which was flushed with a carbon monoxide atmosphere, and reacted at 100°C for 16 hours. The reaction was cooled to room temperature, filtered, washed, and dried to obtain the title compound of this step (980 mg, yield: 86.1%).

MS m/z (ESI): 372.1 [M+H]⁺.

### Step 5: Preparation of 6-amino-2'-chloro-2-(4-fluorophenyl)-6'-methyl-[3,4'-bipyridine]-5-carboxylic acid (In-1)

Methyl 6-amino-2'-chloro-2-(4-fluorophenyl)-6'-methyl-[3,4'-bipyridine]-5-carboxylate (In-1-f) (115 mg, 0.31 mmol) was added to methanol (5 mL), then added with 50% aqueous sodium hydroxide solution (247.17 mg, 6 mmol), and reacted at 60°C for 5 hours. To the reaction system was added dropwise 1 N diluted hydrochloric acid to adjust the pH to 4-5. The reaction solution was concentrated, and the residue was purified by high performance liquid chromatography (Preparation method 1) to obtain the title compound (60 mg, yield: 54.2%).

MS m/z (ESI): 358.1 [M+H]⁺.

### Intermediate Preparation Example 2: Preparation of 2-amino-6-(4-fluorophenyl)-5-(4-methyl quinolin-6-yl)nicotinic acid (In-2)

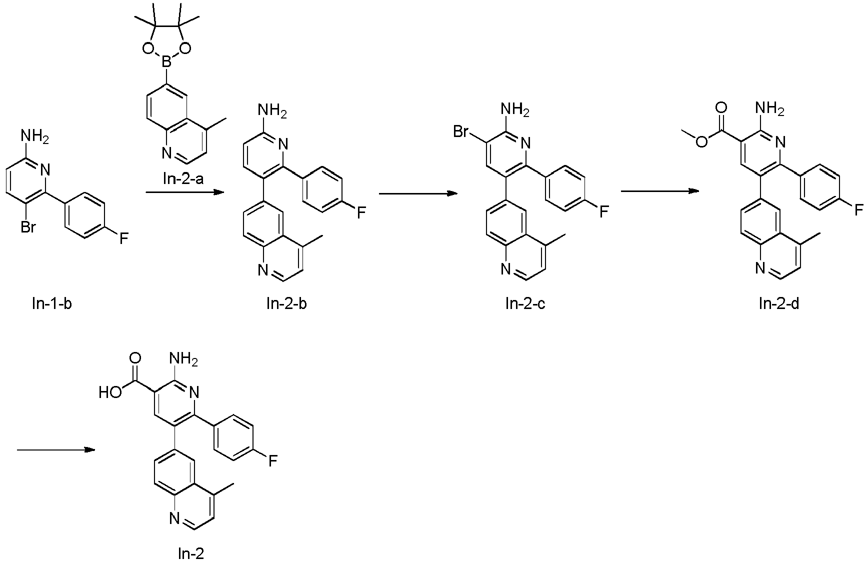

The title compound (49 mg, yield: 50.5%) was obtained according to the synthetic route of Intermediate preparation example 1, with replacing the starting material 2-chloro-6-methyl-4-(4,4,5,5-tetramethyl-1,3,2-dioxaborolan-2-yl)pyridine (In-1-c) in step 2 with 4-methyl-6-(4,4,5,5-tetramethyl-1,3,2-dioxaborolan-2-yl)quinoline (In-2-a).

MS m/z (ESI): 374.1[M+H]⁺.

### Preparation of the compounds of the invention

### Example 1: Preparation of 6-(4-fluorophenyl)-5-(4-methylquinazolin-6-yl)pyridin-2-amine

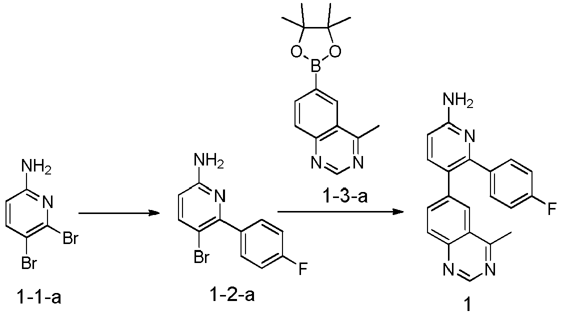

### Step 1: Preparation of 5-bromo-6-(4-fluorophenyl)pyridin-2-amine (1-2-a)

5,6-dibromopyridin-2-amine (1-1-a) (2 g, 7.94 mmol), 4-fluorophenylboronic acid (1.11 g, 7.94 mmol) and sodium carbonate (1.68 g, 15.88 mmol) were added to toluene (40 mL), methanol (4 mL) and water (8 mL), purged with nitrogen for three times, added with tetra(triphenylphosphine)palladium (459 mg, 0.4 mmol), and reacted at 95°C for 11 hours. After the reaction was complete, the reaction solution was cooled to room temperature, concentrated, and the residue was purified by silica gel column chromatography (eluent: ethyl acetate/petroleum ether = 1/3 (v/v)) to obtain the title compound of this step (2 g, yield: 94.3%).

MS m/z (ESI): 267.0 [M+H]⁺.

### Step 2: Preparation of 6-(4-fluorophenyl)-5-(4-methylquinazolin-6-yl)pyridin-2-amine (1)

5-bromo-6-(4-fluorophenyl)pyridin-2-amine (1-2-a) (1.14 g, 4.27 mmol), 4-methyl-6-(4,4,5,5-tetramethyl-1,3,2-dioxaborolan-2-yl)quinazoline (1-3-a) (1.5 g, 5.55 mmol) and potassium carbonate (1.18 g, 8.54 mmol) were added to 1,4-dioxane (20 mL) and water (2 mL), purged with nitrogen for three times, added with tetra(triphenylphosphine)palladium (246.9 mg, 0.21 mmol), and reacted at 95°C for 12 hours. After the reaction was complete, the reaction solution was cooled to room temperature, filtered and concentrated, and the residue was purified by silica gel column chromatography (eluent: ethyl acetate/petroleum ether = 3/2 (v/v)) to obtain the title compound (1.15 g, yield: 81.5%).

MS m/z (ESI): 331.1 [M+H]⁺.

¹H NMR (400 MHz, DMSO-*d*₆) δ: 9.05 (s, 1H), 8.01 (d, *J* = 1.6 Hz, 1H), 7.75 (d, *J* = 8.4 Hz, 1H), 7.68 (d, *J* = 8.4 Hz, 1H), 7.53 (dd, *J* = 8.8, 2.0 Hz, 1H), 7.30-7.27 (m, 2H), 7.06 (t, *J* = 9.2 Hz, 2H), 6.60 (d, *J* = 8.4 Hz, 1H), 6.31 (s, 2H), 2.79 (s, 3H).

### Example 2: Preparation of 3-bromo-6-(4-fluorophenyl)-5-(4-methylquinazolin-6-yl)pyridin-2-amine (2)

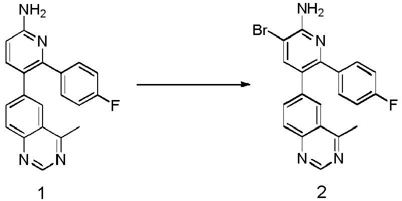

At 0°C, the solution of 6-(4-fluorophenyl)-5-(4-methylquinazolin-6-yl)pyridin-2-amine (1) (3 g, 9.08 mmol) in DMF (30 mL) was added with NBS (1.62 g, 9.08 mmol), and reacted at 25°C for 2 hours. The reaction solution was poured into water, stirred for 10 min, and subsequently extracted with ethyl acetate for three times. The organic phases were combined, dried over anhydrous sodium sulfate, and filtered. The filtrate was concentrated, and the residue was purified by silica gel column chromatography (eluent: ethyl acetate/petroleum ether = 1/2 (v/v)) to obtain the title compound (3.1 g, yield: 83.4%).

MS m/z (ESI): 409.0 [M+H]⁺.

¹H NMR (400 MHz, DMSO-*d*₆) δ: 9.06 (s, 1H), 8.13 (d, *J* = 1.6 Hz, 1H), 8.05 (s, 1H), 7.75 (d, *J* = 8.8 Hz, 1H), 7.51 (dd, *J* = 8.8, 2.0 Hz, 1H), 7.31-7.27 (m, 2H), 7.10-7.05 (m, 2H), 6.59 (s, 2H), 2.84 (s, 3H).

### Example 3: Preparation of methyl 2-amino-6-(4-fluorophenyl)-5-(4-methylquinazolin-6-yl) nicotinate (3)

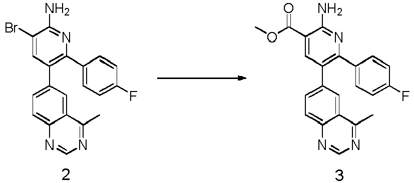

3-bromo-6-(4-fluorophenyl)-5-(4-methylquinazolin-6-yl)pyridin-2-amine (2) (1.2 g, 2.93 mmol), Pd(dppf)Cl₂ (119.63 mg, 0.15 mmol), triethylamine (888.45 mg, 8.80 mmol), methanol (3 mL) and DMF (10 mL) were added sequentially to a stainless steel high pressure reactor, changed to a carbon monoxide atmosphere, and reacted at 100°C for 16 hours. The reaction was cooled to room temperature, filtered, washed, and dried to obtain the title compound (980 mg, yield: 86.1%).

MS m/z (ESI): 389.1 [M+H]⁺.

¹H NMR (400 MHz, DMSO-*d*₆) δ: 9.08 (s, 1H), 8.25 (s, 1H), 8.11 (s, 1H), 7.79 (d, *J* = 8.8 Hz, 1H), 7.56 (d, *J* = 8.8 Hz, 1H), 7.47 (s, 2H), 7.35-7.33 (m, 2H), 7.12-7.07 (m, 2H), 3.87 (s, 3H), 2.82 (s, 3H).

### Example 4: Preparation of 2-amino-6-(4-fluorophenyl)-5-(4-methylquinazolin-6-yl)nicotinic acid (4)

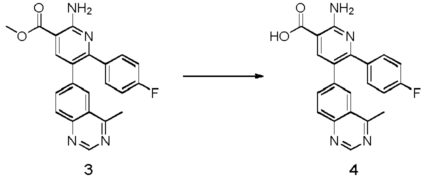

Methyl 2-amino-6-(4-fluorophenyl)-5-(4-methylquinazolin-6-yl)nicotinate (3) (120 mg, 0.31 mmol) was added to methanol (5 mL), then added with 50% aqueous sodium hydroxide solution (247.17 mg, 6 mmol), and reacted at 60°C for 5 hours. To the reaction system was added dropwise 1 N diluted hydrochloric acid to adjust the pH to 4-5. The reaction solution was concentrated, and the residue was purified by high performance liquid chromatography (Preparation method 1) to obtain the title compound (10 mg, yield: 8.7%).

MS m/z (ESI): 375.1 [M+H]⁺.

¹H NMR (400 MHz, DMSO-*d*₆) δ: 9.07 (s, 1H), 8.24-8.09 (m, 3H), 7.78 (d, *J* = 8.8 Hz, 1H), 7.57-7.33 (m, 5H), 7.09 (t, *J* = 8.8 Hz, 2H), 2.82 (s, 3H).

### Example 5: Preparation of 2-amino-6-(4-fluorophenyl)-5-(4-methylquinazolin-6-yl) nicotinonitrile (5)

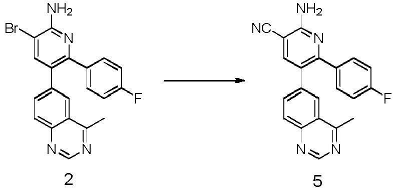

3-bromo-6-(4-fluorophenyl)-5-(4-methylquinazolin-6-yl)pyridin-2-amine (2) (150 mg, 0.37 mmol), zinc cyanide (21.44 mg, 0.18 mmol) and DMF (2 mL) were added sequentially to a 10 mL microwave tube. The reaction solution was bubbled with nitrogen gas for 5 min, added with tris(dibenzylideneacetone)dipalladium (16.79 mg, 0.018 mmol) and dppf (20.31 mg, 0.37 mmol), and microwave heated to 120°C for 2 hours. After the reaction was complete, the reaction solution was cooled to room temperature, filtered. The filtrate was concentrated, and the residue was purified by silica gel column chromatography (eluent: ethyl acetate/petroleum ether = 1/1 (v/v)) to obtain the title compound (110 mg, yield: 84.5%).

MS m/z (ESI): 356.1 [M+H]⁺.

¹H NMR (400 MHz, DMSO-*d*₆) δ: 9.08 (s, 1H), 8.24 (s, 1H), 8.18 (d, *J* = 2.0 Hz, 1H), 7.75 (d, *J* = 8.4 Hz, 1H), 7.49 (dd, *J* = 8.8, 2 Hz, 1H), 7.33-7.31 (m, 2H), 7.25 (s, 2H), 7.13-7.08 (m, 2H), 2.85 (s, 3H).

### Example 6: Preparation of 2-amino-6-(4-fluorophenyl)-5-(4-methylquinazolin-6-yl)nicotinamide (6)

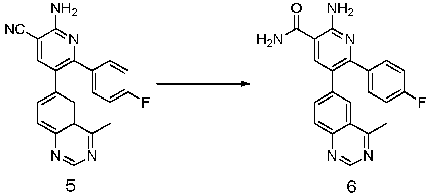

To a solution of 2-amino-6-(4-fluorophenyl)-5-(4-methylquinazolin-6-yl)nicotinonitrile (5) (400 mg, 1.13 mmol) in dimethyl sulfoxide (5 mL), potassium carbonate (155.33 mg, 1.13 mmol) and hydrogen peroxide (2.55 g, 22.51 mmol) were sequentially added, and reacted at room temperature for 2 hours. The reaction solution was poured into water, and extracted with ethyl acetate. The organic phases were combined, washed with saturated aqueous sodium sulfite solution for three times, dried over anhydrous sodium sulfate, and filtered. The filtrate was concentrated, and the residue was purified by silica gel column chromatography (eluent: methanol/dichloromethane=1/12 (v/v)) to obtain the title compound (320 mg, yield: 76.1%).

MS m/z (ESI): 374.1 [M+H]⁺.

¹H NMR (400 MHz, DMSO-*d*₆) δ: 9.07 (s, 1H), 8.23 (s, 1H), 8.15-8.13 (m, 2H), 7.79 (d, *J* = 8.8 Hz, 1H), 7.61-7.49 (m, 4H), 7.36-7.32 (m, 2H), 7.12-7.07 (m, 2H), 2.83 (s, 3H).

### Example 7: Preparation of 2-amino-6-(4-fluorophenyl)-N-methyl-5-(4-methylquinazolin-6-yl) nicotinamide (7)

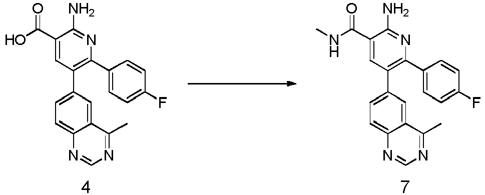

2-amino-6-(4-fluorophenyl)-5-(4-methylquinazolin-6-yl)nicotinic acid (4) (300 mg, 0.31 mmol) and *N,N*'-carbonyldiimidazole (67.48 mg, 0.47 mmol) were added to tetrahydrofuran (5 mL) and DMF (1.4 mL). After reacting at room temperature for 3 hours, a solution of methylamine in tetrahydrofuran (2 M, 0.8 mL) was added, and reacted for 3 hours at room temperature. The reaction solution was poured into water, and adjusted to pH 7-8 with saturated aqueous sodium carbonate solution, and then allowed to stand, filtered by suction, and purified by high performance liquid chromatography (Preparation method 2) to obtain the title compound (25 mg, yield 20.7%).

MS m/z (ESI): 388.1 [M+H]⁺.

¹H NMR (400 MHz, DMSO-*d*₆) δ: 9.07 (s, 1H), 8.58 (d, *J* = 4.8 Hz, 1H), 8.15-8.01 (m, 2H), 7.81 (d*, J* = 8.8 Hz, 1H), 7.62 (dd, *J* = 8.8, 2.0 Hz, 1H), 7.41-7.31 (m, 4H), 7.09 (t, *J* = 9.2 Hz, 2H), 2.87-2.74 (m, 6H).

### Example 8: Preparation of 6-(4-fluorophenyl)-3-(1-methyl-1H-pyrazol-4-yl)-5-(4-methyl quinazolin-6-yl)pyridin-2-amine (8)

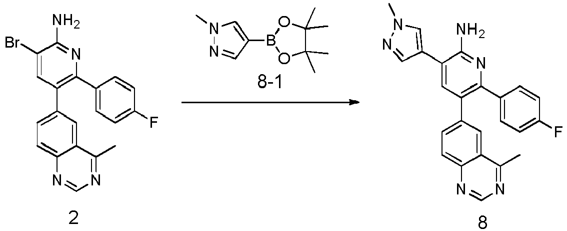

3-bromo-6-(4-fluorophenyl)-5-(4-methylquinazolin-6-yl)pyridin-2-amine (2) (150 mg, 0.37 mmol) and 1-methyl-4-(4,4,5,5-tetramethyl-1,3,2-dioxaborolan-2-yl)-1*H*-pyrazole (8-1) (99.14 mg, 0.48 mmol) were added to 1,4-dioxane (5 mL) and water (0.5 mL), purged with nitrogen for three times, then added with Pd(dppf)Cl₂ (14.95 mg, 0.018 mmol) and cesium carbonate (238.97 mg, 0.73 mmol), and reacted at 95°C for 12 hours. After the reaction was complete, the reaction solution was cooled to room temperature, filtered and concentrated, and the residue was purified by silica gel column chromatography (eluent: methanol/dichloromethane = 1/15 (v/v)) to obtain the title compound (120 mg, yield: 79.8%).

MS m/z (ESI): 411.2 [M+H]⁺.

¹H NMR (400 MHz, DMSO-*d*₆) δ: 9.06 (s, 1H), 8.14-8.11 (m, 2H), 7.87 (s, 1H), 7.77 (d, *J* = 8.8 Hz, 2H), 7.58 (dd, *J* = 8.4, 2.0 Hz, 1H), 7.34-7.30 (m, 2H), 7.10-7.05 (m, 2H), 6.04 (s, 2H), 3.90 (s, 3H), 2.83 (s, 3H).

### Example 9: Preparation of 6-(4-fluorophenyl)-5-(4-methylquinazolin-6-yl)-3-(1H-tetrazol-5-yl) pyridin-2-amine

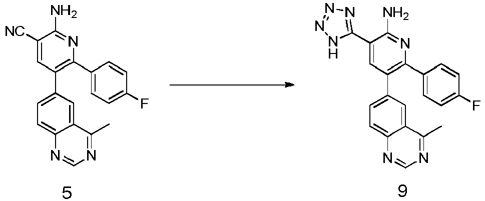

2-amino-6-(4-fluorophenyl)-5-(4-methylquinazolin-6-yl)nicotinonitrile (5) (45 mg, 0.13 mmol) was dissolved in DMF (1 mL), added sequentially with sodium azide (9.88 mg, 0.15 mmol) and ammonium chloride (8.13 mg, 0.15 mmol), and reacted at 95°C for 48 hours. After the reaction was complete, the reaction solution was cooled to room temperature, and was purified by high performance liquid chromatography (preparation method 2) to obtain the title compound (30 mg, yield: 59.5%).

MS m/z (ESI): 399.1 [M+H]⁺.

¹H NMR (400 MHz, DMSO-*d*₆) δ: 9.09 (s, 1H), 8.43 (s, 1H), 8.14 (d, *J* = 1.6 Hz, 1H), 7.84 (d, *J* = 8.8 Hz, 1H), 7.65-7.62 (m, 3H), 7.39-7.36 (m, 2H), 7.13-7.09 (m, 2H), 2.55 (s, 1H) 2.83 (s, 3H).

### Example 10: Preparation of 4-chloro-6-(4-fluorophenyl)-5-(4-methylquinazolin-6-yl)pyridin-2-amine (10)

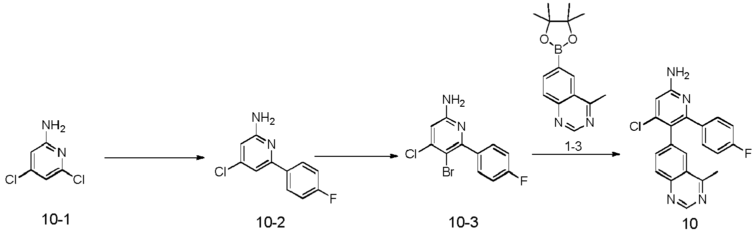

### Step 1: Preparation of 4-chloro-6-(4-fluorophenyl)pyridin-2-amine (10-2)

4,6-dichloropyridin-2-amine (10-1) (1.5 g, 9.20 mmol) was added to a mixed solvent of 1,4-dioxane (20 mL) and water (6 mL), and then added sequentially with 4-fluorophenylboronic acid (1.42 g, 10.10 mmol), potassium carbonate (2.58 g, 18.70 mmol) and Pd(dppf)Cl₂ (369 mg, 0.46 mmol), purged with nitrogen, and reacted at 90°C for 12 hours. After the reaction was completed, the reaction solution was cooled to room temperature, poured into water, and extracted with ethyl acetate. The organic phases were combined, dried over anhydrous sodium sulfate, and filtered. The filtrate was concentrated, and the residue was purified by silica gel column chromatography (eluent: ethyl acetate/petroleum ether = 1/5 (v/v)) to obtain the title compound (1.1 g, yield: 53.7%).

MS m/z (ESI): 223.0 [M+H]⁺.

### Step 2: Preparation of 5-bromo-4-chloro-6-(4-fluorophenyl)pyridin-2-amine (10-3)

4-chloro-6-(4-fluorophenyl)pyridin-2-amine (10-2) (8 g, 35.93 mmol) was added to dry DMF (100 mL), cooled to 0°C, added with *N*-bromosuccinimide (6.65 g, 37.36 mmol), and reacted for 2 hours at room temperature. The reaction solution was diluted with water, and filtered. The residue upon filtering was slurried and purified with ethyl acetate/petroleum ether = 1/10 (v/v) to obtain the title compound of this step (8 g, yield: 67.8%).

MS m/z (ESI): 300.9 [M+H]⁺.

### Step 3: Preparation of 4-chloro-6-(4-fluorophenyl)-5-(4-methylquinazolin-6-yl)pyridin-2-amine (10)

5-bromo-4-chloro-6-(4-fluorophenyl)pyridin-2-amine (10-3) (1.8 g, 5.97 mmol) was dissolved in a mixed solvent of 1,4-dioxane (30 mL) and water (6 mL), and added sequentially with 4-methyl-6-(4,4,5,5-tetramethyl-1,3,2-dioxaborolan-2-yl)quinazoline (1-3) (1.94 g, 7.18 mmol), potassium carbonate (1.65 g, 11.94 mmol) and Pd(dppf)Cl₂ (254 mg, 0.31 mmol), purged with nitrogen for three times, and reacted overnight at 80°C. After the reaction was completed, the reaction solution was cooled to room temperature, poured into water, and extracted with ethyl acetate. The organic phases were combined, dried over anhydrous sodium sulfate, and filtered. The filtrate was concentrated, and the residue was purified by silica gel column chromatography (eluent: ethyl acetate/petroleum ether = 2/1 (v/v)) to obtain the title compound (1.4 g, yield: 64.3%).

MS m/z (ESI): 365.1 [M+H]⁺.

¹H NMR (400 MHz, DMSO-*d*₆) δ: 9.07 (s, 1H), 8.00 (d, *J* = 1.6 Hz, 1H), 7.85 (d, *J* = 8.6 Hz, 1H), 7.70 (dd, *J* = 8.6, 1.8 Hz, 1H), 7.21 (dd, *J* = 8.8, 5.6 Hz, 2H), 6.96 (t, *J* = 8.8 Hz, 2H), 6.71 (s, 1H), 6.55 (s, 2H), 2.75 (s, 3H).

### Example 11: Preparation of 6-amino-2-(4-fluorophenyl)-3-(4-methylquinazolin-6-yl) isonicotinonitrile (11)

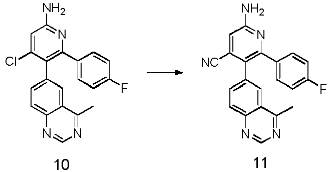

4-chloro-6-(4-fluorophenyl)-5-(4-methylquinazolin-6-yl)pyridin-2-amine (10) (170 mg, 0.47 mmol), zinc powder (3.0 mg, 0.47 mmol), zinc cyanide (55 mg, 0.47 mmol) and DMF (2 mL) were added sequentially to a 10 mL microwave tube. The reaction solution was bubbled with nitrogen gas for 5 min, added with tris(dibenzylideneacetone)dipalladium (46 mg, 0.05 mmol) and dppf (56 mg, 0.1 mmol), and microwave heated to 140°C for 5 hours. The reaction solution was cooled to room temperature, poured into 20 mL water, stirred for 5 min, and extracted with ethyl acetate for three times. The organic phases were washed with saturated brine for three times, dried over anhydrous sodium sulfate, and filtered. The filtrate was concentrated, and the residue was purified by silica gel column chromatography (eluent: ethyl acetate/petroleum ether = 1/1 (v/v)) to obtain the title compound (160 mg, yield: 95.6%).

MS m/z (ESI): 356.1 [M+H]⁺.

¹H NMR (400 MHz, DMSO-*d*₆) δ: 9.11 (s, 1H), 8.24 (d, *J* = 1.6 Hz, 1H), 7.86 (d, *J* = 8.8 Hz, 1H), 7.65 (dd, *J* = 8.8, 2.0 Hz, 1H), 7.32-7.19 (m, 3H), 7.10-6.79 (m, 4H), 2.81 (s, 3H).

### Example 12: Preparation of 2-amino-6-(3,5-difluorophenyl)-5-(4-methylquinazolin-6-yl) nicotinamide (12)

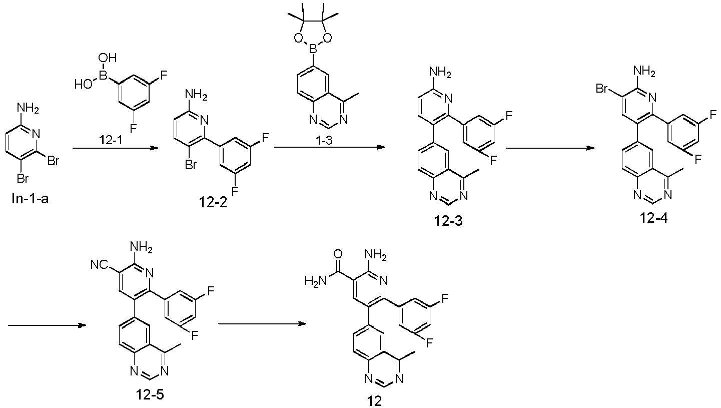

### Step 1: Preparation of 5-bromo-6-(3,5-difluorophenyl)pyridin-2-amine (12-2)

By a similar process to that described in Step 1 of Intermediate Preparation Example 1, by replacing 4-fluorophenylboronic acid in Step 1 of Intermediate Preparation Example 1 with 3,5-difluorophenyl boronic acid (12-1), the title compound (1.4 g, yield: 93.2%) was synthesized.

MS m/z (ESI): 285.0 [M+H]⁺.

### Step 2: Preparation of 6-(3,5-difluorophenyl)-5-(4-methylquinazolin-6-yl)pyridin-2-amine (12-3)

By a similar process to that described in Step 2 of Example 1, by replacing 5-bromo-6-(4-fluorophenyl)pyridin-2-amine (1-2-a) in Step 2 of Example 1 with 5-bromo-6-(3,5-difluorophenyl)pyridin-2-amine (12-2), the title compound (1.1 g, yield: 83.4%) of this step was synthesized.

MS m/z (ESI): 349.1 [M+H]⁺.

### Step 3: Preparation of 3-bromo-6-(3,5-difluorophenyl)-5-(4-methylquinazolin-6-yl)pyridin-2-amine (12-4)

By a similar process to that described in Example 2, by replacing 6-(4-fluorophenyl)-5-(4-methylquinazolin-6-yl)pyridin-2-amine (1) in Example 2 with 6-(3,5-difluorophenyl)-5-(4-methylquinazolin-6-yl)pyridin-2-amine (12-3), the title compound (1.2 g, yield: 82.7%) of this step was synthesized.

MS m/z (ESI): 427.0 [M+H]⁺.

### Step 4: Preparation of 2-amino-6-(3,5-difluorophenyl)-5-(4-methylquinazolin-6-yl)nicotinonitrile (12-5)

By a similar process to that described in Example 5, by replacing 3-bromo-6-(4-fluorophenyl)-5-(4-methylquinazolin-6-yl)pyridin-2-amine (2) in Example 5 with 3-bromo 6-(3,5-difluorophenyl)-5-(4-methylquinazolin-6-yl)pyridin-2-amine (12-4), the title compound (0.63 g, yield: 81.3%) of this step was synthesized.

MS m/z (ESI): 374.1 [M+H]⁺.

### Step 5: Preparation of 2-amino-6-(3,5-difluorophenyl)-5-(4-methylquinazolin-6-yl)nicotinamide (12)

By a similar process to that described in Example 6, by replacing 2-amino-6-(4-fluorophenyl)-5-(4-methylquinazolin-6-yl)pyridin-3-nicotinonitrile (5) in Example 6 with 2-amino-6-(3,5-difluorophenyl)-5-(4-methylquinazolin-6-yl)nicotinonitrile (12-5), the title compound (32 mg, yield: 27.2%) was synthesized.

MS m/z (ESI): 392.1 [M+H]⁺.

¹H NMR (400 MHz, DMSO-*d*₆) δ: 9.09 (s, 1H), 8.25 (s, 1H), 8.19 (s, 1H), 8.16 (d, *J* = 1.6 Hz, 1H), 7.83 (d, *J* = 8.8 Hz, 1H), 7.64 (dd, *J* = 8.8, 2.0 Hz, 1H), 7.55 (d, *J* = 9.6 Hz, 3H), 7.21 (tt, *J* = 9.2, 2.4 Hz, 1H), 7.01-6.92 (m, 2H), 2.86 (s, 3H).

### Example 13: Preparation of 2-amino-6-(2-fluorophenyl)-5-(4-methylquinazolin-6-yl) nicotinamide (13)

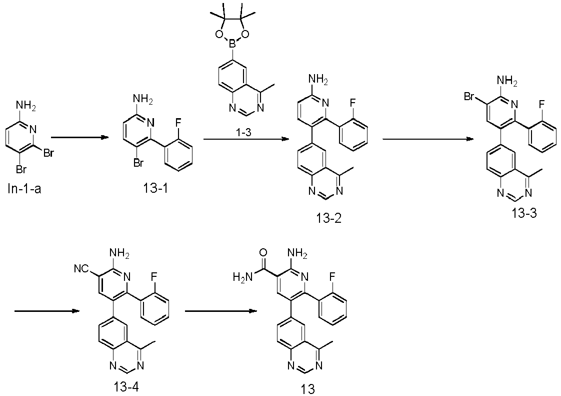

The title compound (50 mg, yield: 36.2%) was obtained according to the synthetic route of Example 12, with replacing the starting material 3,5-difluorophenylboronic acid (12-1) in Step 1 with 2-fluorophenyl boronic acid.

MS m/z (ESI): 374.1 [M+H]⁺.

¹H NMR (400 MHz, DMSO-*d*₆) δ: 9.04 (s, 1H), 8.27 (s, 1H), 8.21 (s, 1H), 7.90 (d, *J =* 1.6 Hz, 1H), 7.84-7.77 (m, 2H), 7.53-7.50 (m, 4H), 7.39-7.36 (m, 1H), 7.27-7.23 (m, 1H), 7.02-6.97 (m, 1H), 2.67 (s, 3H).

### Example 14: Preparation of 2-amino-6-(3-fluorophenyl)-5-(4-methylquinazolin-6-yl) nicotinamide (14)

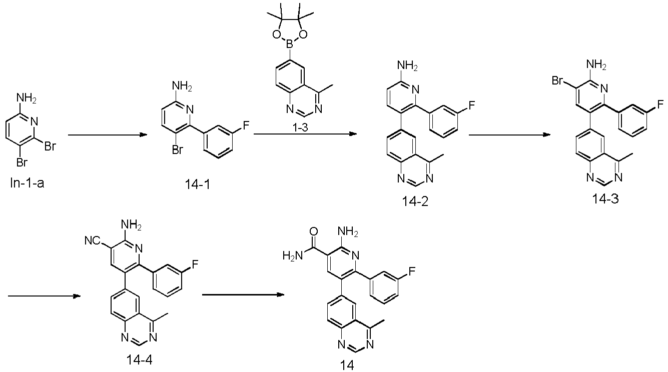

The title compound was obtained (50 mg, yield: 34.2%) according to the synthetic route of Example 12, with replacing the starting material 3,5-difluorophenylboronic acid (12-1) in Step 1 with 3-fluorophenylboronic acid.

MS m/z (ESI): 374.1 [M+H]⁺.

¹H NMR (400 MHz, DMSO-*d*₆) δ: 9.07 (s, 1H), 8.24-8.13 (m, 3H), 7.79 (d, *J* = 8.8 Hz, 1H), 7.64-7.51 (m, 4H), 7.26-7.03 (m, 4H), 2.82 (s, 3H).

### Example 15: Preparation of 2-(4-(2-amino-6-(4-fluorophenyl)-5-(4-methylquinazolin-6-yl) pyridin-3-yl)-1H-pyrazol-1-yl)acetamide (15)

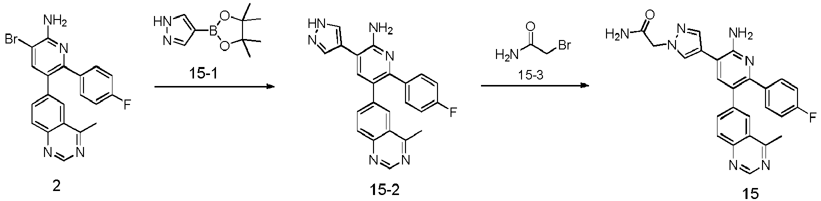

### Step 1: Preparation of 6-(4-fluorophenyl)-5-(4-methylquinazolin-6-yl)-3-(1H-pyrazol-4-yl) pyridin-2-amine (15-2)

By a similar process to that described in Example 8, by replacing 1-methyl-4-(4,4,5,5-tetramethyl-1,3,2-dioxaborolan-2-yl)-1*H*-pyrazole (8-1) in Example 8 with 4-(4,4,5,5-tetramethyl-1,3,2-dioxaborolan-2-yl)-1*H*-pyrazole (15-1), the title compound of this step (0.4 mg, yield: 76%) was synthesized.

MS m/z (ESI): 397.1 [M+H]⁺.

### Step 2: Preparation of 2-(4-(2-amino-6-(4-fluorophenyl)-5-(4-methylquinazolin-6-yl) pyridin-3-yl)-1H-pyrazol-1-yl )acetamide (15)

6-(4-fluorophenyl)-5-(4-methylquinazolin-6-yl)-3-(1*H*-pyrazol-4-yl)pyridin-2-amine (15-2) (200 mg, 0.50 mmol), 2-bromoacetamide (15-3) (104 mg, 0.75 mmol) and potassium carbonate (140 mg, 1.01 mmol) were added to DMF (4 mL) and reacted at room temperature for 16 hours. The reaction solution was poured into water, and extracted with ethyl acetate for three times. The organic phases were combined, dried over anhydrous sodium sulfate, and filtered. The filtrate was concentrated, and the residue was purified by high performance liquid chromatography (Preparation Method 2) to obtain the title compound (35 mg, yield: 14.5%).

MS m/z (ESI): 454.2 [M+H]⁺.

¹H NMR (400 MHz, DMSO-*d*₆) δ: 9.06 (s, 1H), 8.16 (s, 1H), 8.12 *(d, J* = 1.6 Hz, 1H), 7.90 (s, 1H), 7.77 (d, *J* = 8.4 Hz, 2H), 7.59 (dd, *J* = 8.4, 1.6 Hz, 1H), 7.44 (s, 1H), 7.36-7.30 (m, 3H), 7.07 (t, *J* = 8.8 Hz, 2H), 6.05 (s, 2H), 4.81 (s, 2H), 2.83 (s, 3H).

### Example 16: Preparation of 3-(1-(2-(dimethylamino)ethyl)-1H-pyrazol-4-yl)-6-(4-fluorophenyl)-5-(4-methylquinazolin-6-yl)pyridin-2-amine (16)

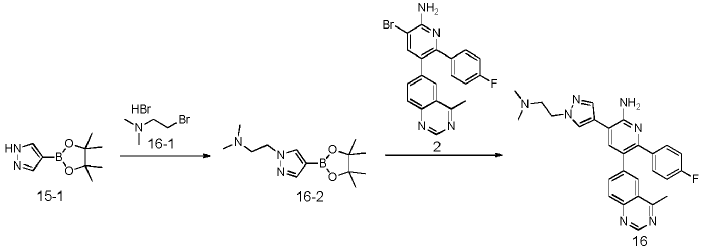

### Step 1: Preparation of N,N-dimethyl-4-(4,4,5,5-tetramethyl-1,3,2-dioxaborolan-2-yl)-1H-pyrazol-1-ethanamine (16-2)

4-(4,4,5,5-tetramethyl-1,3,2-dioxaborolan-2-yl)-1*H*-pyrazole (15-1) (388 mg, 2.0 mmol), 2-bromo-*N,N*-dimethylethylamine hydrobromide (16-1) (700 mg, 3.0 mmol) and acetonitrile (10 mL) were added sequentially into a 50 mL round-bottom flask, added with cesium carbonate (1.3 g, 4.0 mmol) under stirring, and reacted at 80°C for 12 hours. The reaction solution was cooled to room temperature, and filtered. The filtrate was concentrated, and the residue was added with water, and extracted with ethyl acetate for three times. The organic phases were washed with saturated brine for two times, dried over anhydrous sodium sulfate, and filtered. The filtrate was concentrated to obtain the title compound of this step (452 mg, yield: 85.2%).

MS m/z (ESI): 266.2 [M+H]⁺.

### Step 2: Preparation of 3-(1-(2-(dimethylamino)ethyl)-1H-pyrazol-4-yl)-6-(4-fluorophenyl)-5-(4-methylquinazolin-6-yl)pyridin-2-amine (16)

By a similar process to that described in Example 8, by replacing 1-methyl-4-(4,4,5,5-tetrainethyl-1,3,2-dioxaborolan-2-yl)-1*H*-pyrazole (8-1) in Example 8 with *N,N*-dimethyl-4-(4,4,5,5-tetrainethyl-1,3,2-dioxaborolan-2-yl)-1*H*-pyrazole-1-ethylamine (16-2), the title compound of this step (30 mg, yield: 25.0%) was synthesized.

MS m/z (ESI): 468.2 [M+H]⁺.

¹H NMR (400 MHz, DMSO-*d*₆) δ: 9.06 (s, 1H), 8.20 (s, 1H), 8.12 (d, *J* = 1.6 Hz, 1H), 7.88 (s, 1H), 7.77 (t, *J* = 4.4 Hz, 2H), 7.60-7.58 (m, 1H), 7.34-7.30 (m, 2H), 7.07 (t, *J* = 8.8 Hz, 2H), 6.02 (s, 2H), 4.24 (t, *J* = 6.8 Hz, 2H), 2.82 (s, 3H), 2.71 (t, *J* = 6.8 Hz, 2H), 2.19 (s, 6H).

### Example 17: Preparation of 6-(4-fluorophenyl)-3-(1-(2-methoxyethyl)-1H-pyrazol-4-yl)-5-(4-methylquinazolin-6-yl)pyridin-2-amine (17)

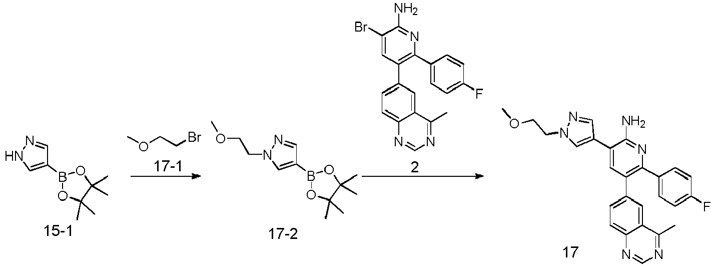

### Step 1: Preparation of 1-(2-methoxyethyl)-4-(4,4,5,5-tetramethyl-1,3,2-dioxaborolan-2-yl)-1H-pyrazole (17-2)

By a similar process to that described in Step 1 of Example 16, by replacing 2-bromo-*N,N*-dimethylethylamine hydrobromide (16-1) in Step 1 of Example 16 with 1-bromo-2-methoxyethane (17-1), the title compound of this step (480 mg, yield: 86.3%) was synthesized.

MS m/z (ESI): 253.2 [M+H]⁺.

### Step 2: Preparation of 6-(4-fluorophenyl)-3-(1-(2-methoxyethyl)-1H-pyrazol-4-yl)-5-(4-methyl quinazolin-6-yl)pyridin-2-amine (17)

By a similar process to that described in Example 8, by replacing 1-methyl-4-(4,4,5,5-tetrainethyl-1,3,2-dioxaborolan-2-yl)-1*H*-pyrazole (8-1) in Example 8 with 1-(2-methoxyethyl)-4-(4,4,5,5-tetramethyl-1,3,2-dioxolane-2-yl)-1*H*-pyrazole (17-2), the title compound (60 mg, yield: 34.2%) was synthesized.

MS m/z (ESI): 455.2 [M+H]⁺.

¹H NMR (400 MHz, DMSO-*d*₆) δ: 9.06 (s, 1H), 8.18 (s, 1H), 8.12 *(d, J* = 1.6 Hz, 1H), 7.90 (s, 1H), 7.78-7.76 (m, 2H), 7.60-7.57 (m, 1H), 7.34-7.31 (m, 2H), 7.09-7.05 (m, 2H), 6.02 (s, 2H), 4.31 (t, *J* = 5.2 Hz, 2H), 3.75 (t, *J* = 5.2 Hz, 2H), 3.26 (s, 3H), 2.83 (s, 3H).

### Example 18: Preparation of 6-(4-fluorophenyl)-3-(1-(2-(2-methoxyethoxy)ethyl)-1H-pyrazol-4-yl)-5-(4-methylquinazolin-6-yl)pyridin-2-amine (18)

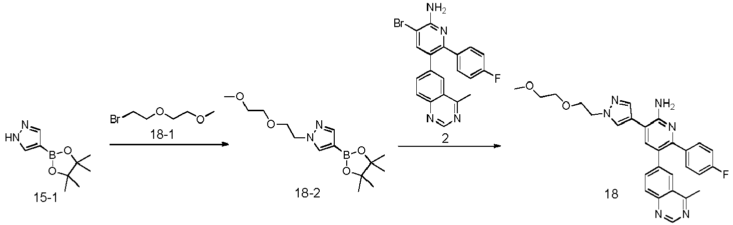

### Step 1: Preparation of 1-(2-(2-methoxyethoxy)ethyl)-4-(4,4,5,5-tetramethyl-1,3,2-dioxaborolan-2-yl)-1H-pyrazole (18-2)

By a similar process to that described in Step 1 of Example 16, by replacing 2-bromo-*N,N*-dimethylethylamine hydrobromide (16-1) in Step 1 of Example 16 with 1-bromo-2-(2-methoxyethoxy)ethane (18-1), the title compound of this step (920 mg, yield: 73.2%) was synthesized.

MS m/z (ESI): 297.2 [M+H]⁺.

### Step 2: Preparation of 1-(2-(2-methoxyethoxy)ethyl)-4-(4,4,5,5-tetramethyl-1,3,2-dioxaborolan-2-yl)-1H-pyrazole (18)

By a similar process to that described in Example 8, by replacing 1-methyl-4-(4,4,5,5-tetramethyl-1,3,2-dioxaborolan-2-yl)-1*H*-pyrazole (8-1) in Example 8 with 1-(2-(2-methoxyethyl)ethyl)-4-(4,4,5,5-tetramethyl-1,3,2-dioxoborolan-2-yl)-1*H*-pyrazole (18-2), the title compound (39 mg, yield: 32.4%) was synthesized.

MS m/z (ESI): 499.2 [M+H]⁺.

¹H NMR (400 MHz, DMSO-*d*₆) δ: 9.06 (s, 1H), 8.18 (s, 1H), 8.12 (d, *J* = 1.6 Hz, 1H), 7.90 (s, 1H), 7.77 (t, *J* = 4.4 Hz, 2H), 7.59 (dd, *J* = 8.8, 1.8 Hz, 1H), 7.32 (dd, *J* = 8.6, 5.6 Hz, 2H), 7.07 (t, *J* = 8.8 Hz, 2H), 6.02 (s, 2H), 4.31 (t, *J* = 5.4 Hz, 2H), 3.83 (t, *J* = 5.4 Hz, 2H), 3.53 (dd, *J* = 5.6, 3.6 Hz, 2H), 3.42 (dd, *J* = 5.6, 3.6 Hz, 2H), 3.20 (s, 3H), 2.82 (s, 3H).

### Example 19: Preparation of 2-(4-(2-amino-6-(4-fluorophenyl)-5-(4-methylquinazolin-6-yl)-pyridin-3-yl)-1H-pyrazol-1-yl)-N-methylacetamide (19)

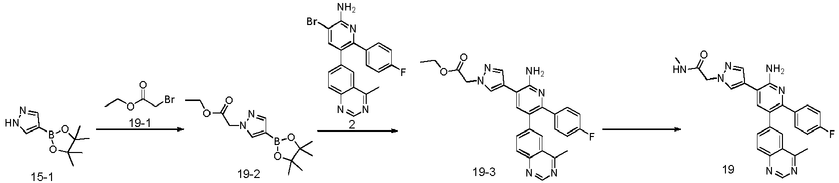

### Step 1: Preparation of ethyl 2-(4,4,5,5-tetramethyl-1,3,2-dioxaborolan-2-yl)-1H-pyrazol-1-yl) acetate (19-2)

By a similar process to that described in Step 1 of Example 16, by replacing 2-bromo-*N,N*-dimethylethylamine hydrobromide (16-1) in Step 1 of Example 16 with ethyl 2-bromoacetate (19-1), the title compound of this step (450 mg, yield: 85.3%) was synthesized.

MS m/z (ESI): 281.2 [M+H]⁺.

### Step 2: Preparation of ethyl 2-(4-(2-amino-6-(4-fluorophenyl)-5-(4-methylquinazolin-6-yl)-pyridin-3-yl)-1H-pyrazol-1-yl)acetate (19-3)

By a similar process to that described in Example 8, by replacing 1-methyl-4-(4,4,5,5-tetramethyl-1,3,2-dioxaborolan-2-yl)-1*H*-pyrazole (8-1) in Example 8 with ethyl 2-(4,4,5,5-tetramethyl-1,3,2-dioxaborolan-2-yl)-1*H*-pyrazol-1-yl)acetate (19-2), the title compound of this step (234 mg, yield: 75.3%) was synthesized.

MS m/z (ESI): 483.2 [M+H]⁺.

### Step 3: Preparation of 2-(4-(2-amino-6-(4-fluorophenyl)-5-(4-methylquinazolin-6-yl)pyridin-3-yl) -1H-pyrazol-1-yl)-N-methylacetamide (19)

Ethyl 2-(4-(2-amino-6-(4-fluorophenyl)-5-(4-methylquinazolin-6-yl)pyridine-3-yl)-1H-pyrazol-1-yl) acetate (19-3) (100 mg, 0.21 mmol), a solution of methylamine in methanol (2.0 mL, 4.0 mmol, 2.0 M), and methanol (2 mL) were added sequentially to a 10 mL round-bottom flask, and reacted at 65°C for 4 hours. The reaction solution was allowed to stand, cooled to room temperature, and then concentrated. The residue obtained was purified by high performance liquid chromatography (Preparation Method 2) to obtain the title compound (50 mg, yield: 50.9%).

MS m/z (ESI): 468.2 [M+H]⁺.

¹H NMR (400 MHz, CDCl₃) δ: 9.15 (s, 1H), 7.91-7.82 (m, 4H), 7.62-7.60 (m, 2H), 7.35-7.31 (m, 2H), 6.95-7.90 (m, 2H), 6.40 (s, 1H), 4.94 (s, 2H), 4.88 (s, 2H), 2.86 (d, *J* = 5.2 Hz, 3H), 2.82 (s, 3H).

### Example 20: Preparation of 3-(4-(2-amino-6-(4-fluorophenyl)-5-(4-methylquinazolin-6-yl)-pyridin-3-yl)-1H-pyrazol-1-yl)propionamide (20)

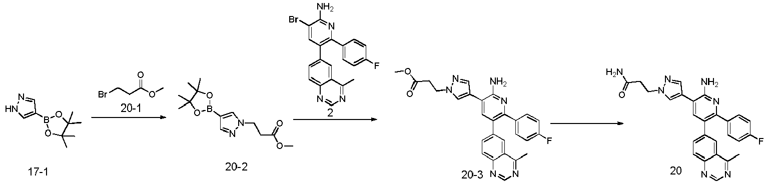

### Step 1: Preparation of methyl 3-(4,4,5,5-tetramethyl-1,3,2-dioxaborolan-2-yl)-1H-pyrazol-1-yl) propionate (20-2)

By a similar process to that described in Step 1 of Example 16, by replacing 2-bromo-*N,N*-dimethylethylamine hydrobromide (16-1) in Step 1 of Example 16 with methyl 3-bromopropionate (20-1), the title compound of this step (480 mg, yield: 86.5%) was synthesized.

MS m/z (ESI): 281.2 [M+H]⁺.

### Step 2: Preparation of methyl 3-(4-(2-amino-6-(4-fluorophenyl)-5-(4-methylquinazolin-6-yl) pyridin-3-yl)-1H-pyrazol-1-yl)propionate (20-3)

By a similar process to that described in Example 8, by replacing 1-methyl-4-(4,4,5,5-tetramethyl-1,3,2-dioxaborolan-2-yl)-1*H*-pyrazole (8-1) in the Example 8 with methyl 3-(4,4,5,5-tetramethyl-1,3,2-dioxaborolan-2-yl)-1*H*-pyrazol-1-yl)propionate (20-2), the title compound of this step (218 mg, yield: 86.4%) was synthesized.

MS m/z (ESI): 483.2 [M+H]⁺.

### Step 3: Preparation of 3-(4-(2-amino-6-(4-fluorophenyl)-5-(4-methylquinazolin-6-yl)pyridin-3-yl)-1H-pyrazol-1-yl)propionamide (20)

The title compound (25 mg, yield: 15.8%) was synthesized by a similar process to that described in Step 3 of Example 19, by replacing ethyl 2-(4-(2-amino-6-(4-fluorophenyl)-5-(4-methylquinazolin-6-yl) pyridin-3-yl)-1*H*-pyrazol-1-yl)acetate (19-3) in Step 3 of Example 19 with methyl 3 -(4-(2-amino-6-(4-fluorophenyl)-5 -(4-methylquinazolin-6-yl)pyridin-3-yl)-1*H*-pyrazol-1-yl)propionate (20-3), and replacing the solution of methylamine in methanol in Step 3 of Example 19 with an solution of ammonia in methanol.

MS m/z (ESI): 468.2 [M+H]⁺.

¹H NMR (400 MHz, CDCl₃) δ: 9.15 (s, 1H), 7.88-7.79 (m, 4H), 7.60-7.58 (m, 2H), 7.35-7.32 (m, 2H), 6.95-6.91 (m, 2H), 5.70 (s, 1H), 5.44 (s, 1H), 5.18 (br, 2H), 4.56-4.53 (m, 2H), 2.91-2.88 (m, 2H), 2.82 (s, 3H).

### Example 21: Preparation of 1-(4-(2-amino-6-(4-fluorophenyl)-5-(4-methylquinazolin-6-yl)-pyridin-3-yl)-1H-pyrazol-1-yl)-2-methylpropan-2-ol (21)

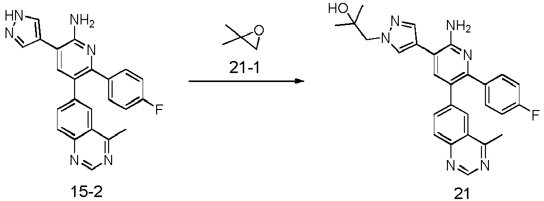

6-(4-fluorophenyl)-5-(4-methylquinazolin-6-yl)-3-(1*H*-pyrazol-4-yl)pyridin-2-amine (15-2) (200 mg, 0.50 mmol), 2,2-dimethyloxirane (21-1) (108 mg, 1.50 mmol) and potassium carbonate (207 mg, 1.50 mmol) were added to DMSO (4 mL) and reacted in an sealed condition at 80°C for 12 hours. The reaction solution was poured into water, and extracted with ethyl acetate for three times. The organic phases were combined, dried over anhydrous sodium sulfate, and filtered. The filtrate was concentrated, and the residue was purified by high performance liquid chromatography (Preparation Method 2) to obtain the title compound (32 mg, yield: 23%).

### MS m/z (ESI): 469.2 [M+H]⁺.

¹H NMR (400 MHz, DMSO-*d*₆) δ: 9.06 (s, 1H), 8.13-8.08 (m, 2H), 7.88 (s, 1H), 7.77 (t, *J* = 4.4 Hz, 2H), 7.59 (dd, *J* = 8.4, 1.6 Hz, 1H), 7.32 (dd, *J* = 8.8, 5.6 Hz, 2H), 7.07 (t, *J* = 8.8 Hz, 2H), 6.03 (s, 2H), 4.76 (s, 1H), 4.07 (s, 2H), 2.82 (s, 3H), 1.11 (s, 6H).

### Example 22: Preparation of 2-amino-6-(4-fluorophenyl)-5-(4-methylquinolin-6-yl)nicotinamide (22)

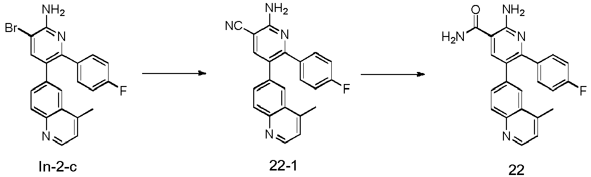

### Step 1: Preparation of 2-amino-6-(4-fluorophenyl)-5-(4-methylquinolin-6-yl)nicotinonitrile (22-1)

By a similar process to that described in Example 5, by replacing 3-bromo-6-(4-fluorophenyl)-5-(4-methylquinazolin-6-yl)pyridin-2-amine (2) in Example 5 with 3-bromo-6-(4-fluorophenyl)-5-(4-methylquinolin-6-yl)pyridin-2-amine (In-2-c), the title compound of this step (0.5 g, yield: 65.4%) was synthesized.

MS m/z (ESI): 355.1 [M+H]⁺.

### Step 2: Preparation of 2-amino-6-(4-fluorophenyl)-5-(4-methylquinolin-6-yl)nicotinamide (22)

By a similar process to that described in Example 6, by replacing 2-amino-6-(4-fluorophenyl)-5-(4-methylquinazolin-6-yl)pyridine-3-carbonitrile (5) in Example 6 with 2-amino-6-(4-fluorophenyl)-5-(4-methylquinolin-6-yl)nicotinonitrile (22-1), the title compound (120 mg, yield: 53.2%) was synthesized.

MS m/z (ESI): 373.1 [M+H]⁺.

¹H NMR (400 MHz, DMSO-*d*₆) δ: 8.70 (d, *J* = 4.4 Hz, 1H), 8.21 (s, 1H), 8.16 (s, 1H), 7.91 (d, *J* = 1.6 Hz, 1H), 7.82 (d, *J* = 8.8 Hz, 1H), 7.47-7.39 (m, 4H), 7.37-7.31 (m, 3H), 7.07 (dd, *J* = 12.0, 5.6 Hz, 2H), 2.57 (s, 3H).

### Example 23: Preparation of 1-(2-amino-6-(4-fluorophenyl)-5-(4-methylquinazolin-6-yl)pyridin-3-yl)-1H-pyrazol-3-carboxamide (23)

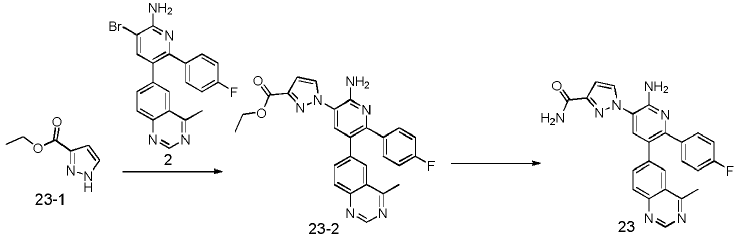

### Step 1: Preparation of ethyl 1-(2-amino-6-(4-fluorophenyl)-5-(4-methylquinazolin-6-yl) pyridin-3-yl)-1H-pyrazol-3-carboxylate (23-2)

3-bromo-6-(4-fluorophenyl)-5-(4-methylquinazolin-6-yl)pyridin-2-amine (2) (100 mg, 0.24 mmol), ethyl 1*H*-pyrazole-3-carboxylate (23-1) (50 mg, 0.36 mmol), cesium carbonate (157 mg, 0.48 mmol) and DMF (2 mL) were added sequentially into a 10 mL microwave tube, bubbled with nitrogen for 5 min, and then added with cuprous iodide (46 mg, 0.24 mmol), trans-(1R,2R)-*N*,*N*'-dimethyl-1,2-cyclohexanediamine (34 mg, 0.24 mmol), bubbled with nitrogen for 3 min, and reacted at 110°C in microwave for 5 hours. The reaction mixture was cooled to room temperature, poured into 20 mL water, and extracted for three times with ethyl acetate. The organic phases were washed with saturated brine, dried over anhydrous sodium sulfate, and filtered. The filtrate was concentrated, and the residue was purified by silica gel column chromatography (eluent: ethyl acetate/petroleum ether = 1/1 (v/v)) to obtain the title compound of this step (82 mg, yield: 72.5%).

MS m/z (ESI): 469.2 [M+H]⁺.

### Step 2: Preparation of 1-(2-amino-6-(4-fluorophenyl)-5-(4-methylquinazolin-6-yl)pyridin-3-yl)-1H-pyrazol-3-carboxamide (23)

Ethyl 1-(2-amino-6-(4-fluorophenyl)-5-(4-methylquinazolin-6-yl)pyridin-3-yl)-1*H*-pyrazol-3-carboxylate (23-2) (80 mg, 0.17 mmol), an ammonia solution in methanol (0.5 mL, 3.5 mmol, 7.0 M), and methanol (1 mL) were added sequentially to a 25 mL round-bottom flask, and reacted at 65°C for 4 hours. The reaction solution was cooled to room temperature, and then concentrated. The residue obtained was purified by high performance liquid chromatography (Preparation Method 2) to obtain the title compound (40 mg, yield: 66.7%).

MS m/z (ESI): 440.2 [M+H]⁺.

¹H NMR (400 MHz, DMSO-*d*₆) δ: 9.08 (s, 1H), 8.54 (d, *J* = 2.8 Hz, 1H), 8.21-8.06 (m, 3H), 7.79 (d, *J* = 8.8 Hz, 1H), 7.63-7.61 (m, 1H), 7.40-7.34 (m, 3H), 7.13-7.06 (m, 4H), 6.91 (d, *J* = 2.4 Hz, 1H), 2.84 (s, 3H).

### Example 24: Preparation of 1-(2-amino-6-(4-fluorophenyl)-5-(4-methylquinazolin-6-yl)pyridin-3-yl)-1H-pyrazol-4-carboxamide (24)

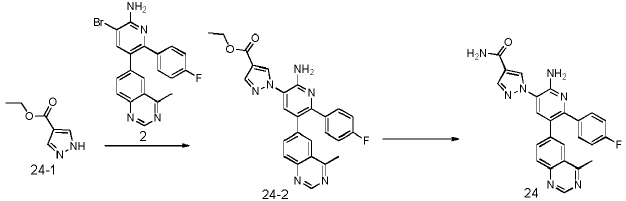

### Step 1: Preparation of ethyl 1-(2-amino-6-(4-fluorophenyl)-5-(4-methylquinazolin-6-yl)pyridin-3-yl)-1H-pyrazol-4-carboxylate (24-2)

By a similar process to that described in Step 1 of Example 23, by replacing ethyl 1*H*-pyrazole-3-carboxylate (23-1) in Step 1 of Example 23 with ethyl 1*H*-pyrazol-4-carboxylate (24-1), the title compound of this step (95 mg, yield: 71.3%) was synthesized.

MS m/z (ESI): 469.2 [M+H]⁺.

### Step 2: Preparation of 1-(2-amino-6-(4-fluorophenyl)-5-(4-methylquinazolin-6-yl)pyridin-3-yl)-1H-pyrazol-4-carboxamide (24)

By a similar process to that described in Step 2 of Example 23, by replacing ethyl 1-(2-amino-6-(4-fluorophenyl)-5-(4-methylquinazolin-6-yl)pyridin-3-yl)-1*H*-pyrazol-3-carboxylate (23-2) in Step 2 of Example 23 with ethyl 1-(2-amino-6-(4-fluorophenyl)-5-(4-methylquinazolin-6-yl)pyridin-3-yl)-1*H*-pyrazol-4-carboxylate (24-2), the title compound (30 mg, yield: 43.0%) was synthesized.

MS m/z (ESI): 440.2 [M+H]⁺.

¹H NMR (400 MHz, DMSO-*d*₆) δ: 9.07 (s, 1H), 8.81 (s, 1H), 8.22 (s, 2H), 8.01 (s, 1H), 7.78 (d, *J* = 8.8 Hz, 1H), 7.72 (s, 1H), 7.59 (d, *J* = 2.0 Hz, 1H), 7.37-7.34 (m, 2H), 7.22 (s, 1H), 7.11 (t, *J* = 8.8 Hz, 2H), 6.82 (s, 2H), 2.85 (s, 3H).

### Example 25: Preparation of 5-(2-amino-6-(4-fluorophenyl)-5-(4-methylquinazolin-6-yl)pyridin-3-yl)-1,3,4-oxadiazol-2-carboxamide (25)

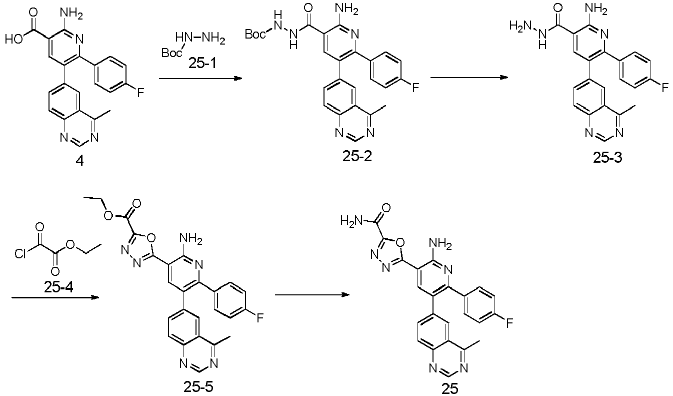

### Step 1: Preparation of tert-butyl 2-(2-amino-6-(4-fluorophenyl)-5-(4-methylquinazolin-6-yl) nicotinoyl)hydrazinecarboxylate (25-2)

2-amino-6-(4-fluorophenyl)-5-(4-methylquinazolin-6-yl)nicotinic acid (4) (2.0 g, 5.35 mmol), carbonyl diimidazole (1.7 g, 10.7 mmol) and DMF (10 mL) were added sequentially to a 50 mL round-bottom flask, and reacted at 25°C for 4 hours. The reaction system was added with *tert*-butyl hydrazinecarboxylate (25-1) (1.32 g, 10.0 mmol), and reacted at 25°C for 12 hours. The reaction mixture was poured into water (100 mL), stirred for 10 min, allowed to stand, filtered with suction, and dried to obtain the title compound of this step (2.4 g, yield: 91.7%).

MS m/z (ESI): 489.2 [M+H]⁺.

### Step 2: Preparation of 2-Amino-6-(4-fluorophenyl)-5-(4-methylquinazolin-6-yl) nicotinohydrazide (25-3)

*Tert*-butyl2-(2-amino-6-(4-fluorophenyl)-5-(4-methylquinazolin-6-yl)nicotinoyl)hydrazine carboxylate (25-2) (2.4 g, 4.91 mmol) and methanol (20 mL) were added to a 50 mL round-bottom flask, added dropwise with a hydrochloride solution in 1,4-dioxane (8 ml, 32 mmol, 4.0 M) and reacted at 25°C for 16 hours. The reaction solution was concentrated. The resultant residue was added with 30 mL water, and adjusted to pH 7-8 with saturated aqueous sodium bicarbonate solution, allowed to stand, and filtered with suction. The filtered residue was dried to obtain the title compound of this step (1.62 g, yield: 85.2%).

MS m/z (ESI): 389.2 [M+H]⁺.

### Step 3: Preparation of ethyl 5-(2-amino-6-(4-fluorophenyl)-5-(4-methylquinazolin-6-yl)pyridin-3-yl)-1,3,4-oxadiazol-2-carboxylate (25-5)

2-amino-6-(4-fluorophenyl)-5-(4-methylquinazolin-6-yl)nicotinohydrazide (25-3) (1.0 g, 2.58 mmol), *N,N*-diisopropylethylamine (0.67 g, 5.16 mmol) and 1,4-dioxane (10 mL) were added sequentially to a 50 mL round-bottom flask, cooled to 0°C, and then reacted at 25°C for 4 hours after adding ethyl 2-chloro-2-oxoacetate (25-4) (0.46 g, 3.35 mmol) dropwise. After cooling to 0°C again, N,N-diisopropylethylamine (0.67 g, 5.16 mmol) and 4-methylbenzenesulfonyl chloride (0.74 g, 3.87 mmol) were added sequentially, and reacted at 25°C for 12 hours. Water was added to the reaction mixture to quench the reaction. The reaction solution was concentrated, and the residue was purified by silica gel column chromatography (eluent: ethyl acetate/petroleum ether = 1/1 (v/v)) to obtain the title compound of this step (0.53 g, yield: 43.3%).

MS m/z (ESI): 471.2 [M+H]⁺.

### Step 4: Preparation of 5-(2-amino-6-(4-fluorophenyl)-5-(4-methylquinazolin-6-yl)pyridin-3-yl)-1,3,4-oxadiazol-2-carboxamide (25)

Ethyl 5-(2-amino-6-(4-fluorophenyl)-5-(4-methylquinazolin-6-yl)pyridin-3-yl)-1,3,4-oxadiazol-2-carboxylate (25-5) (100 mg, 0.22 mmol), an ammonia solution in methanol (1 mL, 7.0 mmol, 7.0 M), and methanol (2 mL) were added sequentially to a 25 mL round-bottom flask, and reacted at 65°C for 4 hours. The reaction solution was cooled to room temperature, and then concentrated. The residue obtained was purified by high performance liquid chromatography (Preparation Method 2) to obtain the title compound (49 mg, yield: 50.5%).

MS m/z (ESI): 442.1 [M+H]⁺.

¹H NMR (400 MHz, DMSO-*d*₆) δ: 9.10 (s, 1H), 8.73 (s, 1H), 8.38 (s, 1H), 8.33 (s, 1H), 8.18 (s, 1H), 7.84-7.61 (m, 4H), 7.41-7.38 (m, 2H), 7.12 (t, *J* = 8.8 Hz, 2H), 2.85 (s, 3H).

### Example 26: Preparation of 1-(6-amino-2'-chloro-2-(4-fluorophenyl)-6'-methyl-[3,4'-bipyridine]-5-yl)-1H-pyrazol-4-carboxamide (26)

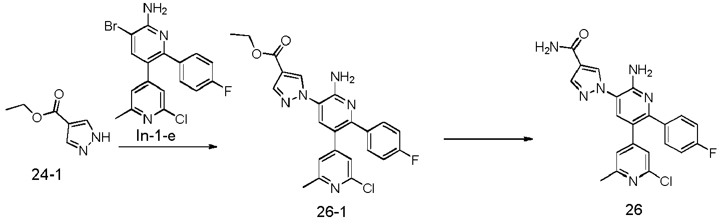

According to the synthetic route of Example 24, with replacing the starting material 3-bromo-6-(4-fluorophenyl)-5-(4-methylquinazolin-6-yl)pyridin-2-amine (2) in Step 1 with 5-bromo-2'-chloro-2-(4-fluorophenyl)-6'-methyl-[3,4'-bipyridine]-6-amine (In-1-e), the title compound was obtained (40 mg, yield: 66.7%).

MS m/z (ESI): 423.1 [M+H]⁺.

¹H NMR (400 MHz, DMSO-*d*₆) δ: 8.84 (s, 1H), 8.23 (s, 1H), 7.92 (s, 1H), 7.75 (br, 1H), 7.41-7.34 (m, 2H), 7.26 (br, 1H), 7.24-7.18 (m, 2H), 7.11 (d, *J* = 3.6 Hz, 2H), 7.00 (br, 2H), 2.36 (s, 3H).

### Example 27: Preparation of 1-(2-amino-6-(4-fluorophenyl)-5-(4-methylquinolin-6-yl) pyridin-3-yl)-1H-pyrazol-3-carboxamide (27)

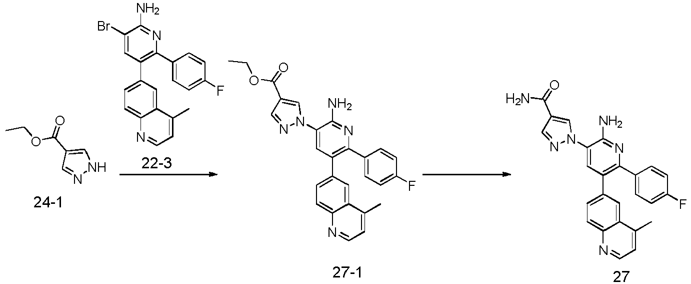

According to the synthetic route of Example 24, with replacing the starting material 3-bromo-6-(4-fluorophenyl)-5-(4-methylquinazolin-6-yl)pyridin-2-amine (2) in Step 1 with 3-bromo-6-(4-fluorophenyl)-5-(4-methylquinolin-6-yl)pyridin-2-amine (22-3), the title compound (40 mg, yield: 66.7%) was obtained.

MS m/z (ESI): 439.2 [M+H]⁺.

¹H NMR (400 MHz, DMSO-*d*₆) δ: 8.81 (s, 1H), 8.71 (s, 1H), 8.21 (s, 2H), 7.98 (s, 1H), 7.83 (d, *J* = 8.8 Hz, 1H), 7.45-7.42 (m, 1H), 7.37-7.33 (m, 3H), 7.11 (t, *J* = 8.8 Hz, 2H), 6.76 (s, 2H), 2.58 (s, 3H).

### Example 28: Preparation of 5-(6-amino-2'-chloro-2-(4-fluorophenyl)-6'-methyl-[3,4'-bipyridine]-5-yl)-1,3,4-oxdiazol-2-carboxamide (28)

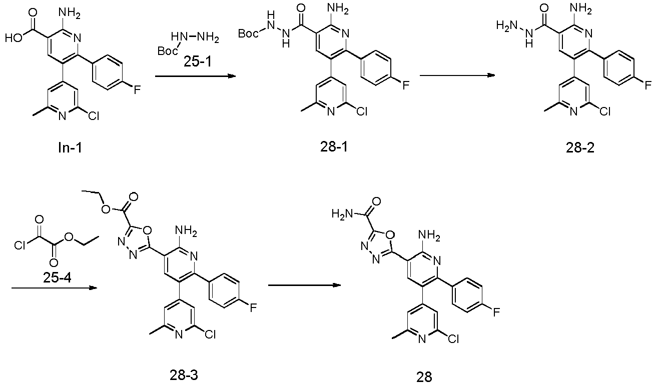

According to the synthetic route of Example 25, with replacing the starting material 2-amino-6-(4-fluorophenyl)-5-(4-methylquinazolin-6-yl)nicotinic acid (4) in Step 1 with 6-amino-2'-chloro-2-(4-fluorophenyl)-6'-methyl-[3,4'-bipyridine]-5-carboxylic acid (In-1), the title compound was obtained (49 mg, yield: 50.5%).

MS m/z (ESI): 425.1[M+H]⁺.

¹H NMR (400 MHz, DMSO-*d*₆) δ: 8.28 (s, 1H), 8.17 (s, 1H), 7.63-7.61 (m, 3H), 7.41-7.39 (m, 2H), 7.20 (t, *J* = 0.8 Hz, 2H), 7.13 (s, 1H), 6.99 (s, 1H), 2.39 (s, 3H).

### Example 29: Preparation of 5-(2-amino-6-(4-fluorophenyl)-5-(4-methylquinolin-6-yl)pyridin-3-yl)-1,3,4-oxadiazol-2-carboxamide (29)

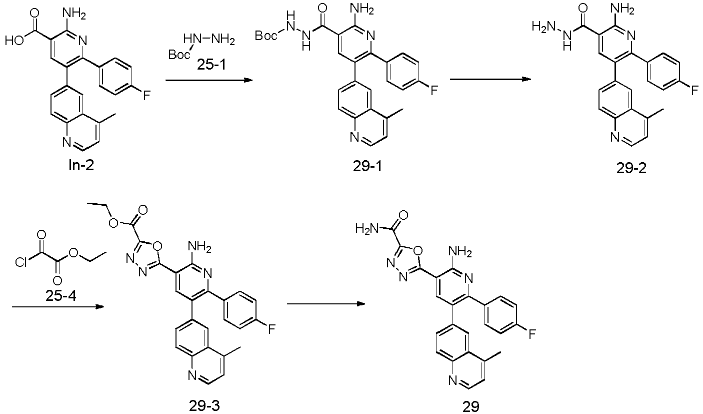

According to the synthetic route of Example 25, with the starting material 2-amino-6-(4-fluorophenyl)-5-(4-methylquinazolin-6-yl)nicotinic acid (4) in Step 1 was replaced with 2-amino-6-(4-fluorophenyl)-5-(4-methylquinolin-6-yl)nicotinic acid (In-2), the title compound was obtained (49 mg, yield: 50.5%).

MS m/z (ESI): 441.1[M+H]⁺.

¹H NMR (400 MHz, DMSO-*d*₆) δ: 8.74 (s, 1H), 8.72 (s, 1H), 8.38 (s, 1H), 8.32 (s, 1H), 7.94 (s, 1H), 7.88 (d, *J* = 8.8 Hz, 1H), 7.48-7.37 (m, 4H), 7.11 (t, *J* = 8.8 Hz, 2H), 2.58 (s, 3H).

### Example 30: Preparation of 3-(4-(6-amino-2'-chloro-2-(4-fluorophenyl)-6'-methyl-[3,4'-bipyridine]-5-yl)-1H-pyrazol-1-yl)propionamide (30)

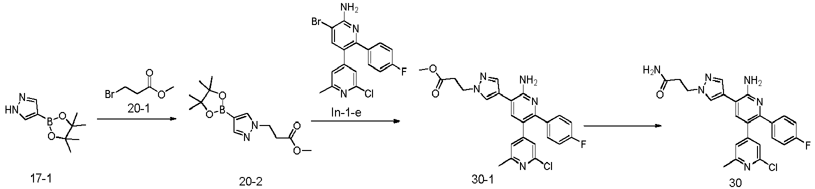

According to the synthetic route of Example 20, with replacing the starting material 3-bromo-6-(4-fluorophenyl)-5-(4-methylquinazolin-6-yl)pyridin-2-amine (2) in Step 2 with 5-bromo-2'-chloro-2-(4-fluorophenyl)-6'-methyl-[3,4'-bipyridine]-6-amine (In-1-e), the title compound was obtained (25 mg, yield: 15.8%).

MS m/z (ESI): 451.1[M+H]⁺.

¹H NMR (400 MHz, DMSO-*d*₆) δ: 8.12 (s, 1H), 7.87 (s, 1H), 7.62 (s, 1H), 7.44 (br, 1H), 7.32-7.29 (m, 2H), 7.16 (t, *J* = 8.8 Hz, 2H), 7.06 (s, 1H), 6.95 (br, 1H), 6.95 (s, 1H), 6.16 (s, 2H), 4.34 (t, *J* = 6.8 Hz, 2H), 2.68 (t, *J* = 6.8 Hz, 2H), 2.34 (s, 3H).

### Example 31: Preparation of 3-(4-(2-amino-6-(4-fluorophenyl)-5-(4-methylquinolin-6-yl) pyridin-3-yl)-1H-pyrazol-1-yl)propionamide (31)

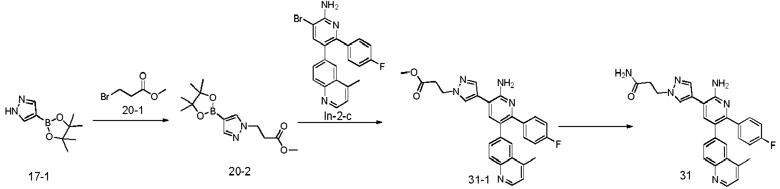

According to the synthetic route of Example 20, with replacing the starting material 3-bromo-6-(4-fluorophenyl)-5-(4-methylquinazolin-6-yl)pyridin-2-amine (2) in Step 2 with 3-bromo-6-(4-fluorophenyl)-5-(4-methylquinolin-6-yl)pyridin-2-amine (In-2-c), the title compound was obtained (25 mg, yield: 15.8%).

MS m/z (ESI): 467.1 [M+H]⁺.

¹H NMR (400 MHz, DMSO-*d*₆) δ: 8.69 (d, *J* = 4.4 Hz, 1H), 8.14 (d, *J* = 0.4 Hz, 1H), 7.88 (dd, *J* = 3.6, 1.2 Hz, 2H), 7.81 (d, *J* = 8.8 Hz, 1H), 7.73 (s, 1H), 7.41 (dd, *J* = 8.8, 2.0 Hz, 1H), 7.36-7.28 (m, 3H), 7.10-7.02 (m, 2H), 5.97 (s, 2H). 4.37 (t,J= 8.8 Hz, 2H), 2.71 (t, *J* = 8.8 Hz, 2H), 2.56 (s, 3H).

### Example 32: Preparation of 1-(4-(6-amino-2'-chloro-2-(4-fluorophenyl)-6'-methyl-[3,4'-bipyridine]-5-yl)-1H-pyrazol-1-yl)-2-methylpropan-2-ol (32)

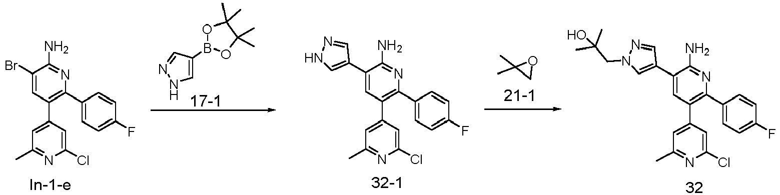

### Step 1: Preparation of 2'-chloro-2-(4-fluorophenyl)-6'-methyl-5-(1H-pyrazol-4-yl)-[3,4'-bipyridine]-6-amine (32-1)

By a similar process to that described in Example 8, by replacing 3-bromo-6-(4-fluorophenyl)-5-(4-methylquinazolin-6-yl)pyridin-2-amine (2) in Example 8 with 5-bromo-2'-chloro-2-(4-fluorophenyl)-6'-methyl-[3,4'-bipyridine]-6-amine (In-1-e), the title compound of this step (218 mg, yield: 76.4%) was synthesized.

MS m/z (ESI): 380.1[M+H]⁺.

### Step 2: Preparation of 1-(4-(6-amino-2'-chloro-2-(4-fluorophenyl)-6'-methyl-[3,4'-bipyridine]-5-yl)-1H-pyrazol-1-yl)-2-methylpropan-2-ol (32)

By a similar process to that described in Example 21, by replacing 6-(4-fluorophenyl)-5-(4-methylquinazolin-6-yl)-3-(1*H*-pyrazol-4-yl)pyridin-2-amine (15-2) in Example 21 with 2'-chloro-2-(4-fluorophenyl)-6'-methyl-5-(1*H*-pyrazol-4-yl)-[3,4'-bipyridine]-6-amine (32-1), the title compound (32 mg, yield: 23%) was synthesized.

MS m/z (ESI): 452.2[M+H]⁺.

¹H NMR (400 MHz, DMSO-*d*₆) δ: 8.08 (s, 1H), 7.86 (s, 1H), 7.63 (s, 1H), 7.32 (dd, *J* = 8.0, 5.6 Hz, 2H), 7.16 (t, *J* = 8.8 Hz, 2H), 7.07 (s, 1H), 6.98 (s, 1H), 6.16 (s, 2H), 4.78 (s, 1H), 4.06 (s, 2H), 2.34 (s, 3H), 1.11 (s, 6H).

### Example 33: Preparation of 1-(4-(2-amino-6-(4-fluorophenyl)-5-(4-methylquinolin-6-yl) pyridin-3-yl)-1H-pyrazol-1-yl)-2-methylpropan-2-ol (33)

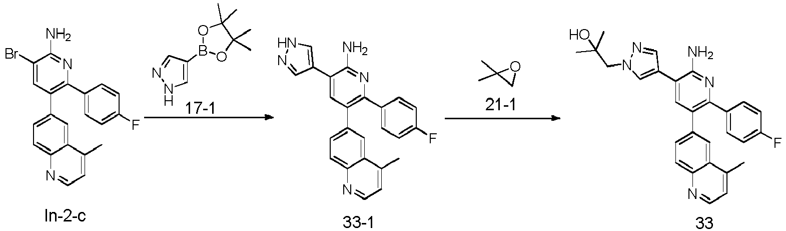

According to the synthetic route of Example 32, with replacing the starting material 5-bromo-2'-chloro-2-(4-fluorophenyl)-6'-methyl-[3,4'-bipyridine]-6-amine (In-1-e) in Step 1 with 3-bromo-6-(4-fluorophenyl)-5-(4-methylquinolin-6-yl)pyridin-2-amine (In-2-c), the title compound was obtained (25 mg, yield: 17.8%).

MS m/z (ESI): 468.2 [M+H]⁺.

¹H NMR (400 MHz, DMSO-*d*₆) δ: 8.78 (s, 1H), 8.10 (s, 1H), 7.83-7.79 (m, 4H), 7.48-7.41 (m, 3H), 7.31 (s,1H), 6.96 (s, 2H), 4.19 (s, 2H), 3.48 (s, 1H), 2.60 (s, 3H), 1.26 (s, 6H).

### Biological Assays

### Experimental Example 1: Determination of the Competitive Inhibition Constant (Kᵢ) for Adenosine A1 and A2a Receptors

### Experimental reagents:

CGS-15943: Sigma, C199
[³H]-DPCPX: PerkinElmer, NET974250UC
[³H]-CGS-21680: PerkinElmer, NET1021250UC
A1 receptor cell membrane (human origin): PerkinElmer, ES-010-M400UA
A2a receptor cell membrane (human origin): PerkinElmer, RBHA2AM400UA
Microscint 20 cocktail scintillation fluid: PerkinElmer, 6013329
PEI (Poly ethyleneimine): Sigma, P3143

Control compound 1: 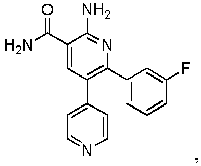 synthesized according to the prior art process.

### Instruments and consumables:

MicroBeta2 Reader: PerkinElmer
Unifilter-96 GF/C filter plate (Perkin Elmer, 6005174)
96-well plate: Agilent, 5042-1385
A1 test buffer: 25mM HEPES, 5mM MgCl₂, 1mM CaCl₂, 100mM NaCl, pH7.4
A1 washing buffer: 25mM HEPES, 5mM MgCl₂, 1mM CaCl₂, 100mM NaCl, pH7.4
A2a test buffer: 50mM Tris-HCl, 10mM MgCl₂, 1mM EDTA, pH7.4
A2a washing buffer: 50mM Tris-HCl, 154mM NaCl, pH7.4

### Experimental method:

The A1 receptor cell membrane was diluted to 0.025µg/µl with the A1 test buffer and the A2a receptor cell membrane was diluted to 0.05µg/µl with the A2a test buffer to obtain an A1 receptor cell membrane dilution and an A2a receptor cell membrane dilution.

The tested compound and CGS15943 were gradiently diluted with DMSO. 1 µl test compound, high-value control (0.5% DMSO), low-value control (1000 nM CGS15943) were added to a 96-well plate, wherein each well was added with 100 µl A1 receptor cell membrane dilution (containing 2.5 µg cell membrane) to obtain an A1 detection plate. Through the same steps, 100 µl A2a receptor cell membrane dilution (containing 5.0 µg cell membrane) was added to each well of a 96-well plate to obtain an A2a detection plate.

100µl of radioisotope-labelled ligand [³H]-DPCPX (diluted in A1 test buffer, working concentration 1.0nM) was added to the A1 detection plate, and 100µl of radioisotope-labelled ligand [³H]-CGS-21680 (diluted in A2a test buffer, working concentration 6.0nM) was added to the A2a detection plate. The A1 and A2a detection plates were sealed with a tap, and incubated at room temperature for 1h and 2h, respectively.

Unifilter-96 GF/C filter plate was prepared. The Unifilter-96 GF/C filter plate was added with 50 µl 0.3% PEI in each well, and incubated at room temperature for no less than 0.5h.

After the incubation, the reaction solutions in the A1 detection plate and the A2a detection plate were transferred to two Unifilter-96 GF/C filter plates. The filter plates were washed with the corresponding pre-cooled washing buffers, then oven dried. After sealing the bottom of the filter plates, 50 µl of Microscint 20 cocktail scintillation fluid was added. After sealing the top of the filter plates, the plates were read in the counter MicroBeta2 Reader.

Data analysis: The inhibition rate was calculated according to the following formula:
Inhibition rate% = 100-(the signal value of the tested wells - the signal average of the low-value control)/(the signal average of the high-value control - the signal average of the low-value control)*100. IC₅₀ was fitted by EXCEL XLfit. The competitive inhibition constant (Kᵢ) was calculated according to the formula: Kᵢ = IC₅₀/(1 + the concentration of the isotope-labelled ligands/K_{d}); K_{d} is the dissociation constant of the isotope-labeled ligands.

The results of the determination of the competitive inhibition constant (Kᵢ) of the compounds of the invention for adenosine A2a and A1 receptors are shown in Table 1:

**Table 1: Competitive Inhibition Constants (Kᵢ) of the compounds of the Invention for Adenosine A2a and A1 Receptors**

| Compound No. | K_{i(Al)} (nM) | K_{i(A2a)} (nM) | K_{i(Al)}/K_{i(A2a)} |
|---|---|---|---|
| 1 | 3254.5 | 9.6 | 339.0 |
| 2 | 291.4 | 2.6 | 112.1 |
| 3 | 1091.7 | 4.9 | 222.8 |
| 4 | 7512.9 | 25.6 | 293.5 |
| 5 | 683.1 | 2.1 | 325.3 |
| 6 | 2191.7 | 3.4 | 644.6 |
| 7 | 4948.9 | 20.2 | 245.0 |
| 8 | 713.0 | 4.8 | 148.5 |
| 9 | 1074.5 | 12.6 | 85.3 |
| 10 | 438.0 | 2.0 | 219.0 |
| 11 | 205.1 | 1.4 | 146.5 |
| 12 | 373.5 | 6.8 | 54.9 |
| 13 | 4379.9 | 12.2 | 359.0 |
| 16 | 1379.1 | 16.7 | 82.6 |
| 17 | 315.8 | 2.3 | 137.3 |
| 19 | 512.6 | 9.9 | 51.8 |
| 20 | 962.7 | 14.0 | 68.8 |
| 21 | 1425.0 | 13.2 | 108.0 |
| 22 | 106.1 | 1.6 | 66.3 |
| 24 | 3480.2 | 23.2 | 150.0 |
| 25 | 132.5 | 2.3 | 57.6 |
| 26 | 4848.2 | 103.6 | 46.8 |
| 27 | 750.2 | 4.0 | 187.6 |
| 29 | 30.0 | 1.2 | 25.0 |
| 30 | 1931.7 | 50.8 | 38.0 |
| 31 | 145.6 | 1.6 | 91.0 |
| Control compound 1 | 25.7 | 138.1 | 0.2 |

The results show that the compounds of the present invention have good binding affinity for adenosine A2a receptors, but weak binding affinity for adenosine A1 receptors, and good selectivity for adenosine A2a receptors. For example, compounds 6 and 7, 12, 13 and 22 of the present invention contain an amide group. Compared to the control compound 1, the compounds of the present invention have significant advantages in both activity and selectivity of adenosine A2a receptors.

### Experimental Example 2: Pharmacokinetics (PK) and Brain Tissue Distribution Study in Rats

The compounds of the present invention and control compound 2 were administered to male SD rats by intravenous injection (IV) and intragastric administration (PO) respectively to study the pharmacokinetic characteristics.

The compounds of the present invention and control compound 2 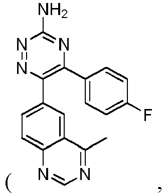 synthesized according to the prior art process) were administered by IV and PO. IV and PO dosages were 1 mg/kg and 5 mg/kg, respectively. The vehicle for the IV administration of the compounds of the present invention was a mixture of 5% DMSO:5% Solutol (polyethylene glycol-15 hydroxystearate):90% physiological saline; and the vehicle for the IV administration of control compound 2 was a mixture of 10% DMSO: 10% Solutol (polyethylene glycol-15 hydroxystearate):80% physiological saline. The vehicle for the PO administration of the compounds was 0.5% MC (sodium methylcellulose). Blood was collected before IV administration (0h), and at time points of 0.083, 0.25, 0.5, 1, 2, 4, 6, 8, 24h or longer (for example, adding time points of 32 and 48h) after IV administration; and blood was collected before PO administration (0h), and at time points of 0.25, 0.5, 1, 2, 4, 6, 8, 24h or longer (for example, adding time points of 32 and 48h) after PO administration. EDTA.K₂ was used for anticoagulation of the blood, and a plasma sample was obtained after centrifugation. Brain tissue homogenates were taken at 0.25, 0.5, 1, and 8 hours after PO administration (3 animals for each time point). The plasma samples and brain tissue homogenate samples were stored at -80°C. Plasma samples and brain tissue homogenates were subjected to analysis by LC-MS/MS, after protein precipitation. The pharmacokinetic parameters were calculated by using non-compartmental model with WinNonlin 6.3 software. The results are shown in Table 2 and Table 3.

**Table 2: Pharmacokinetic Parameters of IV Administered Compounds in Rats**

| Compound No. | Administration Route | Dose mg/kg | AUCₗₐₛₜ h*ng/mL | Cₘₐₓ ng/mL |
|---|---|---|---|---|
| 6 | IV | 1 | 6664 | 1253 |
| 19 | IV | 1 | 3943 | 2957 |
| 20 | IV | 1 | 2529 | 3327 |
| 21 | IV | 1 | 2340 | 1343 |
| Control compound 2 | IV | 1 | 518 | 913 |

The results show that compounds 6 and 19-21 of the present invention have excellent drug exposure (AUCₗₐₛₜ) in rats by IV administration which is 4.5-12.9 times higher than the exposure of control compound 2, and have a maximum blood concentration (Cₘₐₓ) which is 1.4-3.6 times higher than the control compound.

**Table 3: Pharmacokinetic Parameters of PO Administered Compounds in Rats**

| Compound No. | Administration Route | Dose (mg/kg) | AUCₗₐₛₜ | Plasma AUCₗₐₛₜ /Brain AUCₗₐₛₜ |
|---|---|---|---|---|
| 6 | PO | 5 | 24720 (h*ng/mL, plasma) 337 (h*ng/g, brain) | 73/1 |
| 19 | PO | 5 | 18774 (h*ng/mL, plasma) 24.2 (h*ng/g, brain) | 776/1 |
| 20 | PO | 5 | 1896 (h*ng/mL, plasma) undetectable (brain) | Not calculated |
| 21 | PO | 5 | 8014 (h*ng/mL, plasma) 24.2 (h*ng/g, brain) | 331/1 |
| Control Compound 2 | PO | 5 | 1205 (h*ng/mL, plasma) 1013 (h*ng/g, brain) | 1.2/1 |

The results show that compound 6 and compounds 19-21 of the present invention have excellent drug exposure in rat plasma by PO administration, with all ratios of plasma AUCₗₐₛₜ to brain AUCₗₐₛₜ greater than 70. The compounds of the present invention have weak capability to penetrate the blood-brain barrier, especially compound 20 which has only very low exposure to brain tissue, but the capability of control compound 2 to penetrate the blood-brain barrier is significantly stronger.

By calculation, compared to intravenous administration, when orally administered, compound 6 has a bioavailability of 73.9%, compound 19 has a bioavailability of 95.2%, compound 21 has a bioavailability of 68.5%, while control compound 2 has a bioavailability of only 46.5%, which indicates that the compounds of the present invention (such as compounds 6, 19 and 21) have excellent oral absorption in rats.

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A compound or a pharmaceutically acceptable salt, stereoisomer, tautomer, polymorph, solvate, N-oxide, isotopically labeled compound, metabolite or prodrug thereof, wherein the compound has a structure of formula (I): 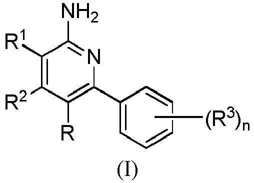 wherein:
R is 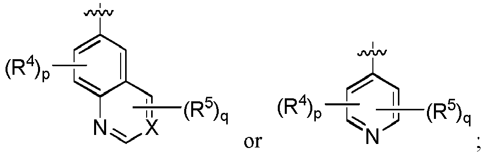
X is N or CH;
R¹ is selected from the group consisting of hydrogen, halogen, cyano, C₁₋₆ alkyl, C₁₋₆ haloalkyl, C₃₋₆ cycloalkyl, carboxyl, C₁₋₆ alkyl-OC(O)-, R^{a}R^{b}N-C(O)-, 5- to 6-membered heterocyclyl and 5- to 6-membered heteroaryl, wherein the heterocyclyl and the heteroaryl groups are optionally substituted with a substituent independently selected from the group consisting of halogen, C₁₋₆ alkyl, C₁₋₆ haloalkyl, C₃₋₆ cycloalkyl, C₁₋₆ alkoxy, hydroxy-C₁₋₆ alkyl-, C₁₋₆ alkoxy-C₁₋₆ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
R² is selected from the group consisting of hydrogen, halogen, cyano, C₁₋₆ alkyl, C₁₋₆ haloalkyl, carboxyl and R^{a}R^{b}N-C(O)-;
R³ is selected from the group consisting of hydrogen, halogen, cyano, C₁₋₆ alkyl, C₁₋₆ haloalkyl and C₁₋₆ alkoxyl;
R⁴ is selected from the group consisting of hydrogen, halogen, C₁₋₆ alkyl, and C₁₋₆ haloalkyl;
R⁵ is selected from the group consisting of hydrogen, halogen, C₁₋₆ alkyl, and C₁₋₆ haloalkyl;
R^{a} and R^{b} are each independently selected from the group consisting of hydrogen, C₁₋₆ alkyl, C₃₋₆ cycloalkyl, hydroxy-C₁₋₆ alkyl-, C₁₋₆ alkoxy-C₁₋₆ alkyl- and 5- to 6-membered heterocyclyl-C₁₋₆ alkyl-;
n is selected from 0, 1 or 2;
p is independently selected from 0, 1 or 2; and
q is independently selected from 0, 1 or 2;
provided that:
when R is 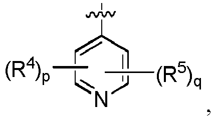 p+q≥2 and at least one of (R⁴)ₚ and (R⁵)_{q} is halogen.

2. The compound of claim 1, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, C₁₋₃ alkyl, C₁₋₃ haloalkyl, C₃₋₆ cycloalkyl, carboxyl, C₁₋₃ alkyl-OC(O)- and R^{a}R^{b}N-C(O)-;
preferably, R¹ is selected from the group consisting of hydrogen, halogen, cyano, carboxy, C₁₋₃ alkyl-OC(O)- and R^{a}R^{b}N-C(O);
preferably, R¹ is selected from the group consisting of hydrogen, halogen, cyano, carboxy, CH₃-OC(O)-, NH₂-C(O)- and NH(CH₃)-C(O)-;
preferably, R¹ is selected from the group consisting of hydrogen, fluorine, chlorine, bromine, iodine, cyano, carboxyl, CH₃-OC(O)-, NH₂-C(O)- and CH₃-NH-C(O)-;
preferably, R¹ is selected from the group consisting of hydrogen, bromine, cyano, carboxyl, CH₃-OC(O)-, NH₂-C(O)- and NH(CH₃)-C(O)-.

3. The compound of claim 1 or 2, or a pharmaceutically acceptable salt, stereoisomer, tautomer, polymorph, solvate, N-oxide, isotopically labeled compound, metabolite or prodrug thereof, wherein:
R¹ is selected from 5- to 6-membered heterocyclyl and 5- to 6-membered heteroaryl, wherein the 5- to 6-membered heterocyclyl and the 5- to 6-membered heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of C₁₋₃ alkyl, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of methyl, ethyl, n-propyl, isopropyl, hydroxy-C₁₋₄ alkyl- (for example, hydroxy-C₁₋₃ alkyl-), C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, the 5- to 6-membered heterocyclyl and the 5- to 6-membered heteroaryl are optionally substituted with a substituent independently selected from the group consisting of methyl, ethyl, n-propyl, isopropyl, hydroxymethyl, hydroxyethyl, OH-(CH₂)₃-, OH-CH(CH₃)-CH₂-, OH-C(CH₃)₂-CH₂-, CH₃O-CH₂-, CH₃O-CH₂CH₂-, CH₃O-(CH₂)₃-, CH₃CH₂O-CH₂-, CH₃CH₂O-CH₂CH₂-, CH₃CH₂O-(CH_{z})₃-, CH₃CH₂CH₂O-(CH₂)₃-, NH₂-CH₂-, NH₂-CH₂CH₂-, NH(CH₃)-CH₂-, NH(CH₃)-CH₂CH₂-, N(CH₃)₂-CH₂-, N(CH₃)₂-CH₂CH₂-, NH₂-C(O)-CH₂-, NH₂-C(O)-CH₂CH₂-, NH(CH₃)-C(O)-CH₂-, NH(CH₃)-C(O)-CH₂CH₂-, N(CH₃)₂-C(O)-CH₂-, N(CH₃)₂-C(O)-CH₂CH₂-, NH₂-C(O)-, NH(CH₃)-C(O)-, N(CH₃)₂-C(O)-, CH₃O-CH₂O-CH₂-, CH₃O-CH₂O-CH₂CH₂-, CH₃O-CH₂O-(CH₂)₃-, CH₃O-CH₂CH₂O-CH₂-, CH₃O-CH₂CH₂O-CH₂CH₂-, CH₃O-CH₂CH₂O-(CH₂)₃-, CH₃CH₂O-CH₂CH₂O-CH₂- and CH₃CH₂O-CH₂CH₂O-CH₂-CH₂-;
preferably, the 5- to 6-membered heterocyclyl and the 5- to 6-membered heteroaryl are optionally substituted with a substituent independently selected from the group consisting of methyl, ethyl, hydroxymethyl, hydroxyethyl, OH-CH(CH₃)-CH₂-, OH-C(CH₃)₂-CH₂-, CH₃O-CH₂-, CH₃O-CH₂CH₂-, CH₃CH₂O-CH₂-, CH₃CH₂O-CH₂CH₂-, NH₂-CH₂-, NH₂-CH₂CH₂-, NH(CH₃)-CH₂-, NH(CH₃)-CH₂CH₂-, NH₂-C(O)-CH₂-, NH₂-C(O)-CH₂CH₂-, NH(CH₃)-C(O)-CH₂-, NH(CH₃)-C(O)-CH₂CH₂-, NH₂-C(O)-, NH(CH₃)-C(O)-, CH₃O-CH₂O-CH₂-, CH₃O-CH₂O-CH₂CH₂-, CH₃O-CH₂CH₂O-CH₂-, CH₃O-CH₂CH₂O-CH₂CH₂- and CH₃CH₂O-CH₂CH₂O-CH₂-.

4. The compound of claim 3, or a pharmaceutically acceptable salt, stereoisomer, tautomer, polymorph, solvate, N-oxide, isotopically labeled compound, metabolite or prodrug thereof, wherein:
the 5- to 6-membered heteroaryl is selected from the group consisting of pyrrolyl, pyrazolyl, imidazolyl, triazolyl, tetrazolyl, oxazolyl, isoxazolyl, thiazolyl, isothiazolyl, oxadiazolyl, thiadiazolyl, pyridyl, pyridazinyl, pyrimidinyl, pyrazinyl and triazinyl;
preferably, the 5- to 6-membered heteroaryl is selected from the group consisting of pyrazolyl, imidazolyl, triazolyl, tetrazolyl, oxazolyl, isoxazolyl, thiazolyl, isothiazolyl, oxadiazolyl, and thiadiazolyl;
preferably, the 5- to 6-membered heteroaryl is selected from the group consisting of pyrazolyl, imidazolyl, triazolyl, tetrazolyl, oxadiazolyl and thiadiazolyl;
preferably, the 5- to 6-membered heteroaryl is selected from the group consisting of pyrazolyl, triazolyl, tetrazolyl, oxadiazolyl, and thiadiazolyl;
preferably, the 5- to 6-membered heteroaryl is selected from the group consisting of pyrazolyl, tetrazolyl, and oxadiazolyl;
preferably, the 5- to 6-membered heteroaryl is selected from

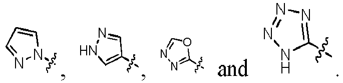

5. The compound of any of claims 1 and 3 to 4, or a pharmaceutically acceptable salt, stereoisomer, tautomer, polymorph, solvate, N-oxide, isotopically labeled compound, metabolite or prodrug thereof, wherein:
R¹ is selected from 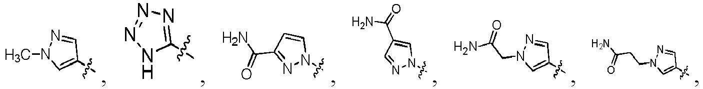 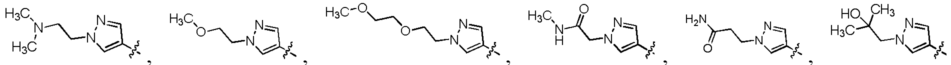 and

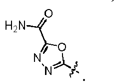

6. The compound of any of claims 1 to 5, or a pharmaceutically acceptable salt, stereoisomer, tautomer, polymorph, solvate, N-oxide, isotopically labeled compound, metabolite or prodrug thereof, wherein: R is

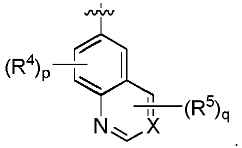

7. The compound of claim 6, or a pharmaceutically acceptable salt, stereoisomer, tautomer, polymorph, solvate, N-oxide, isotopically labeled compound, metabolite or prodrug thereof, wherein:
R¹ is selected from the group consisting of hydrogen, halogen, cyano, C₁₋₆ alkyl, C₁₋₃ haloalkyl, C₃₋₆ cycloalkyl, carboxyl, C₁₋₃ alkyl-OC(O)-, R^{a}R^{b}N-C(O)-, 5- to 6-membered heterocyclyl and 5- to 6-membered heteroaryl, wherein the heterocyclyl and the heteroaryl are optionally substituted with a substituent independently selected from the group consisting of halogen, C₁₋₃ alkyl, C₁₋₃ haloalkyl, C₃₋₆ cycloalkyl, C₁₋₃ alkoxy, hydroxy-C₁₋₄ alkyl-, C₁₋₃ alkoxy-C₁₋₃ alkyl-, R^{a}R^{b}N-C₁₋₆ alkyl-, R^{a}R^{b}N-C(O)-C₁₋₆ alkyl-, R^{a}R^{b}N-C(O)- and C₁₋₆ alkoxy-C₁₋₆ alkoxy-C₁₋₆ alkyl-;
preferably, R¹ is selected from the group consisting of hydrogen, halogen, cyano, carboxy, C₁₋₃ alkyl-OC(O)-, R^{a}R^{b}N-C(O)- and 5- to 6-membered heteroaryl, wherein the heteroaryl is optionally substituted with a substituent independently selected from the group consisting of C₁₋₃ alkyl, hydroxy-C₁₋₄ alkyl-, C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, R¹ is selected from the group consisting of hydrogen, halogen, cyano, carboxy, CH₃O-C(O)-, NH₂-C(O)-, NH(CH₃)-C(O)- and 5- to 6-membered heteroaryl, wherein the heteroaryl is optionally substituted with a substituent independently selected from the group consisting of C₁₋₃ alkyl, hydroxy-C₁₋₄ alkyl-, C₁₋₃ alkoxy-C₁₋₃ alkyl-, R^{a}R^{b}N-C₁₋₃ alkyl-, R^{a}R^{b}N-C(O)-C₁₋₃ alkyl-, R^{a}R^{b}N-C(O)- and C₁₋₃ alkoxy-C₁₋₃ alkoxy-C₁₋₃ alkyl-;
preferably, R¹ is selected from the group consisting of hydrogen, bromine, CH₃O-C(O)-, cyano, carboxyl, NH₂-C(O)-, NH(CH₃)-C(O)-, 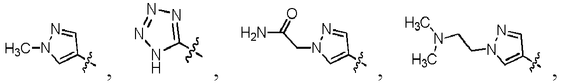 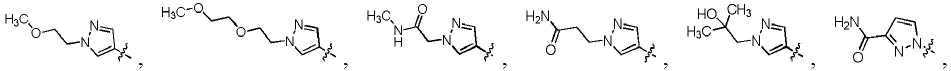 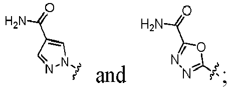
X is selected from N and CH;
R² is selected from the group consisting of hydrogen, chlorine and cyano;
R³ is fluorine;
R⁴ is hydrogen;
R⁵ is methyl;
R^{a} and R^{b} are each independently selected from the group consisting of hydrogen, methyl, ethyl and propyl;
q is 1;
p is 1; and
n is 1 or 2.

8. The compound of any of claims 1 to 5, or a pharmaceutically acceptable salt, stereoisomer, tautomer, polymorph, solvate, N-oxide, isotopically labeled compound, metabolite or prodrug thereof, wherein:
R is 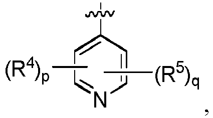 preferably, R is 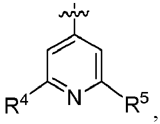 and wherein at least one of R⁴ and R⁵ is halogen.

9. The compound of claim 8, or a pharmaceutically acceptable salt, stereoisomer, tautomer, polymorph, solvate, N-oxide, isotopically labeled compound, metabolite or prodrug thereof, wherein: R¹ is selected from 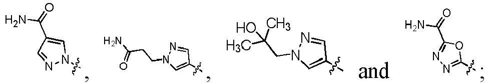 R² is hydrogen; R³ is halogen; R⁴ is selected from C₁₋₃ alkyl; R⁵ is selected from halogen; and n, p and q are each 1.

10. The compound of claim 1, or a pharmaceutically acceptable salt, stereoisomer, tautomer, polymorph, solvate, N-oxide, isotopically labeled compound, metabolite or prodrug thereof, wherein the compound is selected from:

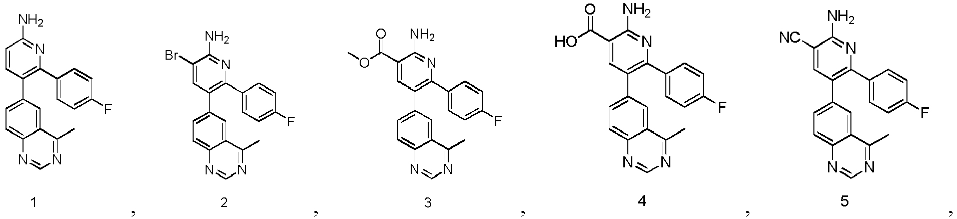

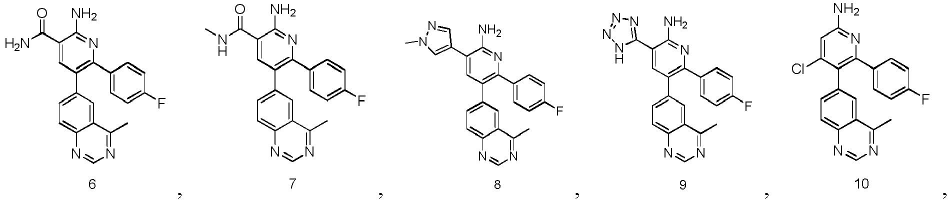

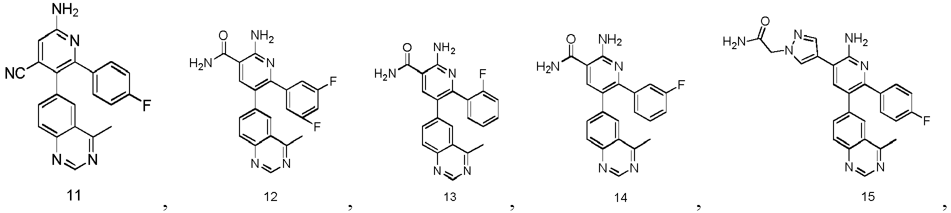

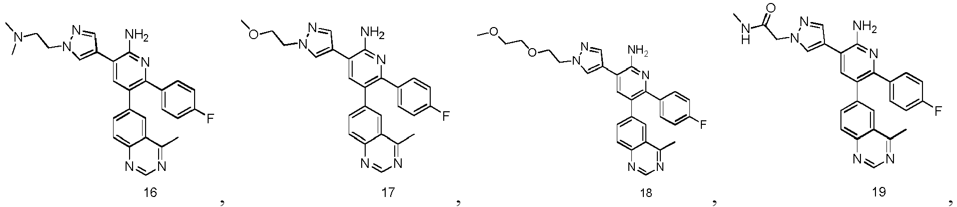

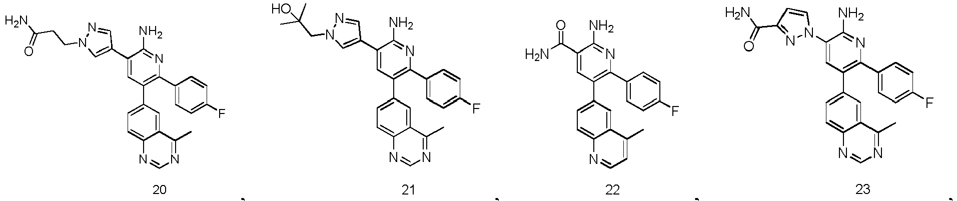

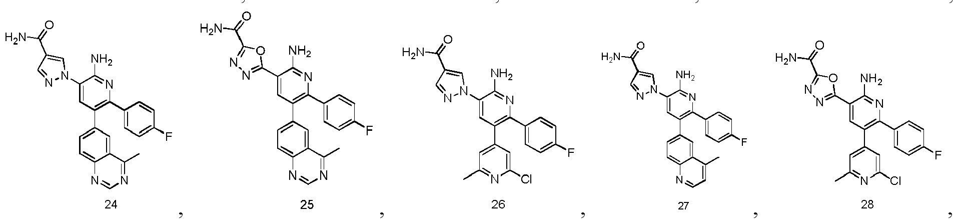

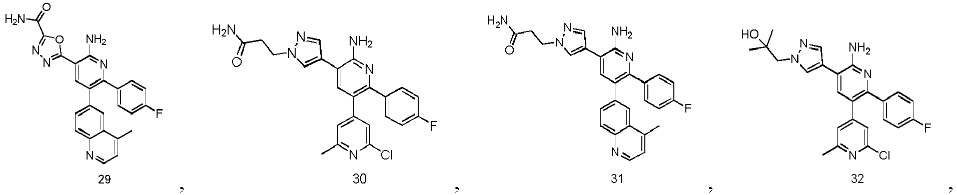

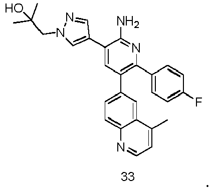

11. A pharmaceutical composition comprising a prophylactically or therapeutically effective amount of the compound of any of claims 1 to 10, or a pharmaceutically acceptable salt, stereoisomer, tautomer, polymorph, solvate, N-oxide, isotopically labeled compound, metabolite or prodrug thereof, and one or more pharmaceutically acceptable carriers.

12. Use of the compound of any of claims 1 to 10 or a pharmaceutically acceptable salt, stereoisomer, tautomer, polymorph, solvate, N-oxide, isotopically labeled compound, metabolite or prodrug thereof, or the pharmaceutical composition of claim 11 in the preparation of a medicament for the prophylaxis or treatment of an adenosine A2a receptor related disease, wherein the medicament is preferably administered via an oral, intravenous, intraarterial, subcutaneous, intraperitoneal, intramuscular, or transdermal route, wherein the adenosine A2a receptor related disease is preferably a tumor.

13. The compound of any of claims 1 to 10 or a pharmaceutically acceptable salt, stereoisomer, tautomer, polymorph, solvate, N-oxide, isotopically labeled compound, metabolite or prodrug thereof, or the pharmaceutical composition of claim 11, for use in the prophylaxis or treatment of an adenosine A2a receptor related disease, preferably by administered via an oral, intravenous, intraarterial, subcutaneous, intraperitoneal, intramuscular, or transdermal route, wherein the adenosine A2a receptor related disease is preferably a tumor.

14. A method for the prophylaxis or treatment of an adenosine A2a receptor related disease, comprising administering to a subject in need thereof an effective amount of the compound of any of claims 1 to 10 or a pharmaceutically acceptable salt, stereoisomer, tautomer, polymorph, solvate, N-oxide, isotopically labeled compound, metabolite or prodrug thereof, or the pharmaceutical composition of claim 11.

15. A method for preparing a compound of formula (Ia-8), (Ia-9), (Ia-10) or (Ia-11), wherein:
the method for preparing the compound of formula (Ia-8) comprises the step of reacting compound Ia-4 with compound IN-e to obtain compound Ia-8; 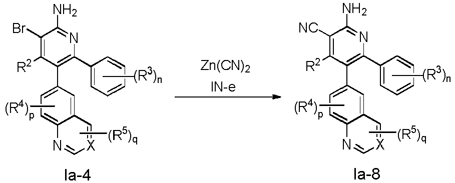
wherein R², R³, R⁴, R⁵, X, p, q, and n are as defined in any of claims 1 to 7;
or
the method for preparing the compound of formula (Ia-9) comprises the step of reacting compound Ia-8 with a base in a solvent to obtain the compound of formula (Ia-9); 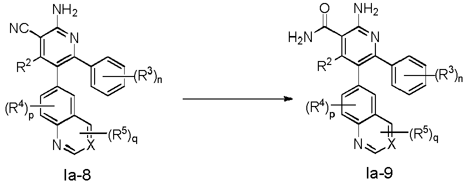
wherein R², R³, R⁴, R⁵, X, p, q, and n are as defined in any of claims 1 to 7;
or
the method for preparing the compound of formula (Ia-10) comprises the step of obtaining the compound of formula (Ia-10) via a coupling reaction between compound Ia-4 and compound IN-f; 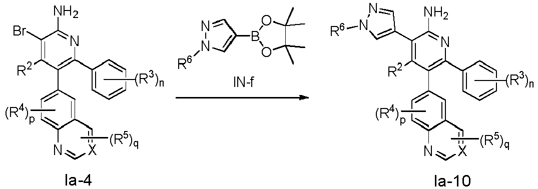
wherein R⁶ is selected from the group consisting of H, C₁₋₆ alkyl (e.g., methyl), C₁₋₆ alkoxy-C₁₋₆ alkyl (e.g., CH₃O-CH₂CH₂-), R^{a}R^{b}N-C₁₋₆ alkyl- (e.g., N(CH₃)₂-CH₂CH₂-), C₁₋₆ alkoxy-C₁₋₆ alkoxy-C₁₋₆ alkyl-(e.g., CH₃O-CH₂CH₂O-CH₂CH₂-) and C₁₋₆ alkoxy-C(O)-C₁₋₆ alkyl- (e.g., CH₃OC(O)-CH₂CH₂- or CH₃CH₂OC(O)-CH₂-); and
R², R³, R⁴, R⁵, R^{a}, R^{b}, X, p, q, and n are as defined in any of claims 1 to 7;
or
the method for preparing the compound of formula (la-11) comprises the step of reacting the compound of formula (Ia-10) with NHR^{a}R^{b} to obtain the compound of formula (Ia-11); 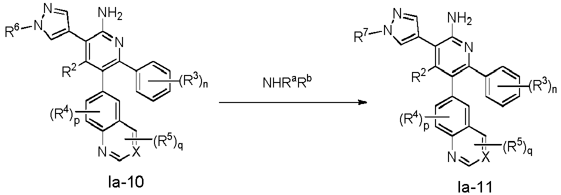
wherein R⁶ is C₁₋₆ alkoxy-C(O)-C₁₋₆ alkyl-, e.g., CH₃OC(O)-CH₂CH₂- or CH₃CH₂OC(O)-CH₂-;
R⁷ is R^{a}R^{b}N-C(O)-C₁₋₆ alkyl-, e.g., NH₂-C(O)-CH₂CH₂- or NH(CH₃)-C(O)-CH₂-; and
R², R³, R⁴, R⁵, R^{a}, R^{b}, X, p, q, and n are as defined in any of claims 1 to 7;
preferably, the compound of formula (Ia-10) is reacted with NHR^{a}R^{b} (e.g., NH₃ or methylamine) in a suitable alcohol (e.g., methanol).
